(19) Europäisches Patentamt
European Patent Office
Office européen des brevets

(11) EP 4 180 422 A1

(12) **EUROPEAN PATENT APPLICATION**
published in accordance with Art. 153(4) EPC

(43) Date of publication:
**17.05.2023 Bulletin 2023/20**

(21) Application number: **21837191.2**

(22) Date of filing: **09.07.2021**

(51) International Patent Classification (IPC):
***C07D 211/34*** *(2006.01)* ***C07D 401/12*** *(2006.01)*
***A61K 31/451*** *(2006.01)* ***A61K 31/454*** *(2006.01)*
***A61P 35/00*** *(2006.01)*

(52) Cooperative Patent Classification (CPC):
**A61K 31/451; A61K 31/454; A61P 35/00; C07D 211/34; C07D 401/12;** Y02P 20/55

(86) International application number:
**PCT/CN2021/105436**

(87) International publication number:
**WO 2022/007940 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.07.2020 CN 202010663125**

(71) Applicants:
- **Jiangsu Hengrui Pharmaceuticals Co., Ltd. Lianyungang, Jiangsu 222047 (CN)**
- **Shanghai Hengrui Pharmaceutical Co., Ltd. Shanghai 200245 (CN)**

(72) Inventors:
- **XU, Jianyan Shanghai 200245 (CN)**
- **GAO, Jianhong Shanghai 200245 (CN)**
- **ZHANG, Ying Shanghai 200245 (CN)**
- **HE, Feng Shanghai 200245 (CN)**
- **TAO, Weikang Shanghai 200245 (CN)**

(74) Representative: **Viganò, Elena et al
Dragotti & Associati S.r.l.
Via Nino Bixio, 7
20129 Milano (IT)**

(54) **SULFONYLBENZAMIDE DERIVATIVE AND CONJUGATE THEREOF, PREPARATION METHOD THEREFOR AND USE THEREOF**

(57) Provided are a sulfonylbenzamide derivative and a conjugate thereof, a preparation method therefor, and the use thereof. In particular, provided is a sulfonylbenzamide derivative having a structure as represented by formula (D), a conjugate thereof, a preparation method therefor, a pharmaceutical composition containing same, and the use thereof in the preparation of a drug for treating cancers by means of receptor regulation. Each substituent in general formula (D) is as defined in the description.

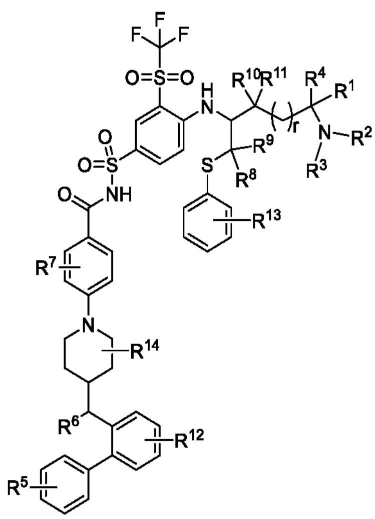

(D)

EP 4 180 422 A1

## Description

## TECHNICAL FIELD

The present disclosure relates to a sulfonylbenzamide derivative and a ligand-drug conjugate (ADC) thereof. Particularly, the present disclosure relates to a sulfonylbenzamide derivative with a brand new structure and an antibody-drug conjugate thereof, a preparation method therefor, a pharmaceutical composition comprising the conjugate, and use of the conjugate or the pharmaceutical composition.

## BACKGROUND

An important feature that distinguishes tumor cells from normal cells is that apoptosis in tumor cells is inhibited, which gives them a greater survival advantage. The proteins of BCL-2 family are important factors in regulating apoptosis inhibition.

The proteins of BCL-2 family are mainly present on mitochondrial membranes and can be classified into two major groups according to their functions: anti-apoptotic and pro-apoptotic proteins. The anti-apoptotic protein includes BCL-2, BCL-xL, BCL-w, MCL-1 and the like. The pro-apoptotic protein includes Bax, Bak and BH3-only protein. Bax and Bak, upon activation, will convert from harmless monomers into deadly oligomers that form pores in the mitochondrial outer membrane, increase the permeability of the mitochondrial membrane of a cell, and promote the release of cytochrome C and the like into the cytoplasm, leading to cell death. The BH3-only protein contains only the BH3 domain. In the surviving state of the cell, the BH3-only protein (e.g., Bim) binds to the anti-apoptotic protein. When the cell is subjected to external pressure, the binding balance is broken, and the BH3-only protein is released to bind to BAX on the mitochondria, thereby promoting the formation of BAX/BAK polymers, promoting the release of cytochrome C and SMAC into the cytoplasm, and activating the downstream apoptotic pathway.

The available preclinical data show that the BCL2/BCL-xL dual-target inhibitor ABT263 is not only effective against hematological tumors, but also has a good inhibitory effect on solid tumors, especially small cell lung cancer. However, the clinical toxicity of ABT263 to platelets limited its efficacy and eventually led to the termination of clinical trials.

There is still a need to investigate the BCL2/BCL-xL dual-target inhibitor, so that it can be used as a single agent or in a combination therapy or in ADCs to meet clinical requirements.

## SUMMARY

The present disclosure provides a compound of general formula (D) or a pharmaceutically acceptable salt thereof:

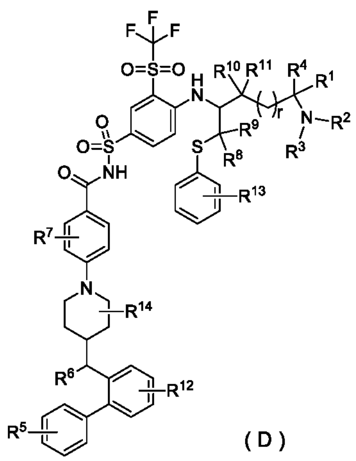

( D )

wherein:

- $R^1$ is selected from the group consisting of deuterium, hydrogen, alkyl and deuterated alkyl;
- $R^2$ is selected from the group consisting of alkyl and hydroxyalkyl;
- or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;
- $R^3$ is selected from the group consisting of hydrogen, hydroxyalkyl, alkyl, deuterated alkyl, cycloalkyl and cycloalky-

lalkyl;

$R^4$ is selected from the group consisting of hydrogen, halogen, deuterated alkyl and alkyl;

$R^5$ is selected from the group consisting of halogen and haloalkyl;

$R^6$ is selected from the group consisting of alkyl, amino, hydroxy and alkoxy;

$R^7$ is selected from the group consisting of hydrogen, halogen, alkyl, deuterated alkyl, hydroxy and alkoxy;

$R^8$ and $R^9$ are each independently selected from the group consisting of hydrogen, alkyl, deuterated alkyl and cycloalkyl; or $R^8$ and $R^9$, together with the atom to which they are attached, form cycloalkyl;

$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;

$R^{12}$ is selected from the group consisting of hydrogen, deuterium, deuterated alkyl, halogen, hydroxy, alkyl, hydroxyalkyl, alkoxy and cycloalkyl;

$R^{13}$ is selected from the group consisting of hydrogen, deuterium, deuterated alkyl, halogen, hydroxy, alkyl, hydroxyalkyl, alkoxy and cycloalkyl;

$R^{14}$ is selected from the group consisting of hydrogen, deuterium, deuterated alkyl, halogen, hydroxy, alkyl, hydroxyalkyl, alkoxy and cycloalkyl; and

r is selected from the group consisting of 0, 1, 2 and 3;

provided that when $R^1$ and $R^2$ do not, together with the atom to which they are attached, form heterocyclyl, $R^5$ is haloalkyl.

In some other embodiments of the present disclosure, provided is the compound of general formula (D) or the pharmaceutically acceptable salt thereof, wherein $R^1$ is hydrogen, and $R^2$ is alkyl.

In some other embodiments of the present disclosure, provided is the compound of general formula (D) or the pharmaceutically acceptable salt thereof, wherein $R^1$ and $R^2$, together with the atom to which they are attached, form heterocyclyl; preferably, the heterocyclyl optionally further comprises 1 to 3 heteroatoms selected from the group consisting of nitrogen and oxygen; more preferably, $R^1$ and $R^2$, together with the atom to which they are attached, form a 3- to -6-membered heterocyclyl; preferably, the 3- to 6-membered heterocyclyl optionally further comprises 1 to 3 heteroatoms selected from the group consisting of nitrogen and oxygen; and most preferably, $R^1$ and $R^2$, together with the atom to which they are attached, form pyrrolidinyl.

In some other embodiments of the present disclosure, provided is the compound of general formula (D) or the pharmaceutically acceptable salt thereof, which is a compound of general formula (D-I) or a pharmaceutically acceptable salt thereof:

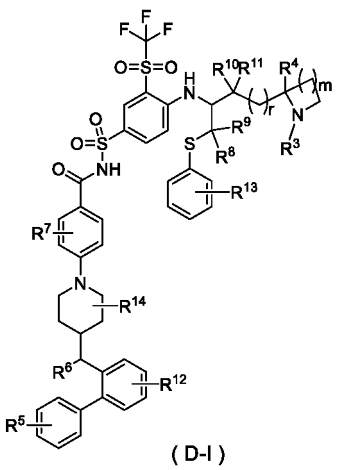

( D-I )

wherein m is selected from the group consisting of 0, 1, 2 and 3; and

r and $R^3$-$R^{14}$ are as defined in general formula (D).

In some other embodiments of the present disclosure, provided is the compound of general formula (D) or the pharmaceutically acceptable salt thereof, which is a compound of general formula (D-II) or a pharmaceutically acceptable salt thereof:

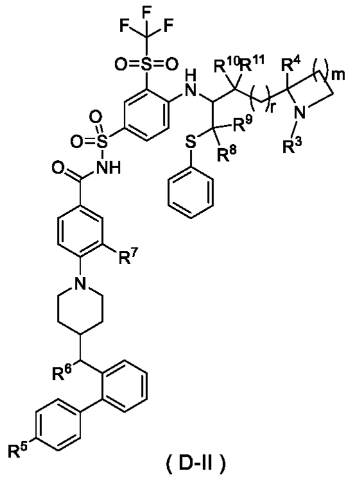

( D-II )

wherein m is selected from the group consisting of 0, 1, 2 and 3; and r and $R^3$-$R^{11}$ are as defined in general formula (D).

In some other embodiments of the present disclosure, provided is the compound of general formula (D) or the pharmaceutically acceptable salt thereof, which is a compound of general formula (D-III) or a pharmaceutically acceptable salt thereof:

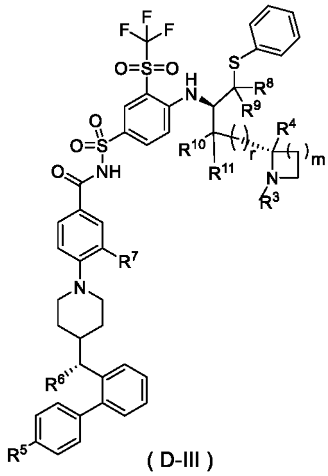

( D-III )

wherein m is selected from the group consisting of 0, 1, 2 and 3; and r and $R^3$-$R^{11}$ are as defined in general formula (D).

In some other embodiments of the present disclosure, provided is the compound of general formula (D) or the pharmaceutically acceptable salt thereof, which is a compound of general formula (D-IV) or a pharmaceutically acceptable salt thereof:

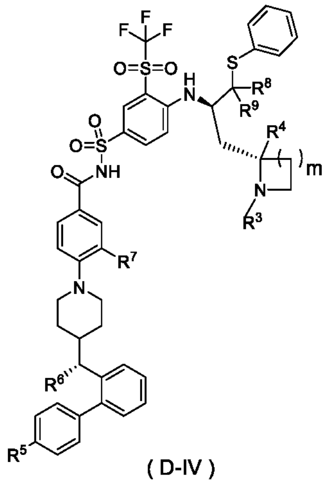

( D-IV )

wherein m is selected from the group consisting of 0, 1, 2 and 3; and
$R^3$-$R^9$ are as defined in general formula (D).

In some other embodiments of the present disclosure, provided is the compound of general formula (D) or the pharmaceutically acceptable salt thereof, wherein m is 2.

In some other embodiments of the present disclosure, provided is the compound of general formula (D) or the pharmaceutically acceptable salt thereof, wherein $R^5$ is chlorine.

In some other embodiments of the present disclosure, provided is the compound of general formula (D) or the pharmaceutically acceptable salt thereof, wherein $R^5$ is trifluoromethyl.

In some other embodiments of the present disclosure, provided is the compound of general formula (D) or the pharmaceutically acceptable salt thereof, wherein $R^3$ is selected from the group consisting of hydrogen, hydroxyalkyl and alkyl; preferably hydroxyalkyl; and more preferably $C_{1-6}$ hydroxyalkyl.

In some other embodiments of the present disclosure, provided is the compound of general formula (D) or the pharmaceutically acceptable salt thereof, which is a compound of general formula (D-V) or a pharmaceutically acceptable salt thereof:

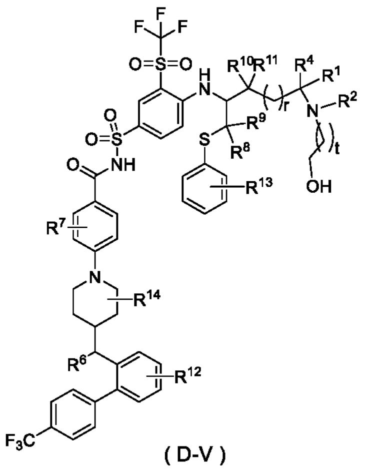

( D-V )

wherein t is selected from the group consisting of 0, 1, 2 and 3, preferably 1; and
r, $R^1$, $R^2$, $R^4$, and $R^6$-$R^{14}$ are as defined in general formula (D).

In some other embodiments of the present disclosure, provided is the compound of general formula (D) or the pharmaceutically acceptable salt thereof, which is a compound of general formula (D-VI) or a pharmaceutically acceptable salt thereof:

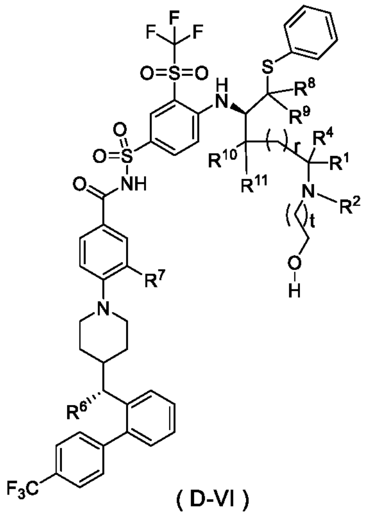

( D-VI )

wherein t is selected from the group consisting of 0, 1, 2 and 3, preferably 1; and
r, $R^1$, $R^2$, $R^4$, and $R^6$-$R^{11}$ are as defined in general formula (D).

In some other embodiments of the present disclosure, provided is the compound of general formula (D) or the pharmaceutically acceptable salt thereof, which is a compound of general formula (D-VII) or a pharmaceutically acceptable salt thereof:

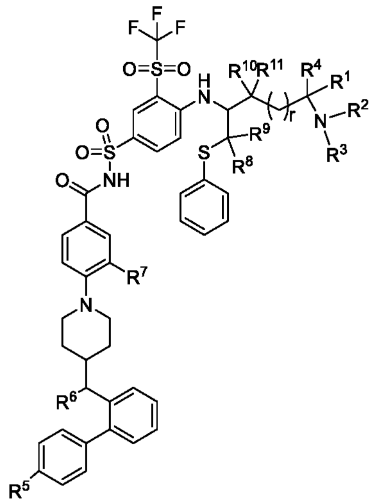

( D-VII )

wherein t is selected from the group consisting of 0, 1, 2 and 3, preferably 1; and
r and $R^1$-$R^{11}$ are as defined in general formula (D).

In some other embodiments of the present disclosure, provided is the compound of general formula (D) or the pharmaceutically acceptable salt thereof, wherein $R^4$ is selected from the group consisting of hydrogen and alkyl; preferably hydrogen and $C_{1-6}$ alkyl; and more preferably hydrogen.

In some other embodiments of the present disclosure, provided is the compound of general formula (D) or the pharmaceutically acceptable salt thereof, wherein $R^6$ is selected from the group consisting of hydroxy and alkoxy; preferably hydroxy and $C_{1-6}$ alkoxy; and more preferably hydroxy.

In some other embodiments of the present disclosure, provided is the compound of general formula (D) or the pharmaceutically acceptable salt thereof, wherein $R^7$, $R^8$ and $R^9$ are hydrogen.

In some other embodiments of the present disclosure, provided is the compound of general formula (D) or the pharmaceutically acceptable salt thereof, wherein $R^{10}$ and $R^{11}$ are each independently selected from the group consisting of hydrogen and alkyl; preferably hydrogen and $C_{1-6}$ alkyl; and more preferably hydrogen.

In some other embodiments of the present disclosure, provided is the compound of general formula (D) or the

pharmaceutically acceptable salt thereof, wherein r is 0 or 1, preferably 0.

In some other embodiments of the present disclosure, provided is the compound of general formula (D), including but not limited to:

| Examples | Structure |
|---|---|
| 2-1 | 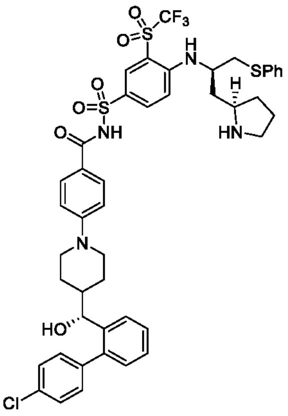 **1** |
| | 4-(4-((*R*)-(4'-chloro-[1,1'-biphenyl]-2-yl)(hydroxy)methyl)piperidin-1-yl)-*N*-((4-(((*R*)-1-(phenylthio)-3-((*R*)-pyrrolidin-2-yl)propan-2-yl)amino)-3-((t rifluoromethyl)sulfonyl)phenyl)sulfonyl)benzamide **1** |
| 2-2 | 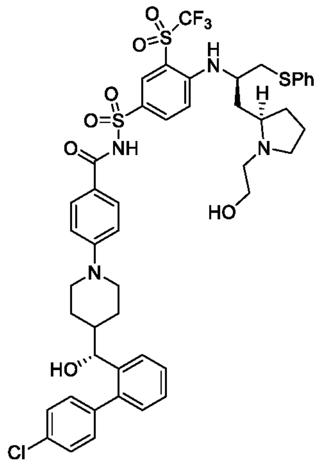 **2** |
| | 4-(4-((*R*)-(4'-chloro-[1,1'-biphenyl]-2-yl)(hydroxy)methyl)piperidin-1-yl)-*N*-((4-(((*R*)-1-((*R*)-1-(2-hydroxyethyl)pyrrolidin-2-yl)-3-(phenylthio)propa n-2-yl)amino)-3-((trifluoromethyl)sulfonyl)phenyl) sulfonyl)benzamide **2** |

(continued)

| Examples | Structure |
|---|---|
| 2-3 | 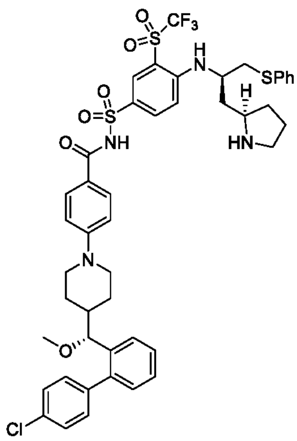<br>**3** |
| | 4-(4-((*R*)-(4'-chloro-[1,1'-biphenyl]-2-yl)(methoxy)methyl)piperidin-1-yl)-*N*-((4-(((*R*)-1-(phenylthio)-3-((*R*)-pyrrolidin-2-yl)propan-2-yl)amino)-3-((t rifluoromethyl)sulfonyl)phenyl)sulfonyl)benzamide **3** |
| 2-4 | 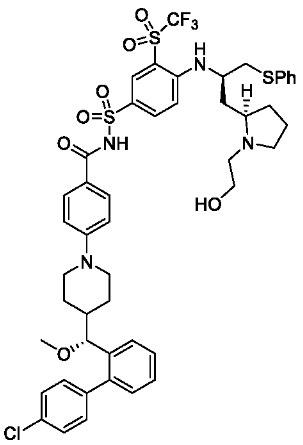<br>**4** |
| | 4-(4-((*R*)-(4'-chloro-[1,1'-biphenyl]-2-yl)(methoxy)methyl)piperidin-1-yl)-*N*-((4-(((*R*)-1-((R)-1-(2-hydroxyethyl)pyrrolidin-2-yl)-3-(phenylthio)propa n-2-yl)amino)-3-((trifluoromethyl)sulfonyl)phenyl) sulfonyl)benzamide **4** |

(continued)

| Examples | Structure |
|---|---|
| 2-5 | 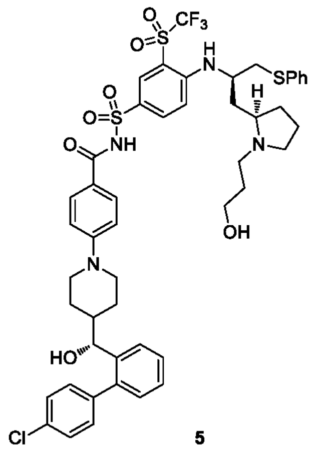 5 |
| | 4-(4-((*R*)-(4'-chloro-[1,1'-biphenyl]-2-yl)(hydroxy)methyl)piperidin-1-yl)-*N*-((4-(((*R*)-1-((*R*)-1-(3-hydroxypropyl)pyrrolidin-2-yl)-3-(phenylthio)prop an-2-yl)amino)-3-((trifluoromethyl)sulfonyl)phenyl) sulfonyl)benzamide **5** |
| 2-6 | 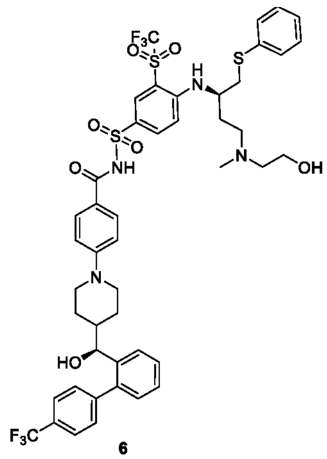 6 |
| | 4-(4-((*S*)-hydroxy(4'-(trifluoromethyl)-[1,1'-biphenyl]-2-yl)methyl)piperidi n-1-yl)-*N*-((4-(((*R*)-4-((2-hydroxyethyl)(methyl)amino)-1-(phenylthio)buta n-2-yl)amino)-3-((trifluoromethyl)sulfonyl)phenyl) sulfonyl)benzamide **6** |

(continued)

| Examples | Structure |
|---|---|
| 2-7 | 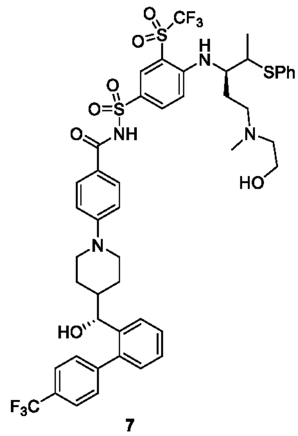 7 |
| | 4-(4-((*R*)-hydroxy(4'-(trifluoromethyl)-[1,1'-biphenyl]-2-yl)methyl)piperidi n-1-yl)-*N*-((4-(((3*R*)-1-((2-hydroxyethyl)(methyl)amino)-4-(phenylthio)pen tan-3-yl)amino)-3-((trifluoromethyl)sulfonyl)phenyl) sulfonyl)benzamide **7** |
| 2-8 | 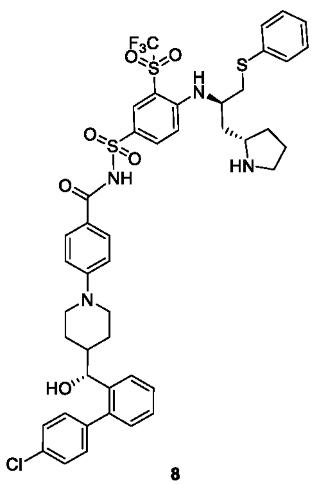 8 |
| | 4-(4-((*R*)-(4'-chloro-[1,1'-biphenyl]-2-yl)(hydroxy)methyl)piperidin-1-yl)-*N*-((4-(((*R*)-1-(phenylthio)-3-((*S*)-pyrrolidin-2-yl)propan-2-yl)amino)-3-((tr ifluoromethyl)sulfonyl)phenyl)sulfonyl)benzamide **8** |

(continued)

| Examples | Structure |
| --- | --- |
| 2-9 | 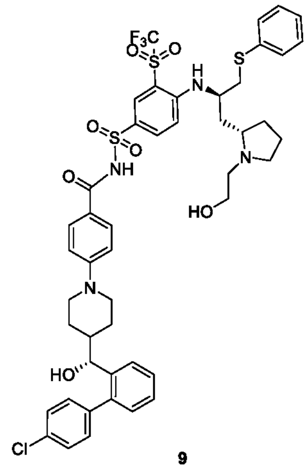 9 |
| | 4-(4-((*R*)-(4'-chloro-[1,1'-biphenyl]-2-yl)(hydroxy)methyl)piperidin-1-yl)-*N*-((4-(((R)-1-((S)-1-(2-hydroxyethyl)pyrrolidin-2-yl)-3-(phenylthio)propa n-2-yl)amino)-3-((trifluoromethyl)sulfonyl)phenyl) sulfonyl)benzamide **9** |
| 2-10 | 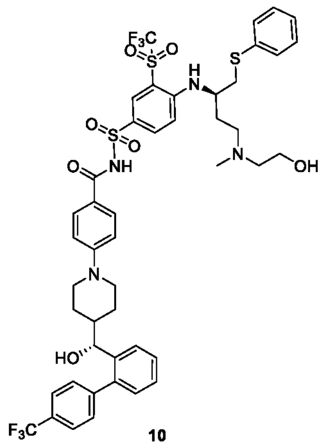 10 |
| | 4-(4-((*R*)-hydroxy(4'-(trifluoromethyl)-[1,1'-biphenyl]-2-yl)methyl)piperidi n-1-yl)-*N*-((4-(((*R*)-4-((2-hydroxyethyl)(methyl)amino)-1-(phenylthio)buta n-2-yl)amino)-3-((trifluoromethyl)sulfonyl)phenyl) sulfonyl)benzamide **10** |

(continued)

| Examples | Structure |
|---|---|
| 2-11 | 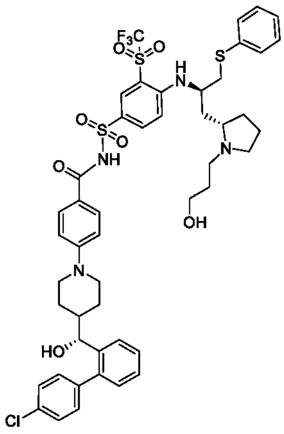 11 |
| | 4-(4-((*R*)-(4'-chloro-[1,1'-biphenyl]-2-yl)(hydroxy)methyl)piperidin-1-yl)-*N*-((4-(((*R*)-1-((S)-1-(3-hydroxypropyl)pyrrolidin-2-yl)-3-(phenylthio)prop an-2-yl)amino)-3-((trifluoromethyl)sulfonyl)phenyl) sulfonyl)benzamide **11** |
| 2-12 | 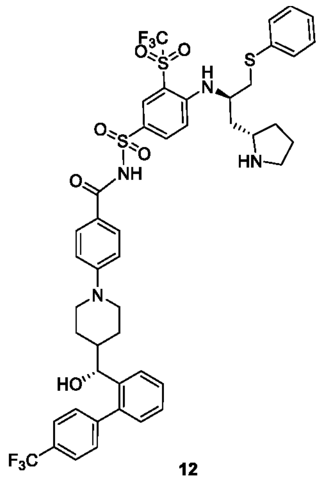 12 |
| | 4-(4-((R)-hydroxy(4'-(trifluoromethyl)-[1,1'-biphenyl]-2-yl)methyl)piperidi n-1-yl)-*N*-((4-(((*R*)-1-(phenylthio)-3-((S)-pyrrolidin-2-yl)propan-2-yl)amin o)-3-((trifluoromethyl)sulfonyl)phenyl)sulfonyl) benzamide **12** |

(continued)

| Examples | Structure |
|---|---|
| 2-13 | 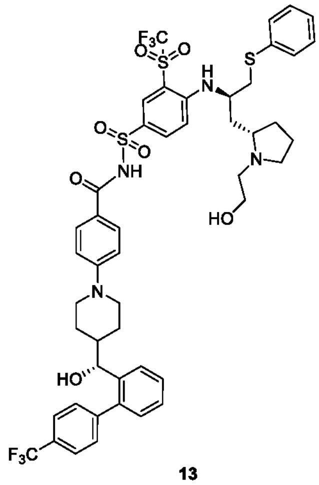<br>**13** |
| | 4-(4-((*R*)-hydroxy(4'-(trifluoromethyl)-[1,1'-biphenyl]-2-yl)methyl)piperidi n-1-yl)-*N*-((4-(((R)-1-((S)-1-(2-hydroxyethyl)pyrrolidin-2-yl)-3-(phenylthi o)propan-2-yl)amino)-3-((trifluoromethyl)sulfonyl) phenyl)sulfonyl)benza mide **13** |
| 2-14 | 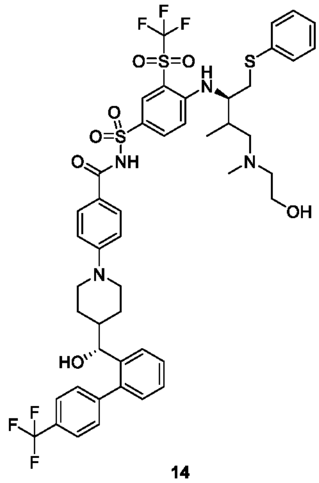<br>**14** |
| | 4-(4-((*R*)-hydroxy(4'-(trifluoromethyl)-[1,1'-biphenyl]-2-yl)methyl)piperidi n-1-yl)-*N*-((4-(((2*R*)-4-((2-hydroxyethyl)(methyl)amino)-3-methyl-1-(phen ylthio)butan-2-yl)amino)-3-((trifluoromethyl)sulfonyl) phenyl)sulfonyl)ben zamide **14** |
| 2-15 | 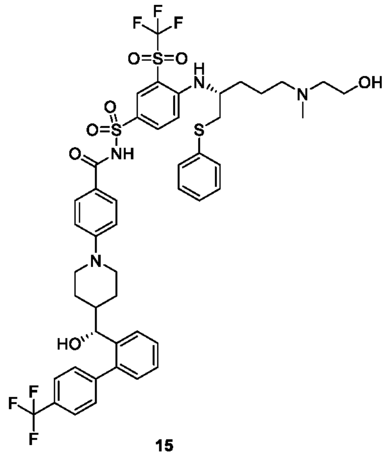<br>**15** |

(continued)

| Examples | Structure |
|---|---|
| | 4-(4-((*R*)-hydroxy(4'-(trifluoromethyl)-[1,1'-biphenyl]-2-yl)methyl)piperidi n-1-yl)-*N*-((4-(((R)-5-((2-hydroxyethyl)(methyl)amino)-1-(phenylthio)pent an-2-yl)amino)-3-((trifluoromethyl)sulfonyl)phenyl) sulfonyl)benzamide **15** |
| 2-16 | 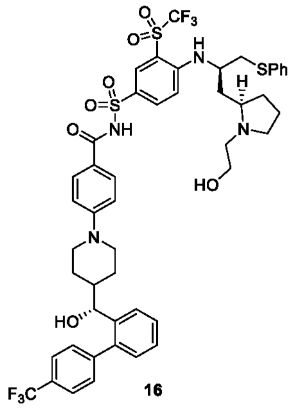 16 |
| | 4-(4-((*R*)-hydroxy(4'-(trifluoromethyl)-[1,1'-biphenyl]-2-yl)methyl)piperidi n-1-yl)-*N*-((4-(((R)-1-((R)-1-(2-hydroxyethyl)pyrrolidin-2-yl)-3-(phenylthi o)propan-2-yl)amino)-3-((trifluoromethyl)sulfonyl) phenyl)sulfonyl)benza mide **16** |
| 2-17 | 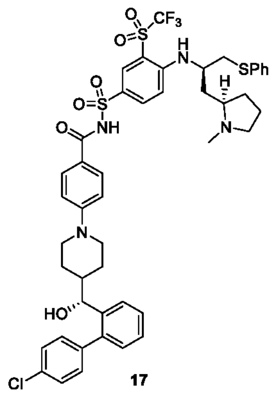 17 |
| | 4-(4-((*R*)-(4'-chloro-[1,1'-biphenyl]-2-yl)(hydroxy)methyl)piperidin-1-yl)-*N*-((4-(((*R*)-1-((R)-1-methylpyrrolidin-2-yl)-3-(phenylthio)propan-2-yl)ami no)-3-((trifluoromethyl)sulfonyl)phenyl)sulfonyl) benzamide **17** |

(continued)

| Examples | Structure |
|---|---|
| 2-18 | 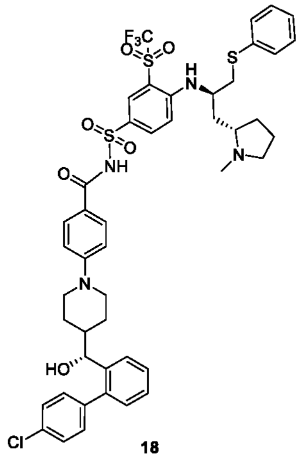 18 |
| | 4-(4-((*R*)-(4'-chloro-[1,1'-biphenyl]-2-yl)(hydroxy)methyl)piperidin-1-yl)-*N*-((4-(((*R*)-1-((*S*)-1-methylpyrrolidin-2-yl)-3-(phenylthio)propan-2-yl)amino)-3-((trifluoromethyl)sulfonyl)phenyl)sulfonyl) benzamide 1**8** |
| 2-19 | 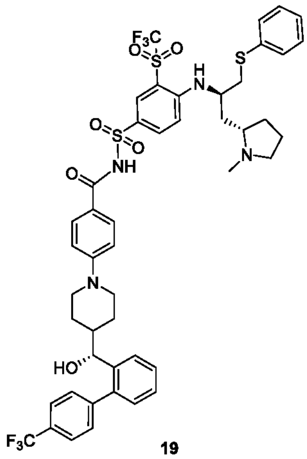 19 |
| | 4-(4-((*R*)-hydroxy(4'-(trifluoromethyl)-[1,1'-biphenyl]-2-yl)methyl)piperidi n-1-yl)-*N*-((4-(((*R*)-1-((*S*)-1-methylpyrrolidin-2-yl)-3-(phenylthio)propan-2 -yl)amino)-3-((trifluoromethyl)sulfonyl)phenyl)sulfonyl) benzamide **19** |

(continued)

| Examples | Structure |
|---|---|
| 2-20 | 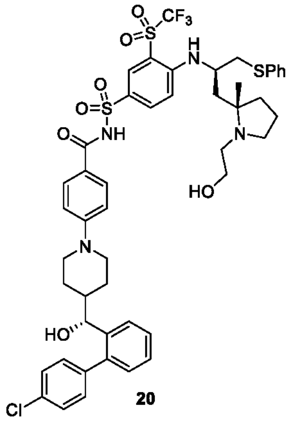 **20** |
| | 4-(4-((*R*)-(4'-chloro-[1,1'-biphenyl]-2-yl)(hydroxy)methyl)piperidin-1-yl)-*N*-((4-(((*R*)-1-((*S*)-1-(2-hydroxyethyl)-2-methylpyrrolidin-2-yl)-3-(phenylt hio)propan-2-yl)amino)-3-((trifluoromethyl)sulfonyl)phenyl)sulfonyl)benz amide **20** |
| 2-21 | 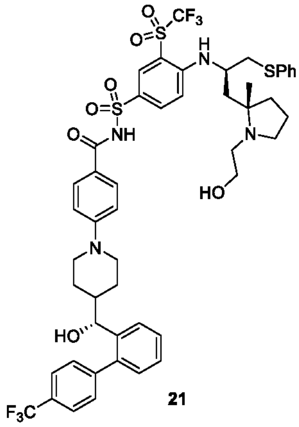 **21** |
| | 4-(4-((*R*)-hydroxy(4'-(trifluoromethyl)-[1,1'-biphenyl]-2-yl)methyl)piperidi n-1-yl)-*N*-((4-(((*R*)-1-((*S*)-1-(2-hydroxyethyl)-2-methylpyrrolidin-2-yl)-3-(phenylthio)propan-2-yl)amino)-3-((trifluoromethyl)sulfonyl)phenyl)sulfon yl)benzamide **21** |
| 22 | 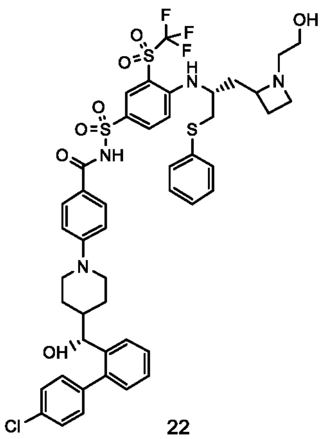 **22** |
| | 4-(4-((*R*)-(4'-chloro-[1,1'-biphenyl]-2-yl)(hydroxy)methyl)piperidin-1-yl)-*N*-((4-(((2*R*)-1-(1-(2-hydroxyethyl)azetidin-2-yl)-3-(phenylthio)propan-2-y l)amino)-3-((trifluoromethyl)sulfonyl)phenyl)sulfonyl)benzamide **22** |

(continued)

| Examples | Structure |
|---|---|
| 23 | 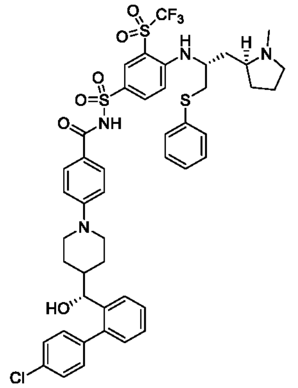 |
| | 4-(4-((*R*)-(4'-chloro-[1,1'-biphenyl]-2-yl)(hydroxy)methyl)piperidin-1-yl)-*N*-((4-(((*R*)-1-((*S*)-1-methylpyrrolidin-2-yl)-3-(phenylthio)propan-2-yl)ami no)-3-((trifluoromethyl)sulfonyl)phenyl)sulfonyl) benzamide **23** |

or the pharmaceutically acceptable salt thereof.

The present disclosure further provides a compound of general formula (DA1) or (DB1) or a pharmaceutically acceptable salt thereof,

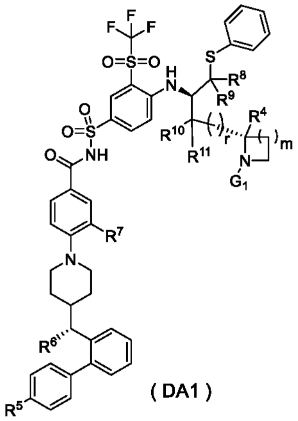

( DA1 )

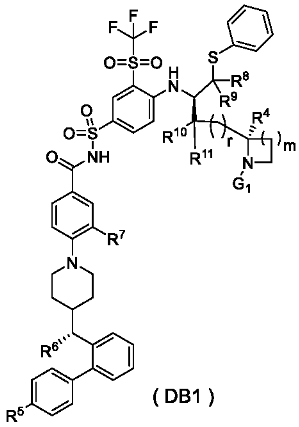

( DB1 )

wherein:

$G_1$ is an amino protecting group, preferably Boc;
m is selected from the group consisting of 0, 1, 2 and 3; and
r and $R^4$-$R^{11}$ are as defined in general formula (D).

In some other embodiments of the present disclosure, provided is the compound of general formula (DA1) or (DB1) or the pharmaceutically acceptable salt thereof, which is selected from any one of the following compounds:

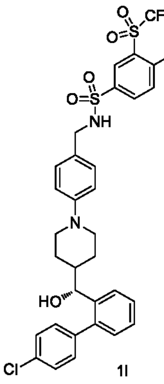
1l
,
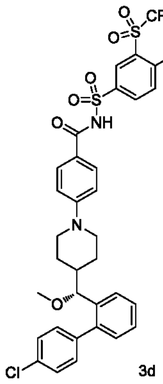
3d
,
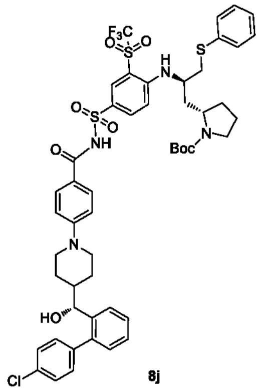
8j

and

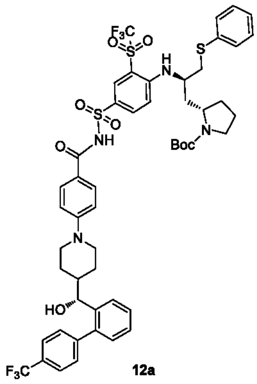
12a
.

The present disclosure further provides a method for preparing a compound of general formula (D-III) or a pharmaceutically acceptable salt thereof, which comprises:

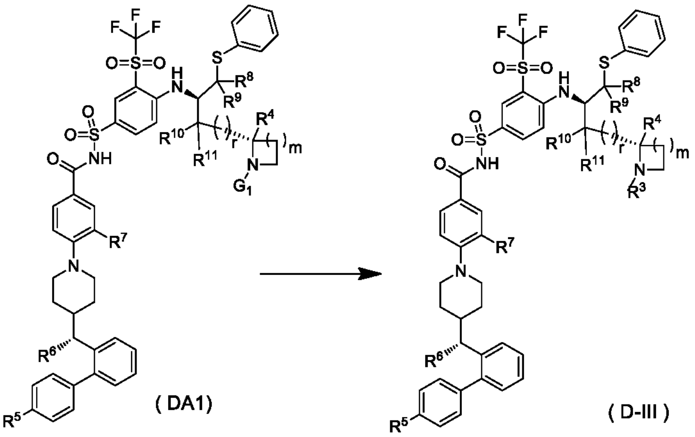
( DA1 ) ( D-III )

subjecting a compound of general formula (DA1) or a pharmaceutically acceptable salt thereof to a deprotection reaction under an acidic condition to obtain the compound of general formula (D-III) or the pharmaceutically acceptable salt thereof; preferably, a reagent used in the acidic condition being HCl;
wherein:

$G_1$ is an amino protecting group, preferably Boc;
m is selected from the group consisting of 0, 1, 2 and 3;
$R^3$ is hydrogen; and
r and $R^4$-$R^{11}$ are as defined in general formula (D).

The present disclosure further provides a compound of general formula (DA2) or (DB2) or a pharmaceutically acceptable salt thereof,

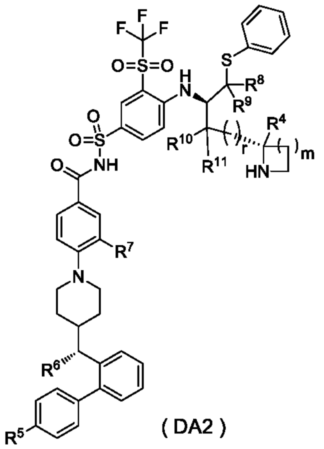

( DA2 )

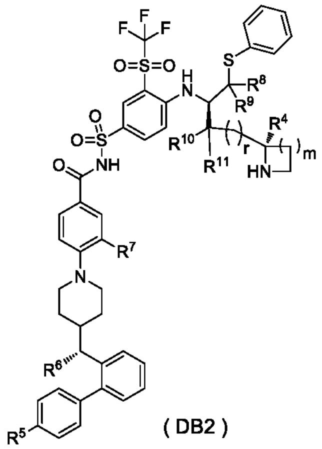

( DB2 )

wherein:

m is selected from the group consisting of 0, 1, 2 and 3; and
r and $R^4$-$R^{11}$ are as defined in general formula (D).

In some other embodiments of the present disclosure, provided is the compound of general formula (DA2) or (DB2) or the pharmaceutically acceptable salt thereof, which is selected from any one of the following compounds:

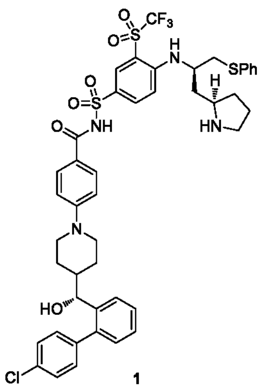

1 ,

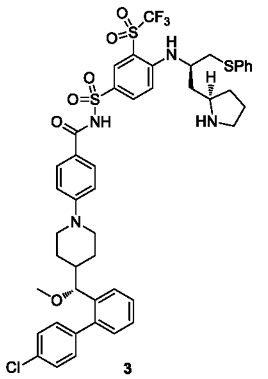

3 ,

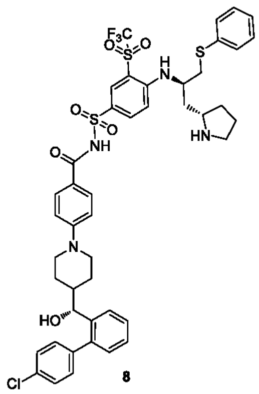

8

and

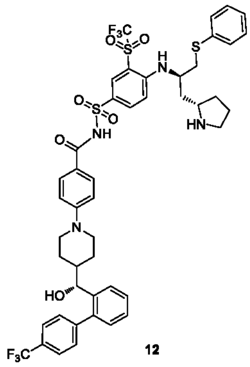

12 .

The present disclosure further provides a method for preparing a compound of general formula (D-III) or a pharmaceutically acceptable salt thereof, which comprises:

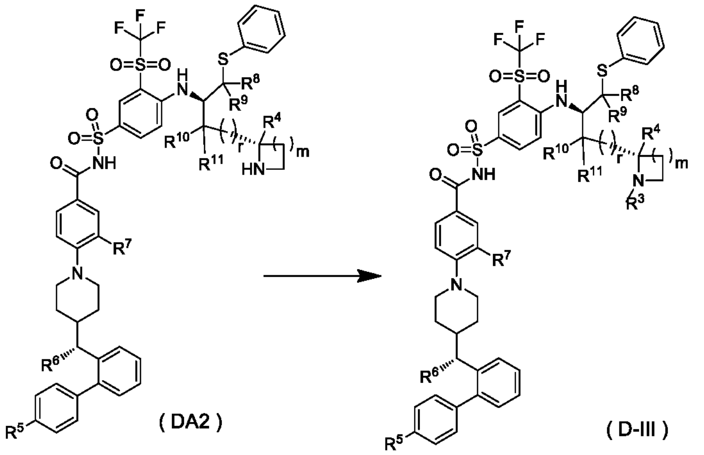

( DA2 ) ( D-III )

subjecting a compound of general formula (DA2) or a pharmaceutically acceptable salt thereof to a substitution reaction under an alkaline condition to obtain the compound of general formula (D-III) or the pharmaceutically acceptable salt thereof; preferably, a reagent used in the alkaline condition being triethylamine;
wherein:

m is selected from the group consisting of 0, 1, 2 and 3;
$R^3$ is hydroxyalkyl; and
r and $R^4$-$R^{11}$ are as defined in general formula (D).

The present disclosure further provides a compound of general formula (DA3), (DB3) or (DC3) or a pharmaceutically acceptable salt thereof,

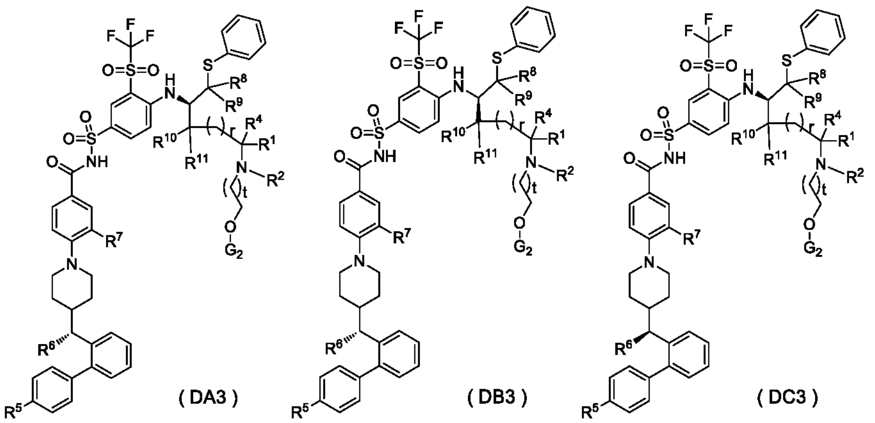

( DA3 ) ( DB3 ) ( DC3 )

wherein:

$G_2$ is a hydroxy protecting group, preferably TBS;
t is selected from the group consisting of 0, 1, 2 and 3; and
r, $R^1$, $R^2$, and $R^4$-$R^{11}$ are as defined in general formula (D).

In some other embodiments of the present disclosure, provided is the compound of general formula (DA3), (DB3) or (DC3) or the pharmaceutically acceptable salt thereof, which is selected from any one of the following compounds:

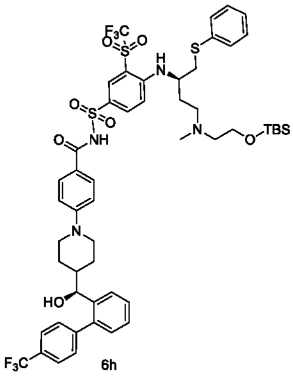

6h ,

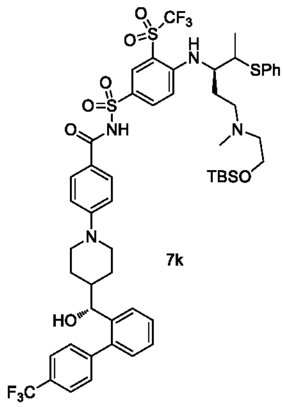

7k

and

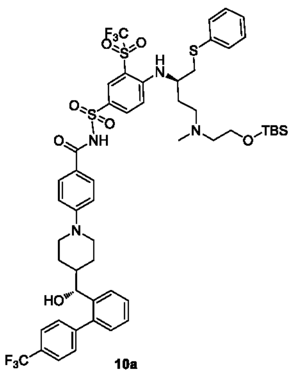

10a .

The present disclosure further provides a method for preparing a compound of general formula (D-VI) or a pharmaceutically acceptable salt thereof, which comprises:

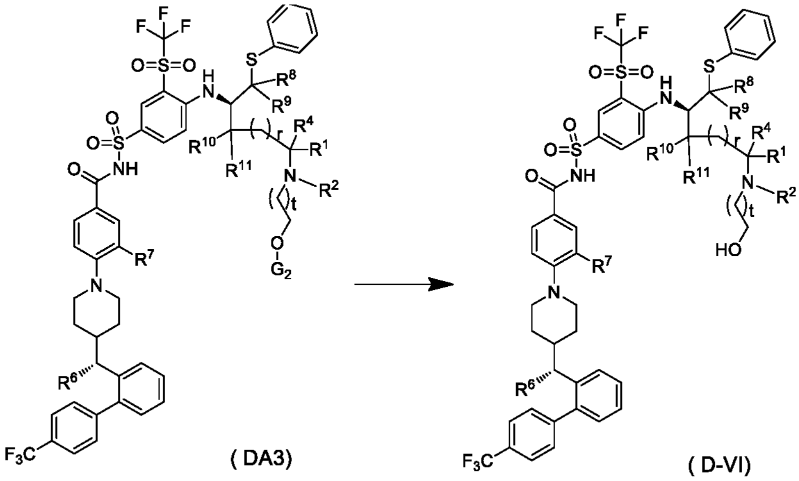

(DA3) (D-VI)

subjecting a compound of general formula (DA3) or a pharmaceutically acceptable salt thereof to a deprotection reaction under an alkaline condition to obtain the compound of general formula (D-VI) or the pharmaceutically acceptable salt thereof; and a reagent used in the alkaline condition being tetrabutylammonium fluoride;
wherein:

$G_2$ is a hydroxy protecting group, preferably TBS;
t is selected from the group consisting of 0, 1, 2 and 3; and
r, $R^1$, $R^2$, and $R^4$-$R^{11}$ are as defined in general formula (D).

The present disclosure further provides a ligand-drug conjugate comprising a structure of formula (-D) or a pharmaceutically acceptable salt thereof:

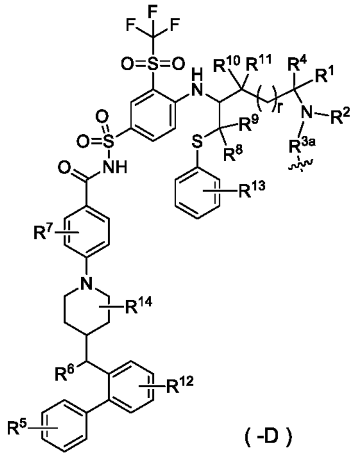

( -D )

wherein:

$R^1$ is selected from the group consisting of deuterium, hydrogen, alkyl and deuterated alkyl;
$R^2$ is selected from the group consisting of alkyl and hydroxyalkyl;
or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;
$R^{3a}$ is selected from the group consisting of a bond and $-(CH_2)_t-CH_2-O-$; the alkyl end being linked to the N atom of the drug moiety, and the -O- end being linked to the linker;
$R^4$ is selected from the group consisting of hydrogen, halogen, deuterated alkyl and alkyl;
$R^5$ is selected from the group consisting of halogen and haloalkyl;
$R^6$ is selected from the group consisting of alkyl, amino, hydroxy and alkoxy;
$R^7$ is selected from the group consisting of hydrogen, halogen, alkyl, deuterated alkyl, hydroxy and alkoxy;
$R^8$ and $R^9$ are each independently selected from the group consisting of hydrogen, alkyl, deuterated alkyl and cycloalkyl; or $R^8$ and $R^9$, together with the atom to which they are attached, form cycloalkyl;
$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;
$R^{12}$ is selected from the group consisting of hydrogen, deuterium, deuterated alkyl, halogen, hydroxy, alkyl, hydroxyalkyl, alkoxy and cycloalkyl;
$R^{13}$ is selected from the group consisting of hydrogen, deuterium, deuterated alkyl, halogen, hydroxy, alkyl, hydroxyalkyl, alkoxy and cycloalkyl;
$R^{14}$ is selected from the group consisting of hydrogen, deuterium, deuterated alkyl, halogen, hydroxy, alkyl, hydroxyalkyl, alkoxy and cycloalkyl;
r is selected from the group consisting of 0, 1, 2 and 3;
t is selected from the group consisting of 0, 1, 2 and 3; and
the wavy line indicates covalent linking to a linker unit or to a ligand.

In some other embodiments of the present disclosure, the ligand-drug conjugate comprising the structure of formula (-D) or the pharmaceutically acceptable salt thereof, wherein the formula (-D) is selected from the group consisting of structures of formulae (-DI) and (-DII):

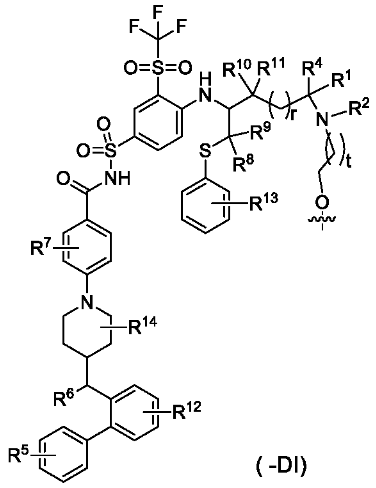

( -DI)

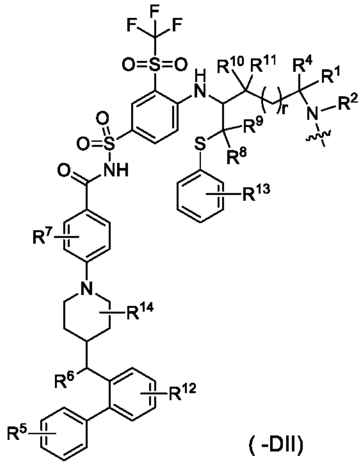

( -DII) ;

wherein t is selected from the group consisting of 0, 1, 2 and 3; and the wavy line, r, $R^1$, $R^2$, and $R^4$-$R^{14}$ are as defined in general formula (-D).

In some other embodiments of the present disclosure, provided is the ligand-drug conjugate comprising the structure of formula (-D) or the pharmaceutically acceptable salt thereof, wherein the formula (-D) is selected from the group consisting of structures of formulae (-DIII), (-DIV) and (-DV):

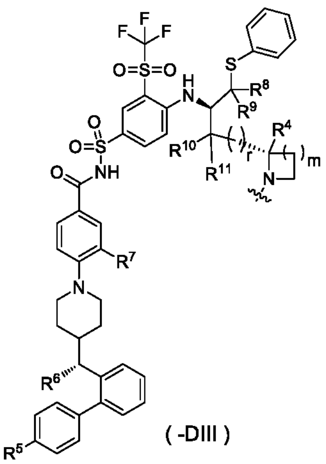

( -DIII )

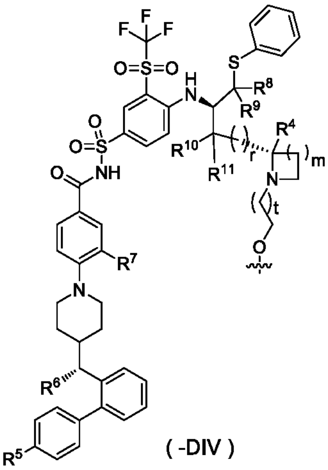

( -DIV )

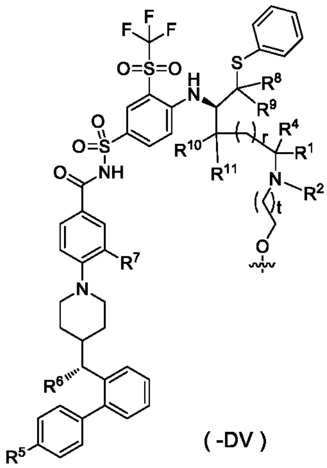

( -DV )

wherein:

t is selected from the group consisting of 0, 1, 2 and 3;
m is selected from the group consisting of 0, 1, 2 and 3; and
the wavy line, r, $R^1$, $R^2$, and $R^4$-$R^{11}$ are as defined in general formula (-D);
preferably, in the formula (-DV), $R^1$ and $R^2$ do not form heterocyclyl.

In some other embodiments of the present disclosure, provided is the ligand-drug conjugate comprising the structure of formula (-D) or the pharmaceutically acceptable salt thereof, wherein the formula (-D) is selected from the group consisting of structures of formulae (-DVI) and (-DVII):

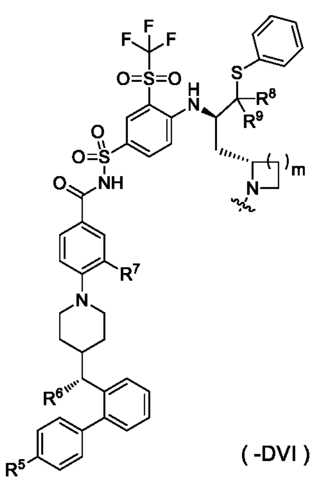
( -DVI )

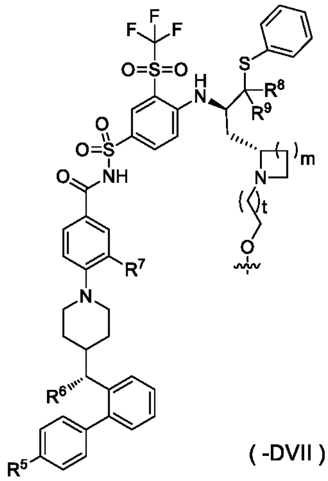
( -DVII )

wherein:

t is selected from the group consisting of 0, 1, 2 and 3;
m is selected from the group consisting of 0, 1, 2 and 3; and
the wavy line, r, and $R^5$-$R^9$ are as defined in general formula (-D).

In some other embodiments of the present disclosure, provided is the ligand-drug conjugate comprising the structure of formula (-D) or the pharmaceutically acceptable salt thereof, which is a ligand-drug conjugate of general formula (Pc-L-D) or a pharmaceutically acceptable salt thereof,

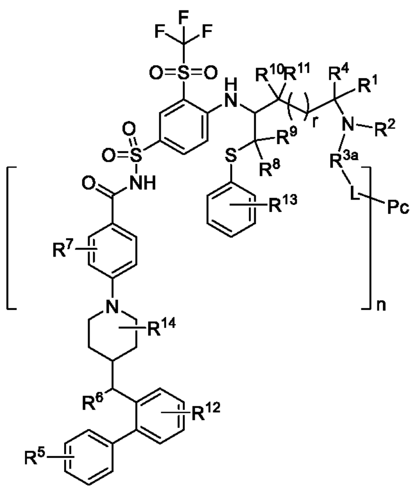
( Pc-L-D )

wherein:

$R^1$ is selected from the group consisting of deuterium, hydrogen, alkyl and deuterated alkyl;
$R^2$ is selected from the group consisting of alkyl and hydroxyalkyl;
or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;
$R^{3a}$ is selected from the group consisting of a bond and $-(CH_2)_t-CH_2-O-$;
$R^4$ is selected from the group consisting of hydrogen, halogen, deuterated alkyl and alkyl;
$R^5$ is selected from the group consisting of halogen and haloalkyl;
$R^6$ is selected from the group consisting of alkyl, amino, hydroxy and alkoxy;
$R^7$ is selected from the group consisting of hydrogen, halogen, alkyl, deuterated alkyl, hydroxy and alkoxy;
$R^8$ and $R^9$ are each independently selected from the group consisting of hydrogen, alkyl, deuterated alkyl and cycloalkyl; or $R^8$ and $R^9$, together with the atom to which they are attached, form cycloalkyl;

$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;

$R^{12}$ is selected from the group consisting of hydrogen, deuterium, deuterated alkyl, halogen, hydroxy, alkyl, hydroxyalkyl, alkoxy and cycloalkyl;

$R^{13}$ is selected from the group consisting of hydrogen, deuterium, deuterated alkyl, halogen, hydroxy, alkyl, hydroxyalkyl, alkoxy and cycloalkyl;

$R^{14}$ is selected from the group consisting of hydrogen, deuterium, deuterated alkyl, halogen, hydroxy, alkyl, hydroxyalkyl, alkoxy and cycloalkyl;

r is selected from the group consisting of 0, 1, 2 and 3;

t is selected from the group consisting of 0, 1, 2 and 3;

n is an integer or a decimal from 1 to 10; and

Pc is a ligand; and L is a linker unit.

In some other embodiments of the present disclosure, provided is the ligand-drug conjugate comprising the structure of formula (-D) or the pharmaceutically acceptable salt thereof, which is a ligand-drug conjugate of general formula (Pc-L-DIII), (Pc-L-DIV) or (Pc-L-DV) or a pharmaceutically acceptable salt thereof:

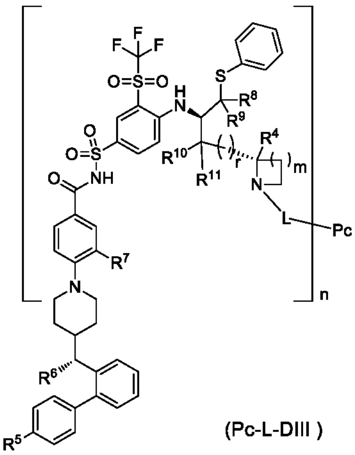
(Pc-L-DIII )

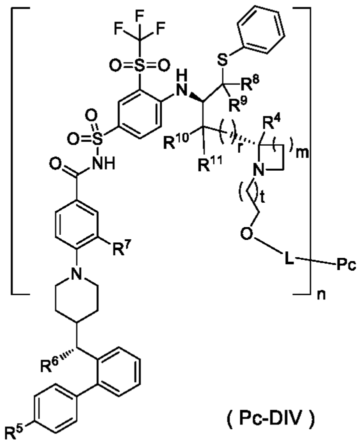
( Pc-DIV )

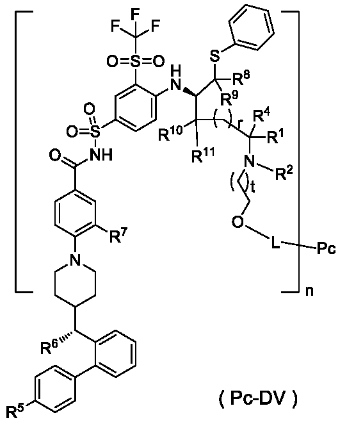
( Pc-DV )

wherein:

t is selected from the group consisting of 0, 1, 2 and 3;

m is selected from the group consisting of 0, 1, 2 and 3; and

r, $R^1$, $R^2$, $R^4$-$R^{11}$, Pc, Land n are as defined in general formula (Pc-L-D).

In some other embodiments of the present disclosure, provided is the ligand-drug conjugate or the pharmaceutically acceptable salt thereof, wherein n is an integer or a decimal from 1 to 8; preferably an integer or a decimal from 2 to 8.

In some other embodiments of the present disclosure, provided is the ligand-drug conjugate or the pharmaceutically acceptable salt thereof, wherein Pc is an antibody.

In some other embodiments of the present disclosure, provided is the ligand-drug conjugate or the pharmaceutically acceptable salt thereof, wherein the antibody is selected from the group consisting of an anti-HER2 (ErbB2) antibody, an anti-Trop-2 antibody, an anti-CD79b antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody and an anti-Mesothelin antibody; preferably Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96 and Glematumamab.

In some other embodiments of the present disclosure, provided is the ligand-drug conjugate or the pharmaceutically acceptable salt thereof, wherein the linker unit -L- is $-L^a-L^b-L^c-$, wherein

$L^a$ is selected from the group consisting of

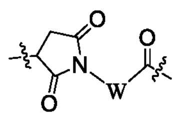, 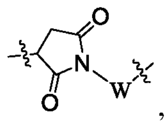, 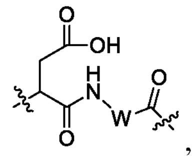,

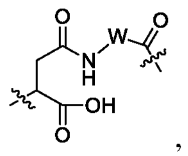, 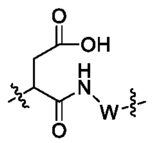 and 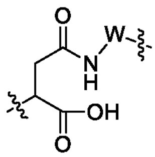,

wherein W is selected from the group consisting of $-C_{1-6}$ alkyl-, $-C_{1-6}$ alkyl-cycloalkyl-, and a linear -heteroalkyl- of 1 to 6 atoms, the heteroalkyl comprising 1 to 3 heteroatoms selected from the group consisting of N, O and S, wherein the $-C_{1-6}$ alkyl-, $-C_{1-6}$ alkyl-cycloalkyl-, or linear -heteroalkyl- of 1 to 6 atoms are each independently and optionally further substituted with one or more substituents selected from the group consisting of halogen, hydroxy, cyano, amino, alkyl, chloroalkyl, deuterated alkyl, alkoxy and cycloalkyl;

$L^b$ is a peptide residue or a chemical bond consisting of 2 to 7 amino acids, wherein the amino acids are optionally substituted with one or more substituents selected from the group consisting of halogen, hydroxy, cyano, amino, alkyl, chloroalkyl, deuterated alkyl, alkoxy and cycloalkyl; and

$L^c$ is selected from the group consisting of $-NR^{17}(CR^{18}R^{19})t-$, $-NH-C(R^{18}R^{19})-O-C(R^{20}R^{21})-C(O)-$, $-NH-R^{22}-(CH_2)_q-OC(O)-$, $-C(O)NR^{17}$, $-C(O)NR^{17}(CH_2)_q-$ and a chemical bond, wherein q is an integer from 1 to 6;

$R^{17}$ is selected from the group consisting of hydrogen, alkyl, haloalkyl, deuterated alkyl and hydroxyalkyl;

$R^{18}$ and $R^{19}$ are identical or different and are each independently selected from the group consisting of hydrogen, halogen, alkyl, haloalkyl, deuterated alkyl and hydroxyalkyl;

$R^{20}$ is selected from the group consisting of alkyl, cycloalkylalkyl and cycloalkyl;

$R^{21}$ is selected from the group consisting of hydrogen, alkyl and haloalkyl;

or, $R^{20}$ and $R^{21}$, together with the carbon atom to which they are attached, form $C_{3-6}$ cycloalkyl;

$R^{22}$ is selected from the group consisting of aryl and heteroaryl.

In some other embodiments of the present disclosure, provided is the ligand-drug conjugate or the pharmaceutically acceptable salt thereof, wherein W is selected from the group consisting of $-(CH_2)_2-$ and $-(CH_2)_5-$.

In some other embodiments of the present disclosure, provided is the ligand-drug conjugate or the pharmaceutically acceptable salt thereof, wherein the peptide residue of $L^b$ is an amino acid residue formed from one or more amino acids selected from the group consisting of phenylalanine, glycine, valine, lysine, citrulline, serine, glutamic acid and aspartic acid; preferably a tetrapeptide residue, a dipeptide residue and a chemical bond; and more preferably a tetrapeptide residue of glycine-glycine-phenylalanine-glycine or a dipeptide residue of valine-citrulline.

In some other embodiments of the present disclosure, provided is the ligand-drug conjugate or the pharmaceutically acceptable salt thereof, wherein the linker unit $L^c$ is selected from the group consisting of $-NH-C(R^{18}R^{19})-O-C(R^{20}R^{21})-C(O)-$, $-NH-R^{22}-(CH_2)_q-OC(O)-$ and a chemical bond, q is an integer from 1 to 6; and $R^{22}$ is selected from the group consisting of aryl and heteroaryl;

preferably, $L^c$ is selected from the group consisting of the following structural formulae:

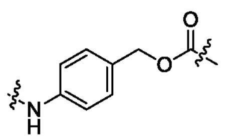 and 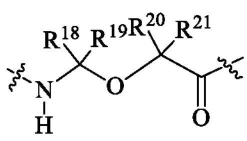;

$R^{18}$ and $R^{19}$ are identical or different and are each independently selected from the group consisting of hydrogen, halogen, alkyl, haloalkyl, deuterated alkyl and hydroxyalkyl;

$R^{20}$ is selected from the group consisting of alkyl, cycloalkylalkyl and cycloalkyl;

$R^{21}$ is selected from the group consisting of hydrogen, alkyl and haloalkyl;

or, $R^{20}$ and $R^{21}$, together with the carbon atom to which they are attached, form $C_{3-6}$ cycloalkyl.

In some other embodiments of the present disclosure, provided is the ligand-drug conjugate or the pharmaceutically acceptable salt thereof, wherein the linker unit -L- is $-L^a-$, wherein $-L^a-$ is as defined above.

In some other embodiments of the present disclosure, provided is the ligand-drug conjugate or the pharmaceutically acceptable salt thereof, wherein the linker unit -L- is $-L^a-L^b-L^c-$, wherein

$L^a$ is selected from the group consisting of

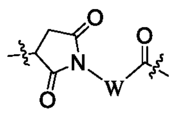,
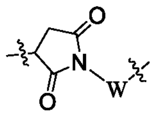,
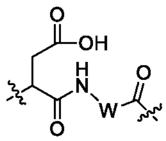,
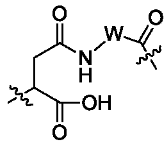,
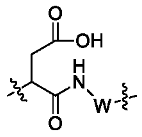 and
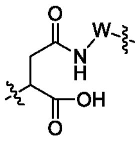,

wherein W is selected from the group consisting of $-(CH_2)_2-$ and $-(CH_2)_5-$;
$L^b$ is selected from the group consisting of a tetrapeptide residue and a dipeptide residue; preferably a tetrapeptide residue of glycine-glycine-phenylalanine-glycine or a dipeptide residue of valine-citrulline; and
$L^c$ is selected from the group consisting of the following structural formulae:

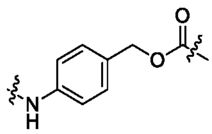 and
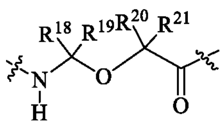;

$R^{18}$ and $R^{19}$ are identical or different and are each independently selected from the group consisting of hydrogen, halogen, alkyl, haloalkyl, deuterated alkyl and hydroxyalkyl;
$R^{20}$ is selected from the group consisting of alkyl, cycloalkylalkyl and cycloalkyl;
$R^{21}$ is selected from the group consisting of hydrogen, alkyl and haloalkyl;
or, $R^{20}$ and $R^{21}$, together with the carbon atom to which they are attached, form $C_{3-6}$ cycloalkyl.

In some other embodiments of the present disclosure, provided is the ligand-drug conjugate or the pharmaceutically acceptable salt thereof, which comprises a linking unit -L-, wherein -L- is selected from the group consisting of:

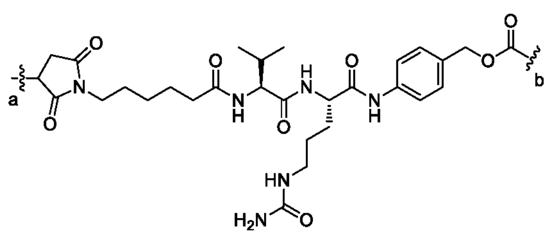,

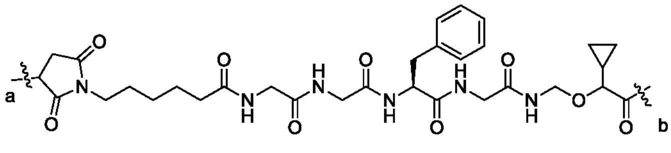,

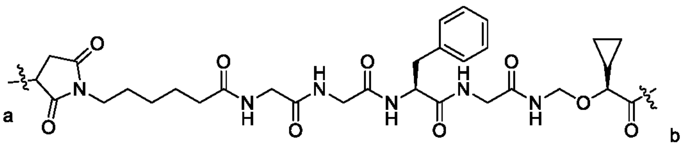,

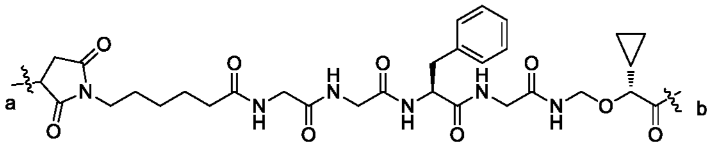,

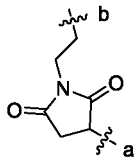, 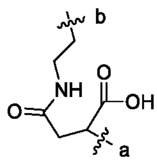, and 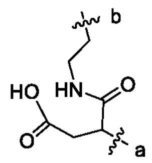;

wherein the a end is linked to a ligand Pc, and the b end is linked to a drug end $R^{3a}$.

In some other embodiments of the present disclosure, provided is the ligand-drug conjugate or the pharmaceutically acceptable salt thereof, wherein the linker unit -L- is $-L^a-L^b-L^c-$, wherein

$L^a$ is selected from the group consisting of

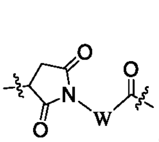, 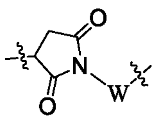, 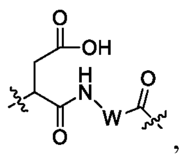,

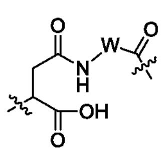, 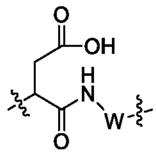 and 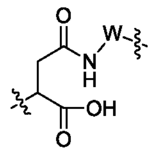,

wherein W is selected from the group consisting of $-(CH_2)_2-$ and $-(CH_2)_5-$;
$L^b$ is selected from the group consisting of a tetrapeptide residue and a dipeptide residue; preferably a tetrapeptide residue of glycine-glycine-phenylalanine-glycine or a dipeptide residue of valine-citrulline; and
$L^c$ is of the following structural formula:

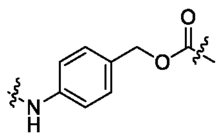.

The present disclosure further provides a compound of general formula (Lu-D) or a pharmaceutically acceptable salt thereof:

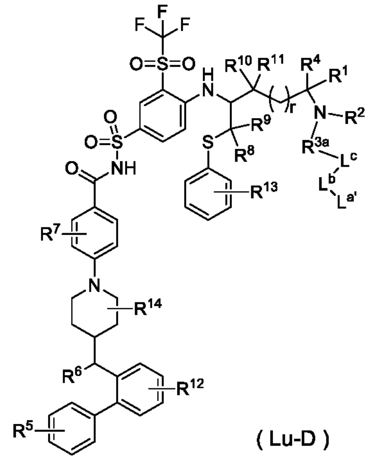

( Lu-D )

wherein:

$L^{a'}$ is selected from the group consisting of

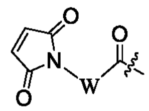 and 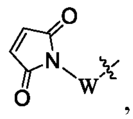,

wherein W is selected from the group consisting of $-(CH_2)_2-$ and $-(CH_2)_5-$;

$L^b$ is a peptide residue or a chemical bond consisting of 2 to 7 amino acids, wherein the amino acids are optionally substituted with one or more substituents selected from the group consisting of halogen, hydroxy, cyano, amino, alkyl, chloroalkyl, deuterated alkyl, alkoxy and cycloalkyl; and

$L^c$ is of the following structural formula:

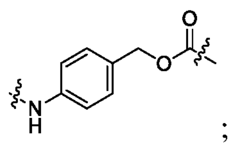

;

$R^1$ is selected from the group consisting of deuterium, hydrogen, alkyl and deuterated alkyl;

$R^2$ is selected from the group consisting of alkyl and hydroxyalkyl;

or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;

$R^{3a}$ is selected from the group consisting of a bond and $-(CH_2)_t-CH_2-O-$;

$R^4$ is selected from the group consisting of hydrogen, halogen, deuterated alkyl and alkyl;

$R^5$ is selected from the group consisting of halogen and haloalkyl;

$R^6$ is selected from the group consisting of alkyl, amino, hydroxy and alkoxy;

$R^7$ is selected from the group consisting of hydrogen, halogen, alkyl, deuterated alkyl, hydroxy and alkoxy;

$R^8$ and $R^9$ are each independently selected from the group consisting of hydrogen, alkyl, deuterated alkyl and cycloalkyl; or $R^8$ and $R^9$, together with the atom to which they are attached, form cycloalkyl;

$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;

$R^{12}$ is selected from the group consisting of hydrogen, deuterium, deuterated alkyl, halogen, hydroxy, alkyl, hydroxyalkyl, alkoxy and cycloalkyl;

$R^{13}$ is selected from the group consisting of hydrogen, deuterium, deuterated alkyl, halogen, hydroxy, alkyl, hydroxyalkyl, alkoxy and cycloalkyl;

$R^{14}$ is selected from the group consisting of hydrogen, deuterium, deuterated alkyl, halogen, hydroxy, alkyl, hydroxyalkyl, alkoxy and cycloalkyl;

r is selected from the group consisting of 0, 1, 2 and 3; and

t is selected from the group consisting of 0, 1, 2 and 3.

In some other embodiments of the present disclosure, provided is the compound of general formula (Lu-D) or the pharmaceutically acceptable salt thereof, which is a compound as shown below:

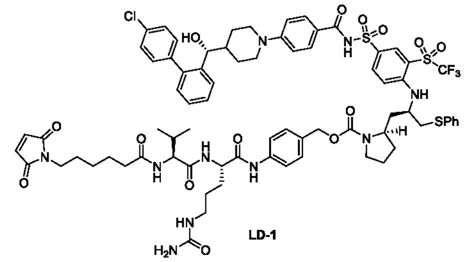

LD-1

,

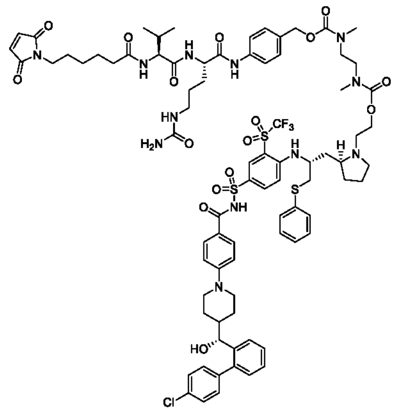

,

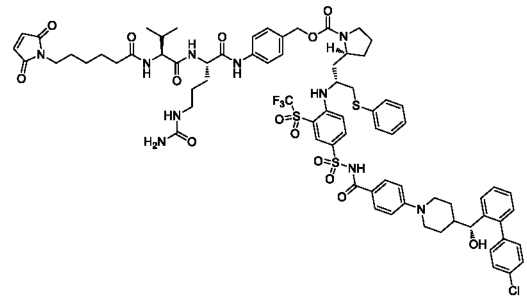

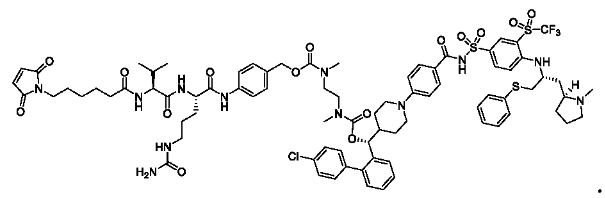

.

and

The present disclosure further provides a pharmaceutical composition comprising a therapeutically effective amount of the compound of general formula (D) or the pharmaceutically acceptable salt thereof, or the ligand-drug conjugate comprising the structure of formula (-D) or the pharmaceutically acceptable salt thereof, or the ligand-drug conjugate of general formula (Pc-L-D) or the pharmaceutically acceptable salt thereof, and a pharmaceutically acceptable carrier, diluent or excipient.

The present disclosure further provides use of the compound of general formula (D) or the pharmaceutically acceptable salt thereof, or the ligand-drug conjugate comprising the structure of formula (-D) or the pharmaceutically acceptable salt thereof, or the ligand-drug conjugate of general formula (Pc-L-D) or the pharmaceutically acceptable salt thereof, or the pharmaceutical composition described above in the preparation of a Bcl-2 and/or Bcl-XL inhibitor.

The present disclosure further provides use of the compound of general formula (D) or the pharmaceutically acceptable salt thereof, or the ligand-drug conjugate comprising the structure of formula (-D) or the pharmaceutically acceptable salt thereof, or the ligand-drug conjugate of general formula (Pc-L-D) or the pharmaceutically acceptable salt thereof, or the pharmaceutical composition described above in the preparation a medicament for treating or preventing a tumor.

The present disclosure further provides use of the compound of general formula (D) or the pharmaceutically acceptable salt thereof, or the ligand-drug conjugate comprising the structure of formula (-D) or the pharmaceutically acceptable salt thereof, or the ligand-drug conjugate of general formula (Pc-L-D) or the pharmaceutically acceptable salt thereof, or the pharmaceutical composition described above in the preparation of a medicament for treating and/or preventing a cancer, wherein the cancer is preferably melanoma, liver cancer, kidney cancer, lung cancer, small cell lung cancer, non-small cell lung cancer, nasopharyngeal cancer, colorectal cancer, colon cancer, rectal cancer, pancreatic cancer, cervical cancer, ovarian cancer, breast cancer, bladder cancer, prostate cancer, leukemia, acute myeloid

leukemia, chronic myeloid leukemia, chronic lymphocytic leukemia, acute lymphocytic leukemia, acute myelogenous leukemia, mantle cell lymphoma, diffuse large B-cell lymphoma, follicular central lymphoma, non-Hodgkin's lymphoma, T cell lymphoma, B cell lymphoma, head and neck squamous cell carcinoma, cervical cancer, thyroid cancer, lymphoma, sarcoma, neuroblastoma, brain tumor, myeloma, astrocytoma, and glioma.

Another aspect of the present disclosure further relates to a method for treating or preventing cancer, which comprises administering a therapeutically effective dose of the compound of general formula (D) or the pharmaceutically acceptable salt thereof, or the ligand-drug conjugate comprising the structure of formula (-D) or the pharmaceutically acceptable salt thereof, or the ligand-drug conjugate of general formula (Pc-L-D) or the pharmaceutically acceptable salt thereof, or the pharmaceutical composition described above; wherein the cancer is preferably melanoma, liver cancer, kidney cancer, lung cancer, small cell lung cancer, non-small cell lung cancer, nasopharyngeal cancer, colorectal cancer, colon cancer, rectal cancer, pancreatic cancer, cervical cancer, ovarian cancer, breast cancer, bladder cancer, prostate cancer, leukemia, acute myeloid leukemia, chronic myeloid leukemia, chronic lymphocytic leukemia, acute lymphocytic leukemia, acute myelogenous leukemia, mantle cell lymphoma, diffuse large B-cell lymphoma, follicular central lymphoma, non-Hodgkin's lymphoma, T cell lymphoma, B cell lymphoma, head and neck squamous cell carcinoma, cervical cancer, thyroid cancer, lymphoma, sarcoma, neuroblastoma, brain tumor, myeloma, astrocytoma, and glioma.

The active compound (e.g., the ligand-drug conjugate or the pharmaceutically acceptable salt thereof according to the present disclosure) may be formulated into a form suitable for administration by any suitable route, preferably in a form of a unit dose, or in a form of a single dose that can be self-administered by a subject. The unit dose of the active compound or composition of the present disclosure may be in a tablet, a capsule, a cachet, a vial, a powder, a granule, a lozenge, a suppository, a powder for reconstitution or a liquid formulation.

The administration dose of the active compound or composition used in the treatment method of the present disclosure will generally vary with the severity of the disease, the weight of the subject, and the efficacy of the active compound. As a general guide, a suitable unit dose may be 0.1-1000 mg.

The pharmaceutical composition of the present disclosure may comprise, in addition to the active compound, one or more auxiliary materials selected from the group consisting of a filler, a diluent, a binder, a wetting agent, a disintegrant, an excipient and the like. Depending on the mode of administration, the composition may comprise 0.1 wt.% to 99 wt.% of the active compound.

The pharmaceutical composition comprising the active ingredient may be in a form suitable for oral administration, for example, in the form of a tablet, a dragee, a lozenge, an aqueous or oil suspension, a dispersible powder or granule, an emulsion, a hard or soft capsule, or a syrup or elixir. Oral compositions can be prepared according to any method known in the art for preparing pharmaceutical compositions. Such oral compositions may comprise a binder, a filler, a lubricant, a disintegrant or a pharmaceutically acceptable wetting agent, and may also comprise one or more ingredients selected from the group consisting of sweetener, corrigent, colorant and preservative, so as to provide a pleasant-to-eye and palatable pharmaceutical formulation.

An aqueous suspension comprises an active substance and an excipient that is used for mixing and suitable for the preparation of the aqueous suspension. The aqueous suspension may also comprise one or more preservatives, one or more colorants, one or more corrigents and one or more sweeteners.

The oil suspension may be formulated by suspending the active ingredient in a vegetable oil. The oil suspension may comprise a thickening agent. The sweeteners and corrigents described above may be added to provide a palatable formulation.

The pharmaceutical compositions may also be prepared as follows: dispersible powders or granules for preparing aqueous suspensions provide the active ingredient, and water is added to mix the active ingredient with one or more of dispersants, wetting agents, suspending agents or preservatives. Other excipients, such as sweeteners, corrigents and colorants, may also be added. Antioxidants such as ascorbic acid are added to preserve these compositions.

The pharmaceutical composition of the present disclosure may also be in the form of an oil-in-water emulsion.

The pharmaceutical composition may be in the form of a sterile injectable aqueous solution. Available and acceptable vehicles or solvents include water, Ringer's solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer solutions and microemulsions in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation

may also be a sterile injection or suspension prepared in a parenterally acceptable non-toxic diluent or solvent. In addition, a sterile fixed oil may be conventionally used as a solvent or a suspending medium.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug. Such materials include cocoa butter, glycerogelatin, hydrogenated vegetable oils, polyethylene glycols of various molecular weights and mixtures of fatty acid esters of polyethylene glycols.

As is well known to those skilled in the art, the dosage of the drug administered depends on a variety of factors, including but not limited to, the activity of the particular compound employed, the age of the subject, the body weight of the subject, the health condition of the subject, the behavior of the subject, the diet of the subject, the time of administration, the route of administration, the rate of excretion, the combination of drugs, and the like. In addition, the optimal treatment regimen, such as the mode of administration, the daily dose of the compound of general formula or the type of pharmaceutically acceptable salts, can be verified according to conventional treatment regimens.

The present disclosure relates to conjugates comprising a targeting (binding) moiety and a therapeutic moiety that is a drug, with improved ability to target different cancer cells, infectious disease organisms, and/or for use in the treatment of autoimmune diseases. The antibody targeting moiety is linked to the compound therapeutic moiety via an intracellularly cleavable linkage that increases the therapeutic efficacy.

For many years, it has been an aim of scientists in the field of specifically targeted drug therapy to use monoclonal antibodies (MAbs) for the specific delivery of toxic agents to human cancers. Conjugates of tumor-associated MAbs with appropriate toxic agents have been developed, but have had mixed success in the treatment of cancer, and little use in other diseases such as infectious diseases and autoimmune diseases. Toxic agents are the most common chemotherapeutic drugs. There is a further need to develop a more effective antibody conjugate with an intracellularly cleavable linker for the treatment of cancer, pathogens, and other diseases.

## Detailed Description of the Invention

Unless defined otherwise, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the commercial product under the trade name, and the drug and active drug component of the commercial product under the trade name.

The term "ligand" refers to a macromolecular compound capable of recognizing and binding to an antigen or a receptor associated with a target cell. The role of the ligand is to present the drug to a target cell population to which the ligand binds, and the ligands include, but are not limited to, protein hormones, lectins, growth factors, antibodies, or other molecules capable of binding to the cells. In the embodiments of the present disclosure, the ligand is denoted as Pc, and the ligand may form a bond with the linking unit through a heteroatom on the ligand, and is preferably an antibody or an antigen-binding fragment thereof, wherein the antibody is selected from the group consisting of a chimeric antibody, a humanized antibody, a fully human antibody and a murine antibody; preferably a monoclonal antibody.

The term "drug" refers to a cytotoxic drug or an immunomodulator. The cytotoxic drug may have a chemical molecule within the tumor cell that is strong enough to disrupt its normal growth. The cytotoxic drug can kill tumor cells in principle at a sufficiently high concentration; however, due to lack of specificity, the cytotoxic drug can cause apoptosis of normal cells while killing tumor cells, resulting in serious side effects. This term includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes. In such an embodiment of the present disclosure, the drug is denoted as D and may be a BCL2 inhibitor, a BCL-XL inhibitor, or a BCL2/BCL-XL dual-target inhibitor.

The term "linker unit", "linker" or "linker fragment" refers to a chemical structural fragment or bond, which is linked to a ligand at one end and to a drug at the other end, and may also be linked to other linkers and then linked to the drug.

The linker includes a stretcher unit, a spacer unit and an amino acid unit, and may be synthesized by methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research, 52: 127-131(1992); U.S. Patent No. 5,208,020).

The term "ligand-drug conjugate" means that a ligand is linked to a biologically active drug by a stable linking unit. In the present disclosure, "ligand-drug conjugate" is preferably an antibody-drug conjugate (ADC), which means

that a monoclonal antibody or an antibody fragment is linked to a biologically active toxic drug by a stable linking unit.

The three-letter and single-letter codes for amino acids used in the present disclosure are as described in J. biol. chem, 243, p3558 (1968).

The "antibody" described herein is used in the broadest sense herein and includes different antibody structures, including but not limited to, a monoclonal antibody, a polyclonal antibody, a murine antibody, a chimeric antibody, a humanized antibody, a multispecific antibody (e.g., a bispecific antibody), and antigen-binding fragments, as long as they exhibit the desired antigen-binding activity and specificity.

Methods of producing and purifying antibodies and antigen-binding fragments are well known in the art, for example, those described in chapters 5-8 and 15 of Antibodies: A Laboratory Manual, Cold Spring Harbor Press. Likewise, antigen-binding fragments can be prepared by conventional methods. The antibody or the antigen-binding fragment described herein is genetically engineered to contain one or more human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "alkyl" refers to a saturated aliphatic hydrocarbon group that is a linear or branched group containing 1 to 20 carbon atoms, preferably alkyl containing 1 to 12 (e.g., 1, 2, 3, 4, 5, 6, 7, 8, 9, 10, 11 and 12) carbon atoms, more preferably alkyl containing 1 to 10 carbon atoms, and most preferably alkyl containing 1 to 6 carbon atoms. Non-limiting examples include methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various side-chain isomers thereof, and the like. More preferred is a lower alkyl having 1 to 6 carbon atoms, and non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted, and when it is substituted, the substitution with a substituent may be performed at any accessible connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having 2 residues derived by removal of two hydrogen atoms from the same carbon atom or two different carbon atoms of the parent alkane, which is a linear or branched group containing 1 to 20 carbon atoms, preferably an alkylene group containing 1 to 12 (e.g., 1, 2, 3, 4, 5, 6, 7, 8, 9, 10, 11 and 12) carbon atoms, and more preferably an alkylene group containing 1 to 6 carbon atoms. Non-limiting examples of alkylene include, but are not limited to, methylene ($-CH_2-$), 1,1-ethylidene ($-CH(CH_3)-$), 1,2-ethylidene ($-CH_2CH_2$)-, 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$), 1,5-butylidene ($-CH_2CH_2CH_2CH_2CH_2-$), and the like. The alkylene may be substituted or unsubstituted, and when it is substituted, the substitution with a substituent may be performed at any accessible connection site, wherein the substituent is preferably independently and optionally selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "cycloalkylene" refers to a cycloalkyl group having 2 residues derived by removal of two hydrogen atoms from the parent cycloalkyl. The cycloalkyl group is as defined above.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer from 0 to 2), excluding a cyclic portion of -O-O-, -O-S- or -S-S-, and the remaining ring atoms are carbon atoms. The heterocyclyl preferably contains 3 to 12 ring atoms, of which 1 to 4 (e.g., 1, 2, 3 and 4) are heteroatoms; more preferably 3 to 10 ring atoms; more preferably 3 to 8 (e.g., 3, 4, 5, 6, 7 and 8) ring atoms, of which 1 to 3 (e.g., 1, 2 and 3) are heteroatoms; more preferably 3 to 6 ring atoms, of which 1 to 3 are heteroatoms; and most preferably 5 or 6 ring atoms, of which 1 to 3 are heteroatoms. Non-limiting examples of monocyclic heterocyclyl include pyrrolidinyl, piperidinyl, piperazinyl, morpholinyl, thiomorpholinyl, homopiperazinyl, etc. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. These rings may contain one or more double bonds, but none of them has a fully conjugated $\pi$-electron system. Preferably, the spiro heterocyclyl is 6- to 14-membered, and more preferably 7- to 10-membered (e.g., 7-membered, 8-membered, 9-mebered or 10-membered). According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

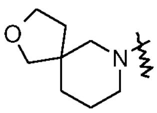 , 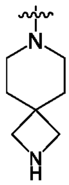 , 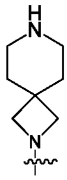 , 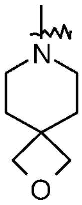 , 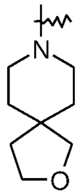 , 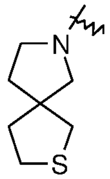 , 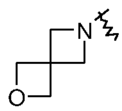 and 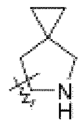 .

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated $\pi$-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the fused heterocyclyl is 6- to 14-membered, and more preferably 7-to 10-membered (e.g., 7-membered, 8-membered, 9-mebered or 10-membered).

According to the number of the formed rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. Non-limiting examples of fused heterocyclyl include:

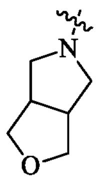 , 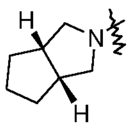 , 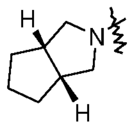 , 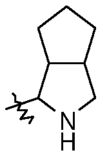 , 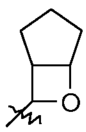 , 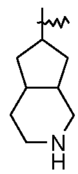 , 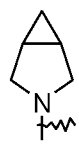 , 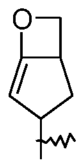 ,

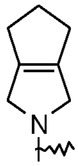 , 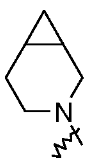 , 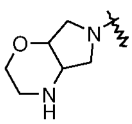 , 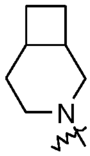 , 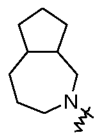 , 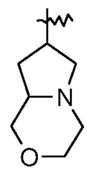 , 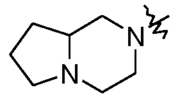

and

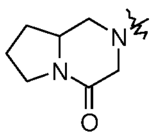.

The term "bridged heterocyclyl" refers to a 5- to 14-membered polycyclic heterocyclyl group in which any two rings share two atoms that are not directly linked to each other, wherein these rings may contain one or more double bonds, but none of them has a fully conjugated π-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the bridged heterocyclyl is 6- to 14-membered, and more preferably 7- to 10-membered (e.g., 7-membered, 8-membered, 9-mebered or 10-membered). According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

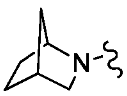, 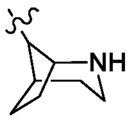, 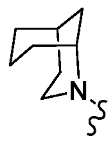, 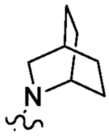, 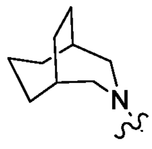, 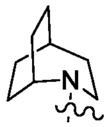, 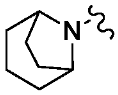,

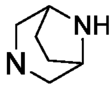 and 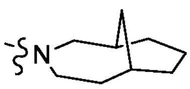.

The heterocyclyl ring may be fused to an aryl, heteroaryl or cycloalkyl ring, wherein the ring attached to the parent structure is heterocyclyl; non-limiting examples include, but are not limited to:

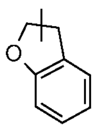, 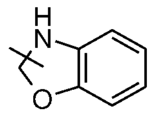, 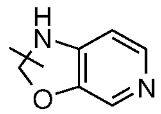, 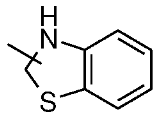,

and the like.

The heterocyclyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heterocyclylene" refers to a heterocyclyl group having 2 residues derived by removal of two hydrogen atoms from identical or different ring atoms of the parent heterocyclyl. The heterocyclyl is as defined above.

The term "aryl" refers to a 6- to 14-membered, preferably 6- to 10-membered, carbon monocyclic or fused polycyclic (i.e., rings sharing a pair of adjacent carbon atoms) group having a conjugated π-electron system, such as phenyl and naphthyl, preferably phenyl. The aryl ring may be fused to a heteroaryl, heterocyclyl or cycloalkyl ring, wherein the ring attached to the parent structure is the aryl ring; non-limiting examples include, but are not limited to:

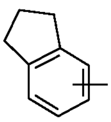, 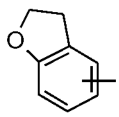, 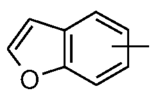, 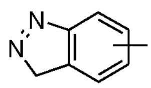, 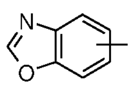, 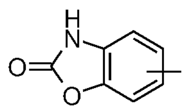,

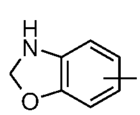, 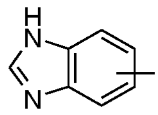, 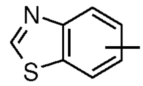, 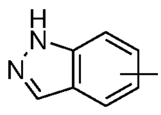, 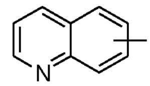, 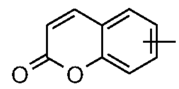

and

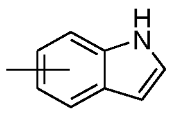.

The aryl may be substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered, more preferably 5- or 6-membered, such as furanyl, thienyl, pyridinyl, pyrrolyl, N-alkylpyrrolyl, pyrimidinyl, pyrazinyl, imidazolyl and tetrazolyl. The heteroaryl ring may be fused to an aryl, heterocyclyl or cycloalkyl ring, wherein the ring attached to the parent structure is heteroaryl; non-limiting examples include, but are not limited to:

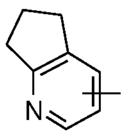, 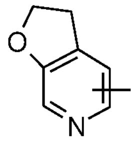, 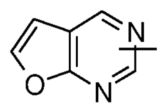, 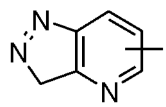, 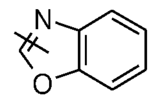, 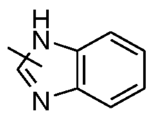,

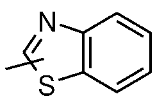, 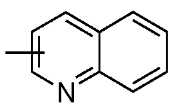, 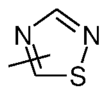 and 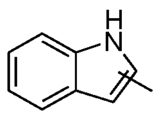.

The heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "amino protecting group" refers to a group that can be readily removed and is intended to protect an amino group from being changed when a reaction is conducted elsewhere in the molecule. Non-limiting examples of the amino protecting group include 9-fluorenylmethoxycarbonyl, *tert*-butoxycarbonyl, acetyl, benzyl, allyl, p-methoxybenzyl, etc. These groups may be optionally substituted with 1 to 3 substituents selected from the group consisting of halogen, alkoxy and nitro. The amino protecting group is preferably 9-fluorenylmethoxycarbonyl.

The term "alkenyl" refers to an alkyl compound containing a carbon-carbon double bond in the molecule, wherein the alkyl is as defined above. The alkenyl may be substituted or unsubstituted, and when it is substituted, the substituent is preferably one or more groups independently selected from one or more substituents of hydrogen, alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing a carbon-carbon triple bond in the molecule, wherein the alkyl is as defined above. The alkynyl may be substituted or unsubstituted, and when it is substituted, the substituent is preferably one or more groups independently selected from one or more substituents of hydrogen, alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkylalkyl" refers to an alkyl group substituted with one or more cycloalkyl groups, preferably one cycloalkyl group, wherein the alkyl is as defined above, and the cycloalkyl is as defined above.

The term "haloalkyl" refers to an alkyl group substituted with one or more halogens, wherein the alkyl is as defined above.

The term "deuterated alkyl" refers to an alkyl group substituted with one or more deuterium atoms, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

The term "acylamino" refers to -C(O)N(alkyl) or -C(O)N(cycloalkyl), wherein the alkyl and cycloalkyl are as defined above.

The term "carboxylate group" refers to -C(O)O(alkyl) or -C(O)O(cycloalkyl), wherein the alkyl and cycloalkyl are as defined above.

In the chemical formula, the abbreviation "Me" refers to methyl.

In the chemical formula, the abbreviation "Ph" refers to phenyl.

The present disclosure also comprises various deuterated forms of the compound of formula (D). Each available hydrogen atom connected to a carbon atom may be independently replaced with a deuterium atom. Those skilled in the art are able to synthesize the deuterated forms of the compound of general formula (D) with reference to the relevant literature. Commercially available deuterated starting materials can be used in preparing the deuterated forms of the compound of formula (D), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. The term "optional" or "optionally" means that the event or circumstance subsequently described may, but not necessarily, occur, and that the description includes instances where the event or circumstance occurs or does not occur. For example, the expression "a heterocyclyl group optionally substituted with alkyl" means that the alkyl may be, but not necessarily, present, and includes instances where the heterocyclyl group is or not substituted with the alkyl.

The term "substituted" means that one or more, preferably up to 5, and more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The pharmaceutical composition is intended to promote the administration to an organism, which facilitates the absorption of the active ingredient, thereby exerting biological activities.

The term "pharmaceutically acceptable salt" refers to a salt of the ligand-drug conjugate of the present disclosure, or a salt of the compound described in the present disclosure. Such salts are safe and effective when used in mammals and possess the required biological activity. The ligand-drug conjugate of the present disclosure at least comprises one amino group and thus may form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and *p*-toluenesulfonate.

The term "drug loading" refers to the mean number of cytotoxic drugs loaded on each ligand in conjugate molecules, and may also be denoted as the drug-to-antibody ratio (DAR). The drug loading may range from 0-12, preferably 1-10, cytotoxic drugs attached to each ligand (Pc). In the embodiments of the present disclosure, the drug loading is denoted as n, and illustratively, may be an average of 1, 2, 3, 4, 5, 6, 7, 8, 9 and 10, ranging from 0 to 12, preferably from 1 to 10, and more preferably from 1 to 8, or from 2 to 8, or from 2 to 7, or from 3 to 8, or from 3 to 7, or from 3 to 6, or from 4 to 7, or from 4 to 6, or from 4 to 5. The drug loading per ADC molecule after the coupling reaction can be characterized by conventional methods such as UV/visible spectroscopy, mass spectrometry, ELISA assays, CE-SDS and HPLC.

The loading of the ligand cytotoxic drug conjugate can be controlled by the following non-limiting methods, including:

(1) controlling a molar ratio of a linking reagent to a ligand,
(2) controlling reaction time and temperature, and
(3) selecting different reaction reagents.

For preparation of conventional pharmaceutical compositions, refer to *Chinese Pharmacopoeia.*

The term "carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, tonicity adjusting agents, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.

The reagents that provides alkaline conditions include organic and inorganic bases, *w*herein the organic bases include, but are not limited to, triethylamine, diethylamine, *N*-methylmorpholine, pyridine, piperidine, *N,N*-diisopropylethylamine, *n*-butyllithium, lithium diisopropylamide, potassium acetate, sodium *tert*-butoxide, tetrabutylammonium fluoride or potassium *tert*-butoxide, and the inorganic bases include, but are not limited to, sodium hydride, potassium phosphate, sodium carbonate, potassium carbonate, cesium carbonate, sodium hydroxide, and lithium hydroxide, preferably *N*,*N*-diisopropylethylamine.

## BRIEF DESCRIPTION OF THE DRAWINGS

FIG. 1: inhibition effect of the compounds of the present disclosure on human acute lymphoblastic leukemia RS4;11 mouse subcutaneous xenograft tumor.

FIG. 2: body weight change caused by inhibition of human acute lymphoblastic leukemia RS4;11 in mouse subcutaneous xenograft tumor.

## DETAILED DESCRIPTION

The following examples further illustrate the present disclosure, but the present disclosure is not limited thereto.

Experimental procedures without conditions specified in the examples of the present disclosure are generally conducted according to conventional conditions, or according to conditions recommended by the manufacturer of the starting materials or commercial products. Reagents without specific origins indicated are commercially available conventional reagents.

I. Synthesis of Compounds

Examples

The structure of the compound is determined by nuclear magnetic resonance (NMR) spectroscopy and/or mass spectrometry (MS). NMR shift ($\delta$) is given in a unit of $10^{-6}$ (ppm). NMR spectra are determined using a Bruker AVANCE NEO 500M nuclear magnetic resonance instrument, with deuterated dimethyl sulfoxide (DMSO-$d_6$), deuterated chloroform ($CDCl_3$) and deuterated methanol ($CD_3OD$) as determination solvents and tetramethylsilane (TMS) as internal standard.

Mass spectra are determined using Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive). High-performance liquid chromatography (HPLC) analysis is performed using the Agilent HPLC 1200DAD, Agilent HPLC 1200VWD and Waters HPLC e2695-2489 high-performance liquid chromatographs.

Chiral HPLC analysis is performed using an Agilent 1260 DAD high-performance liquid chromatograph.

High-performance liquid preparative chromatography is performed using Waters 2545-2767, Waters 2767-SQ

Detecor2, Shimadzu LC-20AP and Gilson GX-281 preparative chromatographs.

Chiral preparative HPLC is performed using a Shimadzu LC-20AP preparative chromatograph.

A CombiFlash rapid preparation instrument used is Combiflash Rf200 (TELEDYNE ISCO).

Huanghai HSGF254 or Qingdao GF254 silica gel plates of specifications 0.15 mm to 0.2 mm are adopted for thin layer chromatography (TLC) analysis and 0.4 mm to 0.5 mm for TLC separation and purification.

Yantai Huanghai silica gel of 200-300 mesh is generally used as a carrier in silica gel column chromatography.

The mean inhibition of kinase and the $IC_{50}$ value are determined using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator or HC2-SS hydrogenator is used in the pressurized hydrogenation reactions.

The hydrogenation reactions usually involve 3 cycles of vacuumization and hydrogen purge.

A CEM Discover-S 908860 microwave reactor is used in the microwave reactions.

In the examples, a solution refers to an aqueous solution unless otherwise specified.

In the examples, the reaction temperature refers to room temperature, i.e., 20 °C to 30 °C, unless otherwise specified.

The monitoring of the reaction progress in the examples is conducted by thin layer chromatography (TLC). The developing solvent for reactions, the eluent system for column chromatography purification and the developing solvent system for thin layer chromatography include: A: dichloromethane/methanol system, and B: n-hexane/ethyl acetate system. The volume ratio of the solvents was adjusted according to the polarity of the compound, or by adding a small amount of basic or acidic reagents such as triethylamine and acetic acid.

Example 2-1

4-(4-((*R*)-(4'-chloro-[1,1'-biphenyl]-2-yl)(hydroxy)methyl)piperidin-1-yl)-*N*-((4-(((*R*)-1-(phenylthio)-3-((*R*)-pyrrolidin-2-yl)propan-2-yl)amino)-3-((trifluoromethyl)sulfonyl)ph enyl)sulfonyl)benzamide **1**

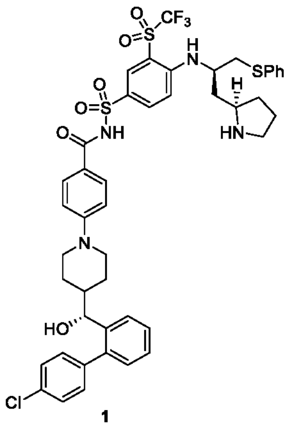

1

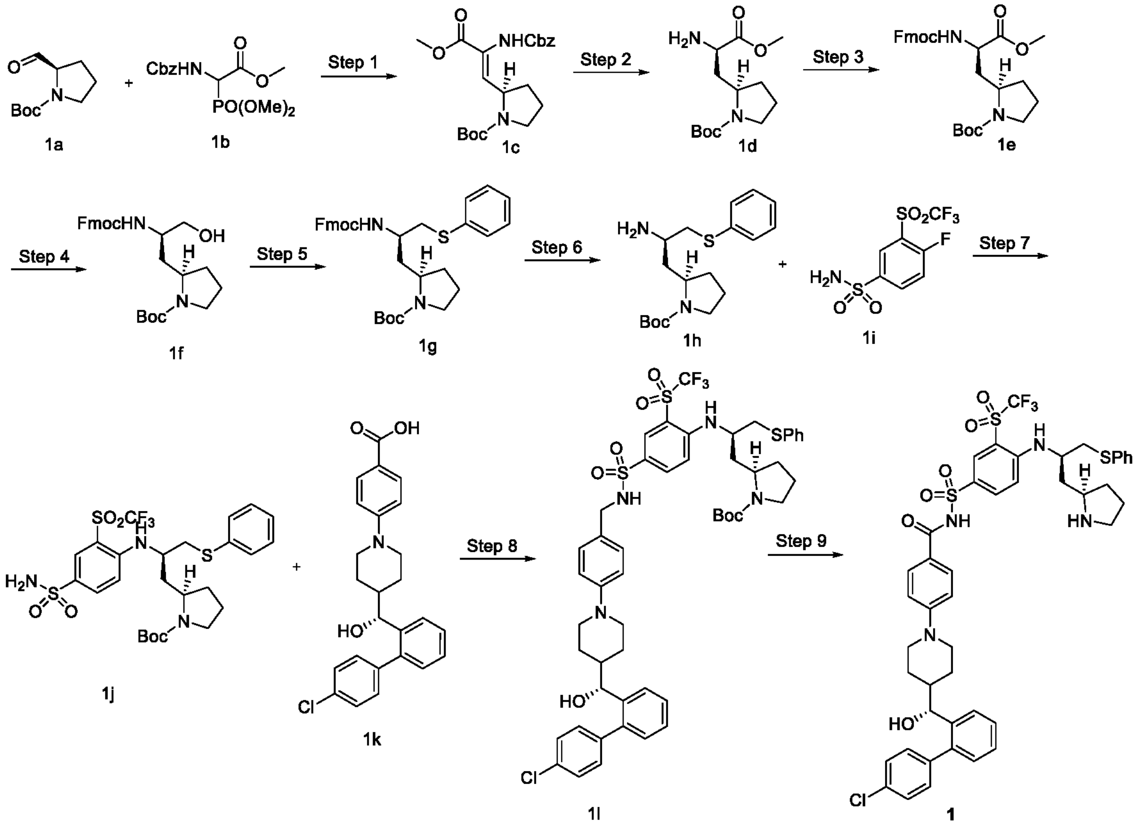


1a 1b 1c 1d 1e 1f 1g 1h 1i 1j 1k 1l 1

Step 1

*Tert*-butyl (*R*,*Z*)-2-(2-(((benzyloxy)carbonyl)amino)-3-methoxy-3-oxoprop-1-en-1-yl)pyrrolidine-1 -carboxylate **1c**

Methyl 2-(((benzyloxy)carbonyl)amino)-2-(dimethoxyphosphoryl)acetate **1b** (1 g, 3.02 mmol, Accela ChemBio) was dissolved in dichloromethane (10 mL), and the solution was cooled to 0 °C under an ice bath. 1,8-diazabicyclo[5.4.0]undec-7-ene (356 mg, 2.34 mmol, Accela ChemBio) was added dropwise, and the mixture was stirred at 0 °C for 20 min, followed by the addition of a solution of *tert-butyl* (R)-2-formylpyrrolidine-1-carboxylate **1a** (500 mg, 2.51 mmol, PharmaBlock) in dichloromethane (5 mL). After the dropwise addition, the ice bath was removed and the mixture was heated to room temperature and reacted for 48 h. The reaction solution was concentrated under reduced pressure. The residue was purified by silica gel column chromatography with an eluent system B to give the title product **1c** (830 g, yield: 81.8%).

MS m/z (ESI): 305.0 [M+H-100].

Step 2

*Tert-butyl* (R)-2-((R)-2-amino-3-methoxy-3-oxopropyl)pyrrolidine-1-carboxylate 1d **1c** (520 mg, 1.28 mmol) was dissolved in isopropanol (10 mL), and palladium on carbon (100 mg, 10% loading, dry basis) was added. The mixture was purged with hydrogen 3 times, and stirred at room temperature for 16 h. The reaction solution was filtered through celite, and the filtrate was concentrated to give the title product **1d** (300 mg, yield: 85.7%), which was directly used in the next step without purification.
MS m/z (ESI): 273.1 [M+1].

Step 3

*Tert-butyl* (*R*)-2-((*R*)-2-((((9*H*-fluoren-9-yl)methoxy)carbonyl)amino)-3-methoxy-3-oxopropyl)pyr rolidine-1-carboxylate **1e**

The crude **1d** (300 mg, 1.10 mmol) was dissolved in 1,4-dioxane (4 mL), and water (1 mL), sodium bicarbonate (278 mg, 3.31 mmol) and fluorenylmethoxycarbonyl chloride (285 mg, 1.10 mmol, Accela ChemBio) were added. The mixture was stirred at room temperature for 1.5 h. Water (10 mL) was added to the reaction solution, and the mixture was extracted with ethyl acetate (10 mL × 3). The organic phases were combined, washed with a saturated sodium chloride solution (20 mL), dried over anhydrous sodium sulfate, and filtered to remove desiccant, and the filtrate was concentrated under reduced pressure. The resulting residue was purified by silica gel column chromatography with an eluent system B to give the title product **1e** (396 mg, yield: 72.7%).
MS m/z (ESI): 395.1 [M+H-100].

Step 4

*Tert-butyl* (*R*)-2-((*R*)-2-((((9*H*-fluoren-9-yl)methoxy)carbonyl)amino)-3-hydroxypropyl)pyrrolidin e-1-carboxylate **1f**

**1e** (200 mg, 0.40 mmol) was dissolved in tetrahydrofuran (10 mL), and ethanol (10 mL) was added, followed by the sequential addition of sodium borohydride (92 mg, 2.43 mmol) and lithium chloride (112 mg, 2.64 mmol). The mixture was stirred at 28 °C for 2.5 h. The reaction solution was cooled under an ice bath, and a saturated ammonium chloride solution (10 mL) was added dropwise to quench the reaction. Water (10 mL) was added, and the mixture was extracted with ethyl acetate (10 mL × 3). The organic phases were combined, washed with a saturated sodium chloride solution (10 mL), dried over anhydrous sodium sulfate and filtered to remove desiccant, and the filtrate was concentrated under reduced pressure. The resulting residue was purified by silica gel column chromatography with an eluent system B to give the title product **1f** (144 mg, yield: 76.3%).
MS m/z (ESI): 367.1 [M+H-100].

Step 5

*Tert-butyl* (*R*)-2-((R)-2-((((9*H*-fluoren-9-yl)methoxy)carbonyl)amino)-3-(phenylthio)propyl)pyrrol idine-1-carboxylate **1g**

**1f** (144 mg, 0.31 mmol) was dissolved in toluene (5 mL), and diphenyl disulfide (225 mg, 1.03 mmol, adamas) and tributylphosphine (208 mg, 1.03 mmol) were added. The reaction system was purged with nitrogen 3 times, and heated to 80 °C and stirred for 12 h. The reaction solution was concentrated under reduced pressure. The resulting residue was purified by silica gel column chromatography with an eluent system B to give the title product **1g** (168 mg, 97.4%).
MS m/z (ESI): 459.1 [M+H-100].

Step 6

*Tert*-butyl (*R*)-2-((*R*)-2-amino-3-(phenylthio)propyl)pyrrolidine-1-carboxylate **1h**

**1g** (168 mg, 0.30 mmol) was dissolved in dichloromethane (2 mL), and diethylamine (2 mL) was added. The mixture was stirred at room temperature for 3 h. The reaction solution was concentrated under reduced pressure to remove the organic solvent, and concentrated with toluene (5 mL) under reduced pressure to give the crude title product **1h** (101 mg), which was directly used in the next step without purification.
MS m/z (ESI): 337.1 [M+1].

Step 7

*Tert-butyl* (*R*)-2-((*R*)-3-(phenylthio)-2-((4-sulfamoyl-2-((trifluoromethyl)sulfonyl)phenyl)amino)pr opyl)pyrrolidine-1-carboxylate **1j**

The crude **1h** (101 mg, 0.30 mmol) was dissolved in *N,N*-dimethylformamide (5 mL), and 4-fluoro-3-((trifluoromethyl)sulfonyl)benzenesulfonamide **1i** (102 mg, 0.33mmol, Bide) and *N,N*-diisopropylethylamine (388 mg, 3.00 mmol) were added. The reaction system was purged with nitrogen 3 times, and heated to 50 °C and stirred for 8 h. Water (10 mL) was added to the reaction solution, and the mixture was extracted with ethyl acetate (10 mL × 3). The organic phases were combined, washed with a saturated sodium chloride solution (20 mL), dried over anhydrous sodium sulfate, and filtered to remove desiccant, and the filtrate was concentrated under reduced pressure. The resulting residue was purified by silica gel column chromatography with an eluent system B to give the title product **1j** (129 mg, 68.9%).

MS m/z (ESI): 524.0 [M+H-100].

Step 8

*Tert-butyl* (*R*)-2-((*R*)-2-((4-(*N*-(4-(4-((*R*)-(4'-chloro-[1,1'-biphenyl]-2-yl)(hydroxy)methyl)piperidi n-1-yl)benzyl)sulfamoyl)-2-((trifluoromethyl)sulfonyl)phenyl)amino)-3-(phenylthio)pro pyl)pyrrolidine-1-carboxylate **1l**

**1j** (22 mg, 0.035 mmol) was dissolved in dichloromethane (3 mL), and **1k** (16 mg, 0.038 mmol, synthesized by referring to the method provided for intermediate 40 on page 79 of the specification in the patent "CN103153954 B") was added, followed by 4-dimethylaminopyridine (8.6 mg, 0.070 mmol) and 1-(3-dimethylaminopropyl)-3-ethylcarbodiimide hydrochloride (20.3 mg, 0.106 mmol). After the addition, the mixture was stirred at room temperature for 4 h under nitrogen atmosphere, supplemented with **1k** (11 mg, 0.026 mmol), and stirred at room temperature overnight after the supplementation. The reaction solution was sequentially washed with water (5 mL) and a saturated sodium chloride solution (5 mL), dried over anhydrous sodium sulfate, and filtered, and the filtrate was concentrated under reduced pressure. The resulting residue was purified by thin layer chromatography with a developing solvent system A to give the title product **1l** (28 mg, yield: 77.2%).

MS m/z (ESI): 1027.0 [M+1].

Step 9

4-(4-((*R*)-(4'-chloro-[1,1'-biphenyl]-2-yl)(hydroxy)methyl)piperidin-1-yl)-*N*-((4-(((*R*)-1-(phenylthio)-3-((R)-pyrrolidin-2-yl)propan-2-yl)amino)-3-((trifluoromethyl)sulfonyl)ph enyl)sulfonyl)benzamide **1**

**1l** (14 mg, 0.014 mmol) was dissolved in dichloromethane (0.2 mL), and a solution of 4 *M* hydrogen chloride in dioxane (0.5 mL) was added. The mixture was stirred at room temperature for 30 min. The reaction solution was concentrated under reduced pressure to remove the organic solvent. The residue was slurried with diethyl ether and filtered. The resulting solid was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **1** (5 mg, yield: 39.6%).

MS m/z (ESI): 927.2 [M+1].

$^1H$ NMR (500 MHz, $CD_3OD$) δ 8.25 (s, 1H), 8.06 (d, 1H), 7.82 (d, 2H), 7.62 (d, 1H), 7.48-7.31 (m, 8H), 7.26 (t, 2H), 7.20 (t, 2H), 6.82 (d, 2H), 6.74 (d, 1H), 4.44 (d, 1H), 4.00-3.95 (m, 1H), 3.78 (d, 1H), 3.61 (d, 1H), 3.52-3.45 (m, 2H), 3.26-3.17 (m, 2H), 2.68 (t, 2H), 2.53 (t, 2H), 2.27-1.92 (m, 6H), 1.81-1.59 (m, 4H), 1.27-1.21 (m, 1H), 1.18-1.12 (m, 1H), 1.02-0.90 (m, 2H).

Example 2-2

4-(4-((*R*)-(4'-chloro-[1,1'-biphenyl]-2-yl)(hydroxy)methyl)piperidin-1-yl)-*N*-((4-(((*R*)-1-((*R*)-1-(2-hydroxyethyl)pyrrolidin-2-yl)-3-(phenylthio)propan-2-yl)amino)-3-((trifluoro methyl)sulfonyl)phenyl)sulfonyl)benzamide **2**

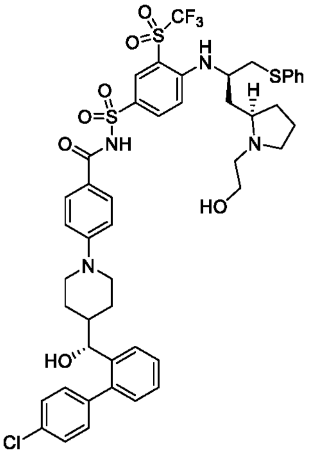

**2**

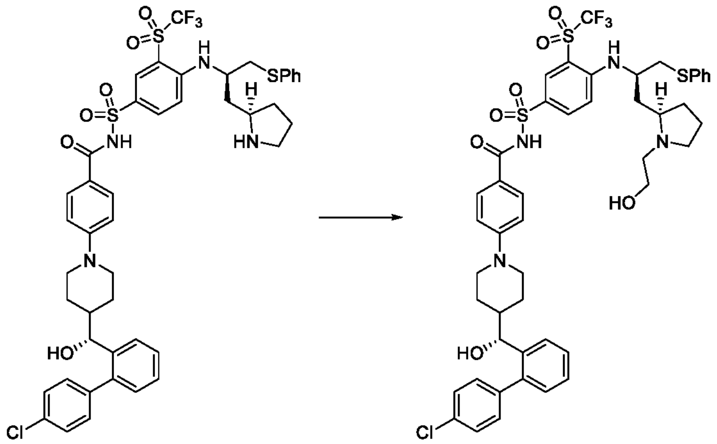

**1** **2**

**1** (13 mg, 0.014 mmol) was placed in a reaction flask, and acetonitrile (5 mL) was added, followed by 2-bromoethanol (26 mg, 0.210 mmol, adamas) and triethylamine (11 mg, 0.112 mmol). The reaction system was purged with nitrogen 3 times, and heated to 70 °C and stirred for 7 h. The reaction solution was concentrated under reduced pressure. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **2** (3 mg, yield: 22.0%).

MS m/z (ESI): 971.2 [M+1].

$^1$H NMR (500 MHz, $CD_3OD$) δ 8.26 (s, 1H), 8.03 (d, 1H), 7.80 (d, 2H), 7.62 (d, 1H), 7.46-7.36 (m, 5H), 7.35-7.27 (m, 5H), 7.24 (d, 1H), 7.18 (d, 1H), 6.81 (d, 2H), 6.63 (d, 1H), 4.42 (d, 1H), 3.91-3.77 (m, 4H),3.63 (d, 2H), 3.25-3.16 (m, 2H), 2.67 (t, 2H), 2.54 (t, 2H), 2.36-2.29 (m, 2H), 2.23-2.13 (m, 3H), 2.09-1.97 (m, 4H),1.81-1.71 (m, 2H), 1.65-1.59 (m, 2H), 1.27-1.21 (m, 1H), 1.17-1.11 (m, 1H), 1.02-0.90 (m, 2H).

Example 2-3

4-(4-((*R*)-(4'-chloro-[1,1'-biphenyl]-2-yl)(methoxy)methyl)piperidin-1-yl)-*N*-((4-(((*R*)-1 -(phenylthio)-3-((*R*)-pyrrolidin-2-yl)propan-2-yl)amino)-3-((trifluoromethyl)sulfonyl)ph enyl)sulfonyl)benzamide **3**

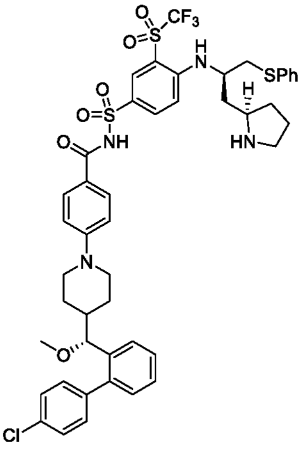

3

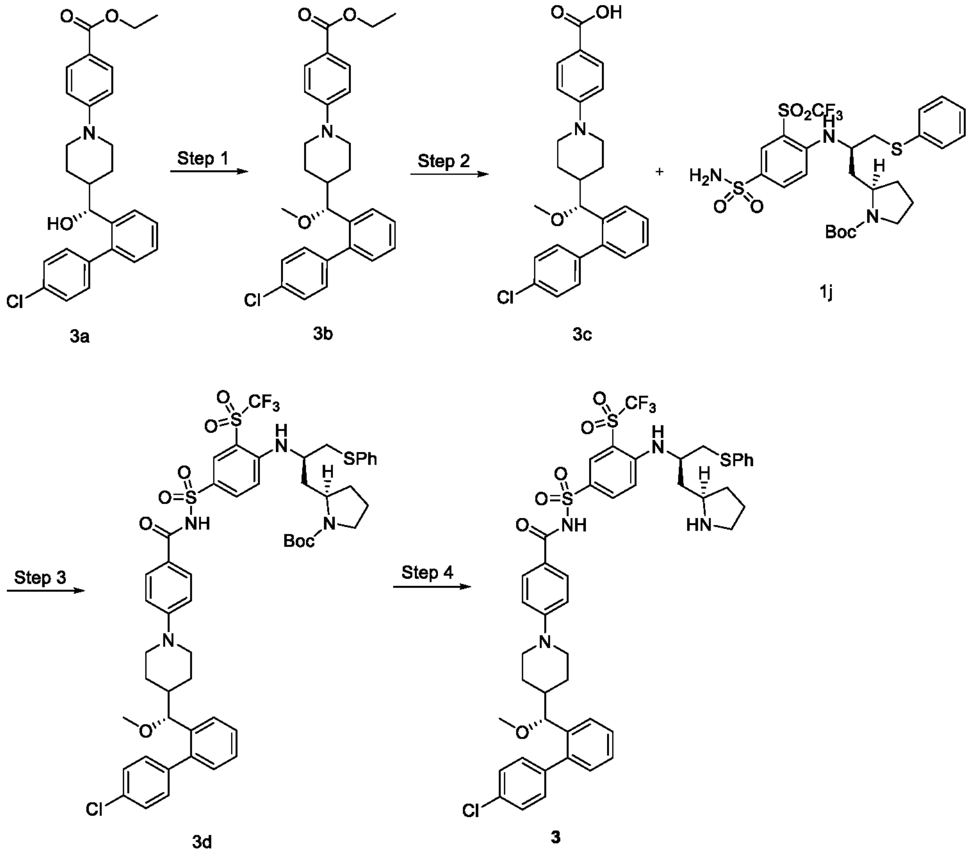


3a 3b 3c 1j

3d 3

Step 1

Ethyl (*R*)-4-(4-((4'-chloro-[1,1'-biphenyl]-2-yl)(methoxy)methyl)piperidin-1-yl)benzoate **3b**

Ethyl (*R*)-4-(4-((4'-chloro-[1,1'-biphenyl]-2-yl)(hydroxy)methyl)piperidin-1-yl)benzoate **3a** (60 mg, 0.133 mmol, synthesized by referring to the method provided for intermediate 11 on page 63 of the specification in the patent "CN103153954 B") was dissolved in 2 mL of a mixed solvent of *N,N*-dimethylformamide and tetrahydrofuran (V:V =1:1). The solution was purged with nitrogen three times, and iodomethane (190 mg, 1.34 mmol) and 60% sodium hydride (11 mg, 0.275 mmol) were sequentially added. The mixture was stirred at room temperature for 3 h. The reaction solution was cooled under an ice bath, and a saturated ammonium chloride solution (5 mL) was added to quench the reaction. Water (5 mL) was added, and the mixture was extracted with ethyl acetate (10 mL × 3). The organic phases were combined, washed with a saturated sodium chloride solution (10 mL), dried over anhydrous sodium sulfate and filtered to remove desiccant, and the filtrate was concentrated under reduced pressure. The resulting residue was purified by silica gel column chromatography with an eluent system B to give the title product **3b** (57 mg, yield: 92.1%).

MS m/z (ESI): 464.1 [M+1].

Step 2

(R)-4-(4-((4'-chloro-[1,1'-biphenyl]-2-yl)(methoxy)methyl)piperidin-1-yl)benzoic acid **3c**

**3b** (57 mg, 0.122 mmol) was dissolved in 3 mL of a mixed solvent of tetrahydrofuran, methanol and water (V:V:V = 4:1:1), and lithium hydroxide monohydrate (26 mg, 0.620 mmol) was added. The mixture was heated to 50 °C and stirred for 16 h. The reaction solution was concentrated under reduced pressure, adjusted to pH 2-3 with 1 *M* hydrochloric acid, and filtered. The filter cake was dissolved in 10 mL of dichloromethane, dried over anhydrous sodium sulfate, and filtered. The filtrate was concentrated under reduced pressure to give the title product **3c** (53 mg, yield: 98.9%). MS m/z (ESI): 436.1 [M+1].

Step 3

*Tert-butyl* (*R*)-2-((*R*)-2-((4-(N-(4-(4-((*R*)-(4'-chloro-[1,1'-biphenyl]-2-yl)(methoxy)methyl)piperidi n-1-yl)benzoyl)sulfamoyl)-2-((trifluoromethyl)sulfonyl)phenyl)amino)-3-(phenylthio)pr opyl)pyrrolidine-1-carboxylate **3d**

**1j** (32 mg, 0.051 mmol) was dissolved in dichloromethane (5 mL), and **3c** (24 mg, 0.055 mmol) was added, followed by 4-dimethylaminopyridine (13 mg, 0.106 mmol) and 1-(3-dimethylaminopropyl)-3-ethylcarbodiimide hydrochloride (30 mg, 0.156 mmol). After the addition, the mixture was stirred at room temperature for 4 h under nitrogen atmosphere, supplemented with **3c** (22 mg, 0.051 mmol), and stirred at room temperature overnight after the supplementation. The reaction solution was sequentially washed with water (10 mL) and a saturated sodium chloride solution (10 mL), dried over anhydrous sodium sulfate, and filtered, and the filtrate was concentrated under reduced pressure. The resulting residue was purified by thin layer chromatography with a developing solvent system A to give the title product **3d** (53 mg, yield: 99.6%).

MS m/z (ESI): 1041.1 [M+1].

Step 4

4-(4-((*R*)-(4'-chloro-[1,1'-biphenyl]-2-yl)(methoxy)methyl)piperidin-1-yl)-*N*-((4-(((*R*)-1 -(phenylthio)-3-((*R*)-pyrrolidin-2-yl)propan-2-yl)amino)-3-((trifluoromethyl)sulfonyl)ph enyl)sulfonyl)benzamide **3**

**3d** (30 mg, 0.028 mmol) was dissolved in dichloromethane (0.4 mL), and a solution of 4 *M* hydrogen chloride in dioxane (1.2 mL) was added. The mixture was stirred at room temperature for 40 min. The reaction solution was concentrated under reduced pressure to remove the organic solvent. The residue was slurried with diethyl ether and filtered. The resulting solid was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **3** (10 mg, yield: 36.9%).

MS m/z (ESI): 941.1 [M+1].

$^1$H NMR (500 MHz, $CD_3OD$) δ 8.26 (s, 1H), 8.04 (dd, 1H), 7.81 (d, 2H), 7.50 (d, 1H), 7.47-7.41 (m, 3H), 7.40-7.32 (m, 3H), 7.30-7.23 (m, 4H), 7.22-7.16 (m, 2H), 6.81 (d, 2H), 6.72 (d, 1H), 4.06 (d, 1H), 3.99-3.93 (m, 1H), 3.77 (d, 1H), 3.66 (d, 1H), 3.55-3.49 (m, 1H), 3.23 (d, 2H), 3.17 (s, 3H), 2.61 (t, 2H), 2.51 (t, 2H), 2.25-1.93 (m, 6H), 1.72-1.58 (m, 4H), 1.27-1.23 (m, 1H), 1.20-1.11 (m, 2H), 0.91 (t, 1H).

Example 2-4

4-(4-((*R*)-(4'-chloro-[1,1'-biphenyl]-2-yl)(methoxy)methyl)piperidin-1-yl)-*N*-((4-(((*R*)-1 -((*R*)-1-(2-hydroxyethyl)pyrrolidin-2-yl)-3-(phenylthio)propan-2-yl)amino)-3-((trifluoro methyl)sulfonyl)phenyl)sulfonyl)benzamide **4**

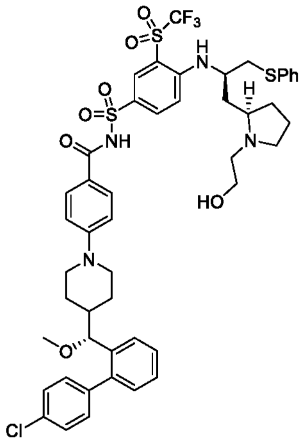

**4**

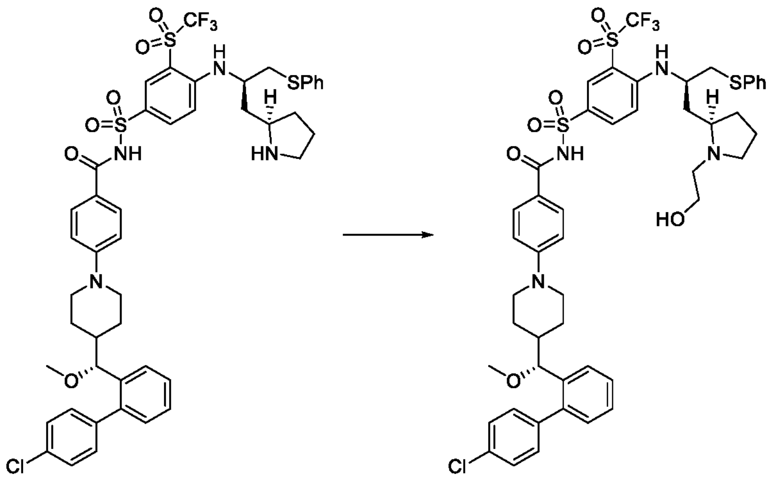

**3** **4**

**3** (15 mg, 0.015 mmol) was placed in a reaction flask, and acetonitrile (5 mL) was added, followed by 2-bromoethanol (29 mg, 0.232 mmol) and triethylamine (16, 0.158 mmol). The reaction system was purged with nitrogen 3 times, and heated to 70 °C and stirred for 16 h. The reaction solution was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **4** (2.5 mg, yield: 16.5%).

MS m/z (ESI): 985.1 [M+1].

$^1$H NMR (500 MHz, $CD_3OD$) $\delta$ 8.27 (s, 1H), 8.03 (dd, 1H), 7.81 (d, 2H), 7.51 (d, 1H), 7.47-7.39 (m, 5H), 7.35 (t, 1H), 7.32-7.21 (m, 5H), 7.18 (d, 1H), 6.82 (d, 2H), 6.63 (d, 1H), 4.06 (d, 1H), 3.90-3.75 (m, 4H), 3.69-3.63 (m, 1H), 3.27-3.20 (m, 2H), 3.17 (s, 3H), 2.62 (t, 2H), 2.52 (t, 2H), 2.36-2.28 (m, 2H), 2.23-2.13 (m, 3H), 2.09-1.94 (m, 4H), 1.78-1.58 (m, 4H), 1.28-1.24 (m, 1H), 1.21-1.13 (m, 2H), 0.91(t, 1H).

Example 2-5

4-(4-((*R*)-(4'-chloro-[1,1'-biphenyl]-2-yl)(hydroxy)methyl)piperidin-1-yl)-*N*-((4-(((*R*)-1-((R)-1-(3-hydroxypropyl)pyrrolidin-2-yl)-3-(phenylthio)propan-2-yl)amino)-3-((trifluor omethyl)sulfonyl)phenyl)sulfonyl)benzamide **5**

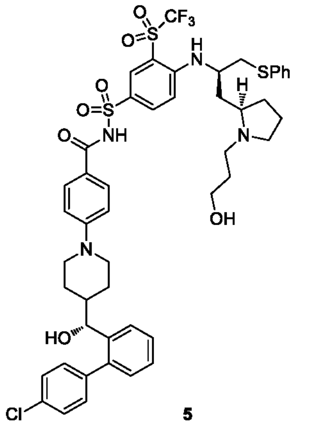

5

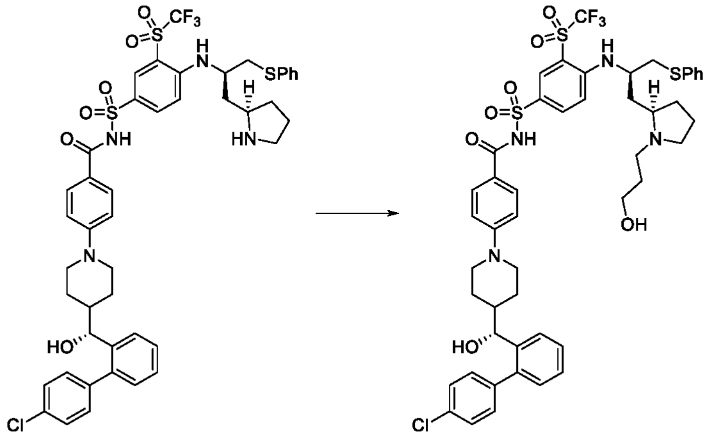

1 5

**1** (10 mg, 0.011 mmol) was placed in a reaction flask, and acetonitrile (3 mL) was added, followed by 3-bromopropanol (15 mg, 0.108 mmol, adamas) and triethylamine (11 mg, 0.108 mmol). The reaction system was purged with nitrogen 3 times, and heated to 70 °C and stirred for 16 h. The reaction solution was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **5** (2.5 mg, yield: 23.5%).

MS m/z (ESI): 985.1 [M+1].

$^1$H NMR (500 MHz, $CD_3OD$) δ 8.26 (s, 1H), 8.03 (d, 1H), 7.81 (d, 2H), 7.61 (d, 1H), 7.46-7.39 (m, 5H), 7.35-7.26 (m, 5H), 7.22 (t, 1H), 7.18 (d, 1H), 6.81 (d, 2H), 6.62 (d, 1H), 4.43 (d, 1H), 3.89-3.84 (m, 1H), 3.79 (d, 1H), 3.67 (t, 2H), 3.63 (d, 2H), 3.25-3.15 (m, 2H), 2.67 (t, 2H), 2.53 (t, 2H), 2.32-2.26 (m, 2H), 2.19 (t, 2H), 2.15-2.11 (m, 1H), 2.08-1.95 (m, 4H), 1.92-1.84 (m, 2H), 1.79-1.67 (m, 2H), 1.65-1.58 (m, 2H), 1.26-1.22 (m, 1H), 1.16-1.12 (m, 1H), 1.02-0.96 (m, 1H), 0.91 (t, 1H).

Example 2-6

4-(4-((*S*)-hydroxy(4'-(trifluoromethyl)-[1,1'-biphenyl]-2-yl)methyl)piperidin-1-yl)-N-((4 -(((*R*)-4-((2-hydroxyethyl)(methyl)amino)-1-(phenylthio)butan-2-yl)amino)-3-((trifluor omethyl)sulfonyl)phenyl)sulfonyl)benzamide **6**

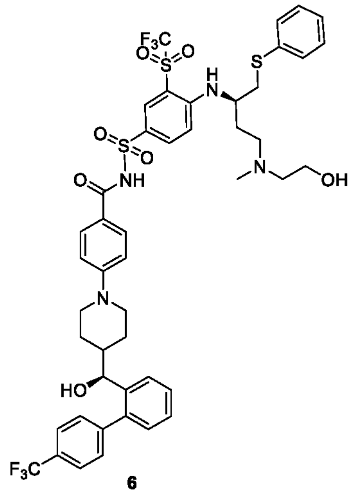

6

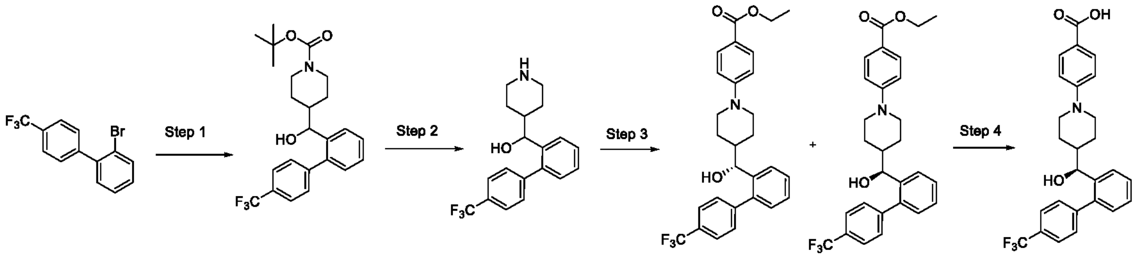


6a 6b 6c 6d 6e 6f

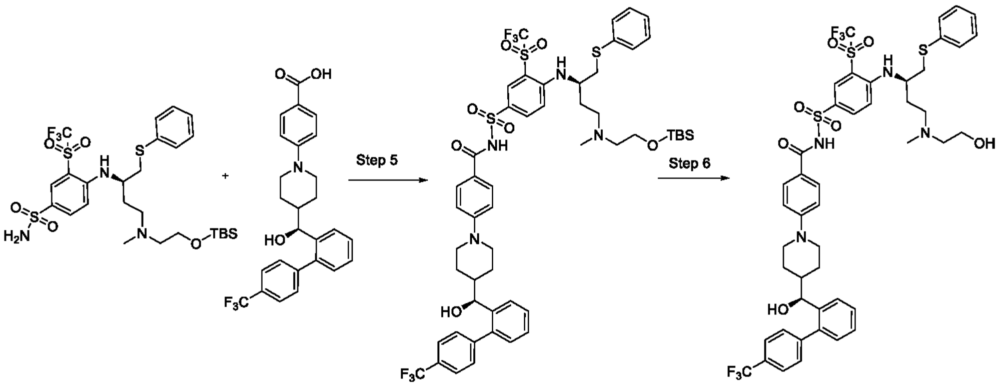


6g 6f 6h 6

Step 1

*Tert-butyl* 4-(hydroxy(4'-(trifluoromethyl)-[1,1'-biphenyl]-2-yl)methyl)piperidine-1-carboxylate **6b**

2-bromo-4'-(trifluoromethyl)-1,1'-biphenyl **6a** (3.3 g, 10.95 mmol, synthesized according to the method disclosed on page 53 of Example 53 in patent "US2004/171833, 2004, A1") placed in a flask, and tetrahydrofuran (50 mL) was added. After the mixture was cooled to -78 °C under nitrogen atmosphere, *n*-butyllithium (1.40 g, 21.92 mmol) was added dropwise into the reaction flask with the internal temperature kept below -65 °C, and after the addition, the mixture was stirred at -78 °C for 40 min, followed by the addition of a solution of *tert*-butyl formylpiperidine-1-carboxylate (4.67 g, 21.92 mmol) in tetrahydrofuran (20 mL). After the addition, the resulting mixture was heated to 0 °C and reacted for three hours, and then a saturated aqueous ammonium chloride solution was added dropwise to quench the reaction. The reaction solution was concentrated under reduced pressure and extracted with ethyl acetate (40 mL × 3). The organic phase was combined, sequentially washed with water (40 mL) and saturated brine (30 mL), dried over anhydrous sodium sulfate, and filtered, and the filtrate was concentrated under reduced pressure. The resulting residue was purified by silica gel column chromatography with an eluent system B to give the title product **6b** (5.5 g, yield: > 100%).

MS m/z (ESI): 434.1 [M-1].

Step 2

Piperidin-4-yl(4'-(trifluoromethyl)-[1,1'-biphenyl]-2-yl)methanol **6c** **6b** (5.5 g, 12.63 mmol) was placed in a reaction flask, and a solution of 4 *M* hydrogen chloride in dioxane (20 mL) was added under an ice bath. After the addition, the mixture was naturally warmed to room temperature and stirred for 2 h. The reaction solution was concentrated under reduced pressure, and toluene (10 mL) was added to the residue. The resulting mixture was concentrated under reduced pressure and dried with an oil pump to give crude title product **6c** (4.23 g, yield: 100%), which was directly used in the next step without purification.

MS m/z (ESI): 336.0 [M+1].

Step 3

Ethyl (*R*)-4-(4-(hydroxy(4'-(trifluoromethyl)-[1,1'-biphenyl]-2-yl)methyl)piperidin-1-yl)benzo ate **6d**

Ethyl (*S*)-4-(4-(hydroxy(4'-(trifluoromethyl)-[1,1'-biphenyl]-2-yl)methyl)piperidin-1-yl)benzo ate **6e**

The crude **6c** (270 mg, 0.805 mmol) was placed in a reaction flask, and dimethyl sulfoxide (3 mL) was added, followed by the sequential addition of ethyl p-fluorobenzoate (270 mg, 1.61 mmol, Accela ChemBio) and N,N-diisopropylethylamine (1.04 g, 8.05 mmol). After the addition, the mixture was heated to 120 °C and reacted for 24 h under nitrogen atmosphere. 5 mL of water was added to the reaction solution, and the mixture was extracted with ethyl acetate (4 mL × 5). The organic phases were combined, sequentially washed with water (4 mL) and saturated brine (4 mL), dried over anhydrous sodium sulfate, filtered and concentrated. The resulting residue was purified by silica gel column chromatography with an eluent system B to give a mixture (360 mg), which was sent to chiral preparative HPLC (preparation conditions: column: CHIRALPAK IG, 0.50 cm I.D*25 cm L, 0.1 μm; mobile phase: MeOH = 100%; detection wavelength: UV 214 nm; temperature: 38 °C) to give two fractions, Peak1 with smaller retention time (which was **6d** (73 mg)) and Peak 2 with larger retention time (which was **6e** (72 mg)).

MS m/z (ESI): 484.1 [M+1].

Peak 1: chiral HPLC analysis: retention time: 4.008 min, chiral purity: 100% (column: CHIRALPAK IG 150*4.6 mm, 5 μm (with a guard column); mobile phase: *n*-hexane/ethanol = 70/30 (v/v)).

Peak 2: chiral HPLC analysis: retention time: 6.289 min, chiral purity: 100% (column CHIRALPAK IG 150*4.6 mm, 5 μm (with a guard column); mobile phase: *n*-hexane/ethanol = 70/30 (v/v)).

Step 4

(*R*)-4-(4-(hydroxy(4'-(trifluoromethyl)-[1,1'-biphenyl]-2-yl)methyl)piperidin-1-yl)benzo ic acid **6f**

**6e** (70 mg, 0.145 mmol) was placed in a flask, and tetrahydrofuran (2 mL), water (0.5 mL) and methanol (0.5 mL) was added, followed by lithium hydroxide monohydrate (30.3 mg, 0.723 mmol). The mixture was heated to 50 °C and reacted for 16 h under nitrogen atmosphere. The reaction solution was concentrated under reduced pressure, and water (5 mL) was added to the residue. The resulting mixture was cooled under an ice bath, adjusted to pH 4 with a 1 *N* hydrochloric acid solution, and filtered. The filter cake was rinsed with *n*-hexane (5 mL) and dried with an oil pump to give the title product **6f** (63 mg, yield: 95.54%).

MS m/z (ESI): 456.0 [M+1].

Step 5

*N*-((4-(((*R*)-4-((2-((*tert*-butyldimethylsilyl)oxy)ethyl)(methyl)amino)-1-(phenylthio)buta n-2-yl)amino)-3-((trifluoromethyl)sulfonyl)phenyl)sulfonyl)-4-(4-((*S*)-hydroxy(4'-(triflu oromethyl)-[1,1'-biphenyl]-2-yl)methyl)piperidin-1-yl)benzamide **6h**

**6g** (30 mg, 0.040 mmol, synthesized by referring to the method provided for intermediate 67 on page 90 of the specification in patent "CN103153954 B") was dissolved in dichloromethane (3 mL), and **6f** (25 mg, 0.055 mmol) was added, followed by the sequential addition of 1-(3-dimethylaminopropyl)-3-ethylcarbodiimide hydrochloride (17.5 mg, 0.091 mmol) and 4-dimethylaminopyridine (11.2 mg, 0.091 mmol). After the addition, the mixture was stirred at room temperature for 4 h under nitrogen atmosphere, supplemented with **6f** (16.6 mg, 0.037 mmol), 1-(3-dimethylaminopropyl)-3-ethylcarbodiimide hydrochloride (10.5 mg, 0.055 mmol) and 4-dimethylaminopyridine (6.7 mg, 0.055 mmol), and stirred at room temperature overnight after the supplementation. The reaction solution was sequentially washed with water (5 mL) and saturated brine (5 mL), dried over anhydrous sodium sulfate, filtered, and concentrated under reduced pressure. The resulting residue was purified by thin layer chromatography with an elution system A to give the title product **6h** (40 mg, yield: 79.9%).

MS m/z (ESI): 1093.4 [M+1].

Step 6

4-(4-((*S*)-hydroxy(4'-(trifluoromethyl)-[1,1'-biphenyl]-2-yl)methyl)piperidin-1-yl)-N-((4 -(((R)-4-((2-hydroxyethyl)(methyl)amino)-1-(phenylthio)butan-2-yl)amino)-3-((trifluor omethyl)sulfonyl)phenyl)sulfonyl)benzamide **6**

**6h** (40 mg, 0.036 mmol) was added to tetrahydrofuran (2 mL), and tetrabutylammonium fluoride (16.4 mg, 0.073 mmol) was added. The mixture was stirred at room temperature for 4 h. The reaction solution was concentrated under reduced pressure, and the residue was dissolved in dichloromethane (8 mL) and washed with a saturated ammonium chloride solution (10 mL × 2). The organic phase was concentrated under reduced pressure, and the residue was dissolved in ethyl acetate (8 mL), sequentially washed with a saturated ammonium chloride solution (10 mL × 6), water (10 mL) and saturated brine (10 mL), dried over anhydrous sodium sulfate, filtered, and concentrated under reduced pressure. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **6** (17 mg, yield: 47.5%).

MS m/z (ESI): 979.2 [M+1].

$^1$H NMR (500 MHz, $CD_3OD$) δ 8.28 (d, 1H), 8.09-8.03 (m, 1H), 7.80 (d, 2H), 7.74 (d, 2H), 7.66 (d, 1H), 7.55 (d, 2H), 7.48 (t, 1H), 7.42-7.34 (m, 2H), 7.29-7.16 (m, 3H), 6.81 (dd, 2H), 4.41 (d, 1H), 4.02-3.97 (m, 1H), 3.84-3.75 (m, 2H), 3.64 (d, 1H), 3.37 (s, 2H), 3.30-3.18 (m, 3H), 3.12-3.06 (m, 2H), 2.75 (s, 2H), 2.72-2.63 (m, 1H), 2.59-2.50 (m, 1H), 2.29-2.19 (m, 2H), 2.08-2.02 (m, 2H), 1.82-1.72 (m, 1H), 1.66-1.58 (m, 1H), 1.39-1.31 (m, 4H), 1.27-1.12 (m, 2H), 1.03-0.89 (m, 2H).

Example 2-7

4-(4-((*R*)-hydroxy(4'-(trifluoromethyl)-[1,1'-biphenyl]-2-yl)methyl)piperidin-1-yl)-N-((4 -(((3*R*)-1-((2-hydroxyethyl)(methyl)amino)-4-(phenylthio)pentan-3-yl)amino)-3-((triflu oromethyl)sulfonyl)phenyl)sulfonyl)benzamide **7**

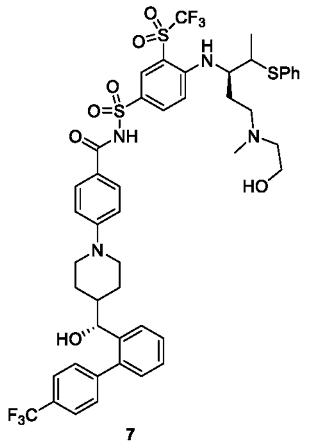

7

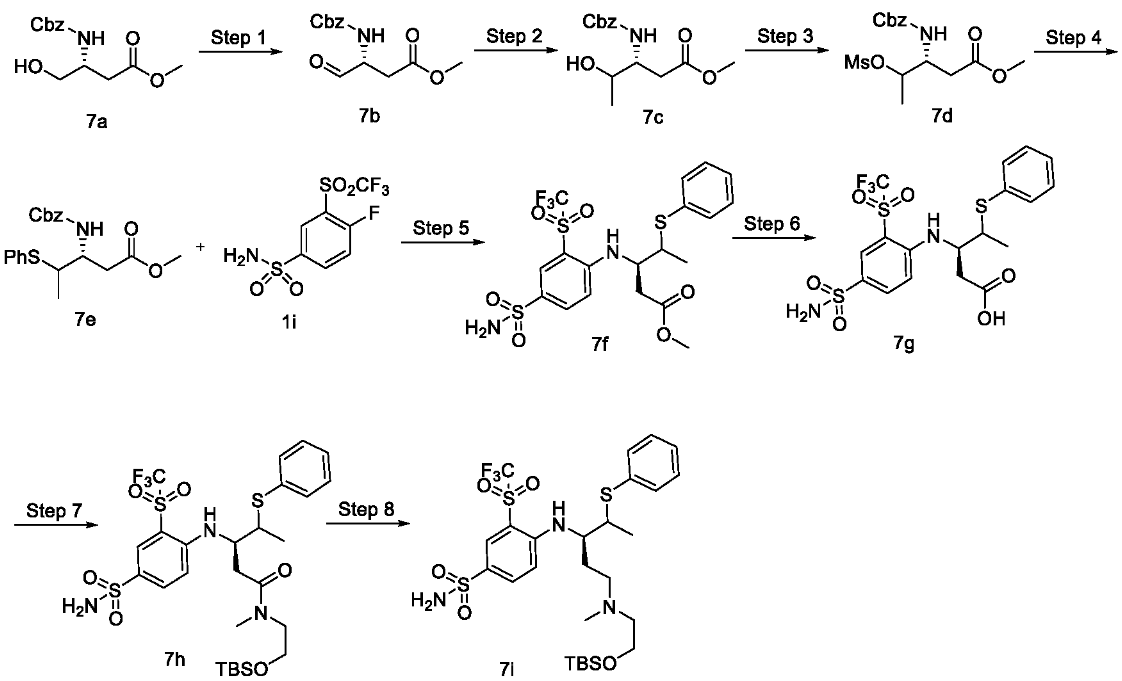


7a 7b 7c 7d

7e 1i 7f 7g

7h 7i

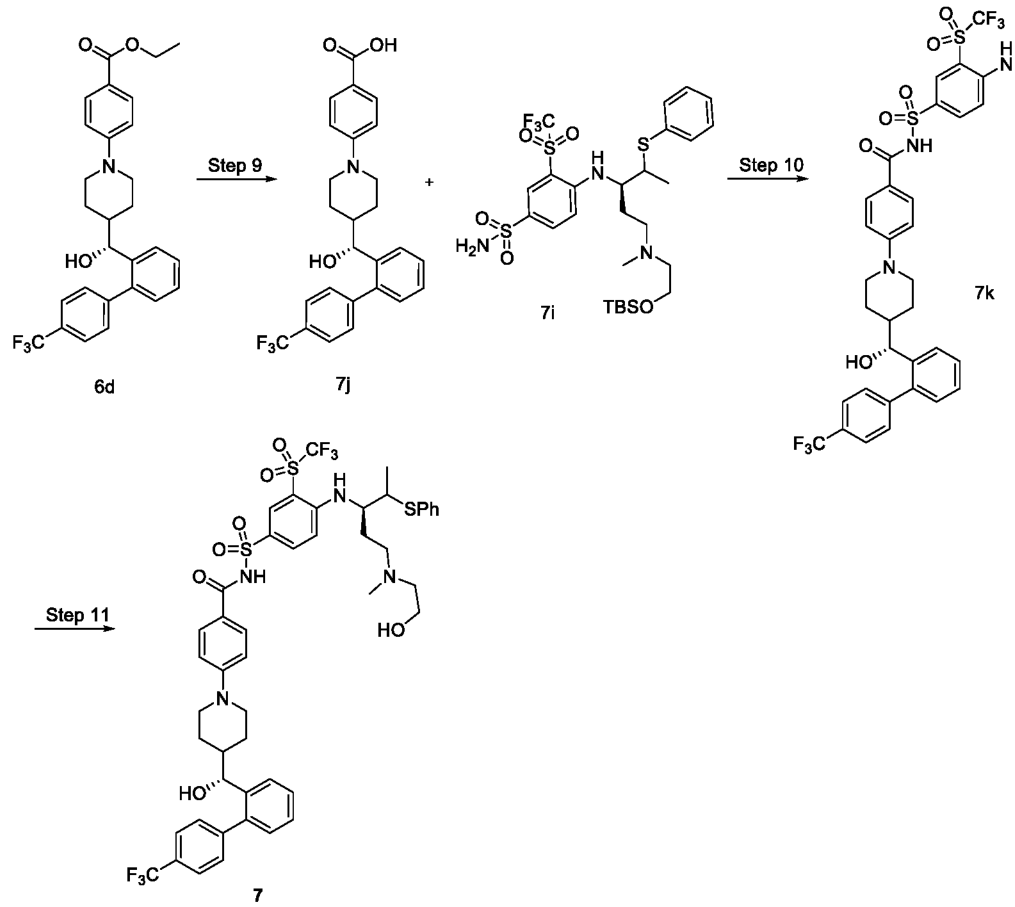


6d 7j 7i 7k 7

Step 1

Methyl (*R*)-3-(((benzyloxy)carbonyl)amino)-4-oxobutanoate **7b**

Oxalyl chloride (772 mg, 6.08 mmol) was dissolved in dichloromethane (5mL), and the solution was purged with nitrogen three times, cooled to -78 °C, followed by the dropwise addition of a solution of dimethyl sulfoxide (950 mg, 12.16 mmol) in dichloromethane (5mL). After the dropwise addition, the mixture was stirred at -78 °C for 30 min, and a solution of methyl (*R*)-3-(((benzyloxy)carbonyl)amino)-4-hydroxybutyrate **7a** (650 mg, 2.43 mmol, synthesized by referring to the method provided for intermediate 4 on page 59 of the specification in the patent "CN103153954 B") in dichloromethane (5 mL) was added dropwise. After the dropwise addition, the mixture was stirred at -78 °C for 1 h, and *N,N*-diisopropylethylamine (3.14 g, 24.29 mmol) was added dropwise. After the dropwise addition, the resulting mixture was heated to -60 °C and stirred for 30 min, and then heated to 0 °C and stirred for 30 min. 1 *M* cold hydrochloric acid (20 mL) was added at 0 °C to quench the reaction, and the mixture was subjected to liquid separation. The organic phase was washed with a phosphate buffered solution (30 mL) of pH 7 and a saturated sodium chloride solution (30 mL), dried over anhydrous sodium sulfate, and filtered, and the filtrate was concentrated under reduced pressure. The resulting residue was purified by silica gel column chromatography with an eluent system B to give the title product **7b** (540 mg, yield: 83.7%).

MS m/z (ESI): 266.2 [M+1].

Step 2

Methyl (3R)-3-(((benzyloxy)carbonyl)amino)-4-hydroxypentanoate **7c**

**7b** (490 mg, 1.95 mmol) was dissolved in diethyl ether (15 mL), and the solution was purged with nitrogen three times and cooled to -78 °C, followed by the dropwise addition of a solution of 1 *M* methylmagnesium bromide in tetrahydrofuran (2 mL, 2.03 mmol). After the dropwise addition, the mixture was naturally warmed to 0 °C and stirred for 3 h. A saturated ammonium chloride solution (20 mL) was added at 0 °C to quench the reaction. Water (10 mL) was added, and the mixture was extracted with ethyl acetate (10 mL × 3). The organic phases were combined, washed with a

saturated sodium chloride solution (10 mL), dried over anhydrous sodium sulfate and filtered to remove desiccant, and the filtrate was concentrated under reduced pressure. The resulting residue was purified by silica gel column chromatography with an eluent system B to give the title product **7c** (188 mg, yield: 36.2%).
MS m/z (ESI): 282.1 [M+1].

Step 3

Methyl (3R)-3-(((benzyloxy)carbonyl)amino)-4-((methylsulfonyl)oxy)pentanoate **7d**

**7c** (220 mg, 0.782 mmol) was dissolved in dichloromethane (8 mL), and pyridine (93 mg, 1.176 mmol) and 4-dimethylaminopyridine (9.5 mg, 0.078 mmol) were added. The mixture was purged with nitrogen three times and cooled to 0 °C, and methanesulfonic anhydride (164 mg, 0.941 mmol) was slowly added. After the addition, the mixture was heated to room temperature and stirred for 1.5 h. Water (20 mL) was added to the reaction solution, and the mixture was extracted with dichloromethane (8 mL × 3). The organic phases were combined, washed with a saturated sodium chloride solution (10 mL), dried over anhydrous sodium sulfate, and filtered to remove desiccant, and the filtrate was concentrated under reduced pressure. The resulting residue was purified by silica gel column chromatography with an eluent system B to give the title product **7d** (216 mg, yield: 76.8%).
MS m/z (ESI): 360.0 [M+1].

Step 4

Methyl (3*R*)-3-(((benzyloxy)carbonyl)amino)-4-(phenylthio)pentanoate **7e**

**7d** (216 mg, 0.601 mmol) was dissolved in toluene (8 mL), and sodium thiophenolate (397 mg, 3.004 mmol) was added. The mixture was purged with nitrogen three times, and heated to 105 °C and stirred for 16 h. Water (20 mL) was added after the reaction solution was cooled to room temperature, and the mixture was extracted with ethyl acetate (10 mL × 3). The organic phases were combined, washed with a saturated sodium chloride solution (30 mL), dried over anhydrous sodium sulfate, and filtered, and the filtrate was concentrated under reduced pressure. The resulting residue was purified by silica gel column chromatography with an eluent system B to give the title product **7e** (120 mg, yield: 53.5%).
MS m/z (ESI): 374.1 [M+1].

Step 5

Methyl (3R)-4-(phenylthio)-3-((4-sulfamoyl-2-((trifluoromethyl)sulfonyl)phenyl)amino)pentan oate **7f**

**7e** (112 mg, 0.300 mmol) was dissolved in tetrahydrofuran (1.5 mL), and trifluoroacetic acid (5mL) was added. The mixture was warmed to 72 °C and stirred for 1.5 h. The reaction solution was concentrated under reduced pressure with dichloromethane (20 mL), and dried under vacuum for 2 h. The residue was dissolved in N,N-dimethylformamide (4 mL), and **1i** (93 mg, 0.302 mmol) and N,N-diisopropylethylamine (388 mg, 3.00 mmol) were added. The reaction system was purged with nitrogen 3 times, and heated to 50 °C and stirred for 24 h. Water (20 mL) was added to the reaction solution, and the mixture was extracted with ethyl acetate (10 mL × 3). The organic phases were combined, washed with a saturated sodium chloride solution (20 mL), dried over anhydrous sodium sulfate, and filtered to remove desiccant, and the filtrate was concentrated under reduced pressure. The resulting residue was purified by silica gel column chromatography with an eluent system B to give the title product **7f** (119 mg, 75.3%).
MS m/z (ESI): 524.9 [M-1].

Step 6

(3R)-4-(phenylthio)-3-((4-sulfamoyl-2-((trifluoromethyl)sulfonyl)phenyl)amino)pentan oic acid **7g**

**7f** (119 mg, 0.226 mmol) was dissolved in 5 mL of a mixed solvent of tetrahydrofuran, methanol and water (V:V:V = 3:1:1), and lithium hydroxide monohydrate (29 mg, 0.691 mmol) was added. The mixture was stirred at room temperature for 3 h. The reaction solution was concentrated under reduced pressure, adjusted to pH 3-4 with 1 *M* hydrochloric acid, and extracted with dichloromethane (8 mL × 3). The organic phases were combined, washed with a saturated sodium chloride solution (20 mL), dried over anhydrous sodium sulfate, and filtered to remove desiccant, and the filtrate was concentrated under reduced pressure to obtain the title product **7g** (115 mg, yield: 99.3%).
MS m/z (ESI): 511.0 [M-1].

Step 7

(3*R*)-*N*-(2-((*tert*-butyldimethylsilyl)oxy)ethyl)-*N*-methyl-4-(phenylthio)-3-((4-sulfamoyl -2-((trifluoromethyl)sulfonyl)phenyl)amino)pentanamide **7h**

**7g** (115 mg, 0.224 mmol) was dissolved in tetrahydrofuran (3 mL), and 2-((*tert*-butyldimethylsilyl)oxy)-N-methylethyl-1-amine (43 mg, 0.227 mmol, Accela ChemBio), 3-(diethoxya-*o*-acyloxy)-1,2,3-benzotriazin-4-one (134 mg, 0.448 mmol), and *N*,*N*-diisopropylethylamine (58 mg, 0.448 mmol) were sequentially added. After the addition, the mixture was stirred at room temperature for 3 h under nitrogen atmosphere. The reaction solution was concentrated, and the residue was dissolved in ethyl acetate (10 mL), sequentially washed with a saturated aqueous ammonium chloride solution (20 mL), a saturated sodium bicarbonate solution (20 mL) and a saturated sodium chloride solution (20 mL). The organic phase was dried over anhydrous sodium sulfate and filtered, and the filtrate was concentrated under reduced pressure. The resulting residue was purified by silica gel column chromatography with an eluent system B to give the title product **7h** (107 mg, yield: 69.7%).
MS m/z (ESI): 684.0 [M+1].

Step 8

4-(((3*R*)-1-((2-((*tert*-butyldimethylsilyl)oxy)ethyl)(methyl)amino)-4-(phenylthio)pentan -3-yl)amino)-3-((trifluoromethyl)sulfonyl)benzenesulfonamide **7i**

**7h** (107 mg, 0.156 mmol) was placed in a reaction flask, and a 1 *M* borane-tetrahydrofuran complex solution (5 mL) was added. After the addition, the mixture was heated to 60 °C and stirred for 24 h. The reaction system was cooled to 0 °C, and 4 mL of methanol was added dropwise to quench the reaction. The mixture was concentrated under reduced pressure, and a solution of 7 M ammonia in methanol (10mL) was added to the residue. The mixture was stirred at room temperature for 48 h after sealing and concentrated under reduced pressure. The resulting residue was purified by silica gel column chromatography with an eluent system B to give the title product **7i** (10 mg, yield: 9.5%).
MS m/z (ESI): 670.1 [M+1].

Step 9

(R)-4-(4-(hydroxy(4'-(trifluoromethyl)-[1,1'-biphenyl]-2-yl)methyl)piperidin-1-yl)benzo ic acid **7j**

**6d** (70 mg, 0.145 mmol) was placed in a reaction flask, and tetrahydrofuran (2 mL), water (0.5 mL) and methanol (0.5 mL) were added, followed by lithium hydroxide monohydrate (30 mg, 0.72 mmol). The mixture was heated to 50 °C and reacted for 16 h under nitrogen atmosphere. The reaction solution was concentrated under reduced pressure, and water (5 mL) was added to the residue. The resulting mixture was cooled under an ice bath, adjusted to pH 4 with *a* 1 *M* hydrochloric acid solution, and filtered. The filter cake was rinsed with n-hexane (5 mL) and dried with an oil pump to give the title product **7j** (63 mg, yield: 95.5%).
MS m/z (ESI): 456.0 [M+1].

Step 10

*N*-((4-(((3*R*)-1-((2-((tert-butyldimethylsilyl)oxy)ethyl)(methyl)amino)-4-(phenylthio)pe ntan-3-yl)amino)-3-((trifluoromethyl)sulfonyl)phenyl)sulfonyl)-4-(4-((*R*)-hydroxy(4'-(tr ifluoromethyl)-[1,1'-biphenyl]-2-yl)methyl)piperidin-1-yl)benzamide **7k**

**7i** (16 mg, 0.024 mmol) was dissolved in dichloromethane (4 mL), and **7j** (13 mg, 0.028 mmol) was added, followed by 4-dimethylaminopyridine (6 mg, 0.049 mmol) and 1-(3-dimethylaminopropyl)-3-ethylcarbodiimide hydrochloride (14 mg, 0.073 mmol). After the addition, the mixture was purged with nitrogen three times, stirred at room temperature for 4 h, supplemented with **7j** (13 mg, 0.028 mmol), and stirred at room temperature for 16 h after the supplementation. The reaction solution was sequentially washed with water (8 mL) and a saturated sodium chloride solution (8 mL), dried over anhydrous sodium sulfate, and filtered, and the filtrate was concentrated under reduced pressure. The resulting residue was purified by thin layer chromatography with a developing solvent system A to give the title product **7k** (14 mg, yield: 52.9%).
MS m/z (ESI): 1107.2 [M+1].

Step 11

4-(4-((*R*)-hydroxy(4'-(trifluoromethyl)-[1,1'-biphenyl]-2-yl)methyl)piperidin-1-yl)-*N*-((4 -(((3*R*)-1-((2-hydroxyethyl)(methyl)amino)-4-(phenylthio)pentan-3-yl)amino)-3-((triflu oromethyl)sulfonyl)phenyl)sulfonyl)benzamide **7**

**7k** (14 mg, 0.013 mmol) was dissolved in tetrahydrofuran (3 mL), and tetrabutylammonium fluoride (6 mg, 0.027 mmol) was added. The mixture was stirred at room temperature for 1 h. The reaction solution was concentrated under reduced pressure, and the residue was dissolved in dichloromethane (5 mL) and washed with a saturated ammonium chloride solution (5 mL × 2). The organic phase was concentrated under reduced pressure, and the residue was dissolved in ethyl acetate (8 mL), sequentially washed with a saturated ammonium chloride solution (5 mL × 6), water (10 mL) and a saturated sodium chloride solution (10 mL), dried over anhydrous sodium sulfate, filtered, and concentrated under reduced pressure. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **7** (4.5 mg, yield: 35.8%).

MS m/z (ESI): 993.2 [M+1].

$^1$H NMR (500 MHz, $CD_3OD$) δ 8.33 (s, 1H), 8.02 (d, 1H), 7.76 (dd, 4H), 7.65 (d, 1H), 7.56 (dd, 4H), 7.48 (t, 1H), 7.44-7.33 (m, 4H), 7.21 (d, 1H), 6.81 (d, 2H), 6.60 (d, 1H), 4.41 (d, 1H), 3.97-3.92 (m, 1H), 3.84-3.72 (m, 3H), 3.69-3.56 (m, 3H), 2.76-2.62 (m, 4H), 2.55 (t, 2H), 2.32-2.17 (m, 3H), 2.10-1.87 (m, 4H), 1.82-1.72 (m, 2H), 1.64-1.58 (m, 2H), 1.27-1.22 (m, 1H), 1.21-1.12 (m, 2H), 1.03-0.96 (m, 1H), 0.92 (t, 1H).

Example 2-8

4-(4-((*R*)-(4'-chloro-[1,1'-biphenyl]-2-yl)(hydroxy)methyl)piperidin-1-yl)-*N*-((4-(((*R*)-1-(phenylthio)-3-((*S*)-pyrrolidin-2-yl)propan-2-yl)amino)-3-((trifluoromethyl)sulfonyl)phe nyl)sulfonyl)benzamide **8**

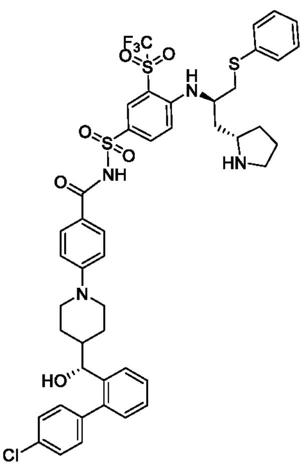

8

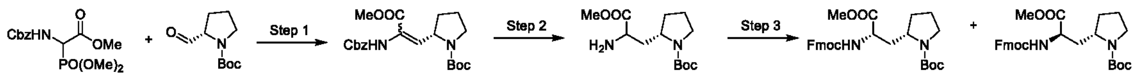


1b 8a 8b 8c 8d 8e

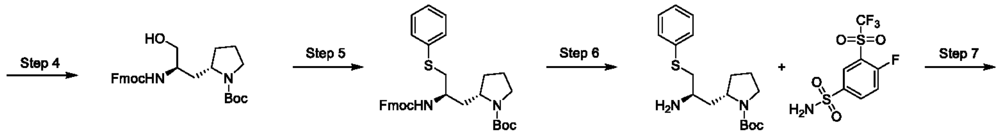


8f 8g 8h 1l

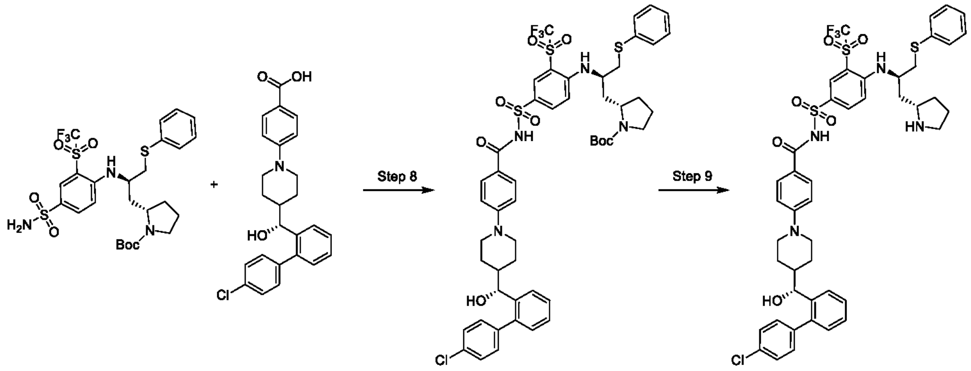


8i 1k 8j 8

Step 1

*Tert-butyl* (*S*)-2-(2-(((benzyloxy)carbonyl)amino)-3-methoxy-3-oxoprop-1-en-1-yl)pyrrolidine-1-c arboxylate **8b**

**1b** (2.49 g, 7.53 mmol, Accela ChemBio) was dissolved in tetrahydrofuran (20 mL), and the solution was cooled to 0 °C under an ice bath, followed by the dropwise addition of N,N-fithium diisopropylamide (661 mg, 6.17 mmol). The mixture was stirred at 0 °C for 1 h. The reaction system was cooled to -78 °C, and a solution of *tert*-butyl (*S*)-2-formylpyrrolidine-1-carboxylate **8a** (1.00 g, 5.02 mmol, PharmaBlock) in tetrahydrofuran (20 mL) was added dropwise. After the dropwise addition, the dry ice-acetone bath was removed, and the mixture was heated to room temperature and reacted for 12 h. A saturated ammonium chloride solution (20 mL) was added to quench the reaction, and the mixture was extracted with ethyl acetate (20 mL × 3). The organic phase was dried over anhydrous sodium sulfate and filtered, and the filtrate was concentrated under reduced pressure. The resulting residue was purified by silica gel column chromatography with an eluent system B to give the title product **8b** (1.90 g, yield: 94.1 %).

MS m/z (ESI): 305.0 [M+H-100].

Step 2

*Tert-butyl* (2S)-2-(2-amino-3-methoxy-3-oxopropyl)pyrrolidine-1-carboxylate **8c**

**8b** (1.90 g, 4.7 mmol) was dissolved in isopropanol (50 mL), and palladium on carbon (380 mg, 10% loading, dry basis) was added. The mixture was purged with hydrogen 3 times, and stirred at room temperature for 8 h. The reaction solution was filtered through celite, and the filter cake was rinsed with ethyl acetate (20 mL) and methanol (20 mL). The filtrate was concentrated to give the crude title product **8c** (1.28 g), which was directly used in the next step without purification.

Step 3

*Tert-butyl* (*S*)-2-((*S*)-2-((((9*H*-fluoren-9-yl)methoxy)carbonyl)amino)-3-methoxy-3-oxopropyl)pyr rolidine-1-carboxylate **8d**

*Tert-butyl* (*S*)-2-((*R*)-2-((((9*H*-fluoren-9-yl)methoxy)carbonyl)amino)-3-methoxy-3-oxopropyl)pyr rolidine-1-carboxylate **8e**

The crude **8c** (1.28 g, 4.7 mmol) was dissolved in 1,4-dioxane (40 mL), and water (10 mL), sodium bicarbonate (1.17 g, 14.1 mmol) and fluorenylmethoxycarbonyl chloride (1.21 g, 4.7 mmol) were added. The mixture was stirred at room temperature for 3 h. Water (30 mL) was added to quench the reaction, and the mixture was extracted with ethyl acetate (20 mL × 3). The organic phase was dried over anhydrous sodium sulfate, filtered and concentrated. The resulting residue was purified by silica gel column chromatography with an eluent system A to give the title product **8d** (1.03 g, 44.2%, peak 1) and **8e** (513 mg, 22.1%, peak 2).
MS m/z (ESI): 495.2 [M+1].

Peak 1: UPLC analysis: retention time: 2.77 min (column: ACQUITY UPLC BEHC18 50*2.1 mm, 1.7 μm; mobile phase: acetonitrile/water/formic acid = 10/90/0.1 (v/v/v) to 95/5/0.1 (v/v/v) gradient elution).
Peak 2: UPLC analysis: retention time: 2.69 min (column: ACQUITY UPLC BEHC18 50*2.1 mm, 1.7 μm; mobile phase: acetonitrile/water/formic acid = 50/50/0.1 (v/v/v) to 95/5/0.1 (v/v/v) gradient elution).

Step 4

*Tert-butyl* (*S*)-2-((*R*)-2-((((9*H*-fluoren-9-yl)methoxy)carbonyl)amino)-3-hydroxypropyl)pyrrolidin e-1-carboxylate **8f**

**8e** (169 mg, 0.34 mmol) was dissolved in tetrahydrofuran (8 mL), and ethanol (8 mL), sodium borohydride (77 mg, 2.05 mmol) and lithium chloride (94 mg, 2.22 mmol) were added. The mixture was stirred at 28 °C for 2.5 h, and saturated ammonium chloride (5 mL) was added to quench the reaction. The solvent was concentrated to dryness, and the residue was dissolved in ethyl acetate (10 mL) and sequentially washed with water (10 mL) and saturated sodium chloride (10 mL). The organic phase was dried over anhydrous sodium sulfate, filtered and concentrated,. The resulting residue was purified by silica gel column chromatography with an eluent system A to give the title product **8f** (125 mg, 78.4%).
MS m/z (ESI): 467.2 [M+1].

Step 5

*Tert-butyl* (*S*)-2-((*R*)-2-((((9*H*-fluoren-9-yl)methoxy)carbonyl)amino)-3-(phenylthio)propyl)pyrroli dine-1-carboxylate **8g**

**8f** (120 mg, 0.26 mmol) was dissolved in toluene (5 mL), and diphenyl disulfide (168 mg, 0.77 mmol) and tributylphosphine (156 mg, 0.77 mmol) were added. The mixture was heated to 80 °C and reacted for 12 h under nitrogen atmosphere. The solvent was concentrated to dryness. The resulting residue was purified by silica gel column chromatography with an eluent system A to give the title product **8g** (135 mg, 93.9%). MS m/z (ESI): 559.2 [M+1].

Step 6

*Tert*-butyl (*S*)-2-((*R*)-2-amino-3-(phenylthio)propyl)pyrrolidine-1-carboxylate **8h**

**8g** (135 mg, 0.24 mmol) was dissolved in dichloromethane (4 mL), and diethylamine (4 mL) was added. The mixture was stirred at room temperature for 4 h. The reaction solution was concentrated under reduced pressure to remove the organic solvent to give the title product **8h** (81.6 mg), which was directly used in the next step without purification.

Step 7

*Tert-butyl* (S)-2-((R)-4-(phenylthio)-3-((4-sulfamoyl-2-((trifluoromethyl)sulfonyl)phenyl)amino)pr opyl)pyrrolidine-1-carboxylate **8i**

The crude **8h** (81.6 mg, 0.24 mmol) was dissolved in *N,N*-dimethylformamide (5 mL), and **1i** (82 mg, 0.27mmol) and N,N-diisopropylethylamine (314 mg, 2.4 mmol) were added. The reaction system was heated to 50 °C and stirred for 12 h under nitrogen atmosphere. Water (10 mL) was added to quench the reaction, and the mixture was extracted with ethyl acetate (5 mL × 3). The organic phase was dried over anhydrous sodium sulfate and filtered, and the filtrate was concentrated under reduced pressure. The resulting residue was purified by silica gel column chromatography with an eluent system A to give the title product **8i** (105 mg, 69.3%).
MS m/z (ESI): 622.1 [M-1].

Step 8

*Tert-butyl* (*S*)-2-((*R*)-2-((4-(*N*-(4-(4-((*R*)-(4'-chloro-[1,1'-biphenyl]-2-yl)(hydroxy)methyl)piperidin -1-yl)benzoyl)sulfamoyl)-2-((trifluoromethyl)sulfonyl)phenyl)amino)-3-(phenylthio)pro pyl)pyrrolidine-1-carboxylate **8j**

**8i** (30 mg, 0.048 mmol) was dissolved in dichloromethane (3 mL), and **1k** (24 mg, 0.058 mmol) was added, followed by the sequential addition of 1-(3-dimethylaminopropyl)-3-ethylcarbodiimide hydrochloride (19 mg, 0.096 mmol) and 4-dimethylaminopyridine (12 mg, 0.096 mmol). After the addition, the mixture was stirred at room temperature for 4 h under nitrogen atmosphere, supplemented with **1k** (12 mg, 0.038 mmol), 1-(3-dimethylaminopropyl)-3-ethylcarbodiimide hydrochloride (19 mg, 0.096 mmol) and 4-dimethylaminopyridine (12 mg, 0.096 mmol), and stirred at room temperature overnight after the supplementation. The reaction solution was sequentially washed with water (5 mL) and saturated brine (5 mL), dried over anhydrous sodium sulfate, filtered, and concentrated under reduced pressure. The resulting residue was purified by thin layer chromatography with a developing solvent system A to give the title product **8j** (30 mg, yield: 60.7%).
MS m/z (ESI): 1027.2 [M+1].

Step 9

4-(4-((*R*)-(4'-chloro-[1,1'-biphenyl]-2-yl)(hydroxy)methyl)piperidin-1-yl)-*N*-((4-(((*R*)-1-(phenylthio)-3-((*S*)-pyrrolidin-2-yl)propan-2-yl)amino)-3-((trifluoromethyl)sulfonyl)phe nyl)sulfonyl)benzamide **8**

**8j** (30 mg, 0.029 mmol) was dissolved in dichloromethane (2 mL), and a solution of 4 M hydrogen chloride in 1,4-dioxane (2 mL) was added. The mixture was stirred at room temperature for 20 min. The reaction solution was concentrated under reduced pressure to remove the organic solvent. The residue was dissolved in dichloromethane and concentrated under reduced pressure to dryness, which were repeated three times. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **8** (25 mg, yield: 92.3%).
MS m/z (ESI): 927.1 [M+1].
$^1$H NMR (500 MHz, $CD_3OD$) δ 8.24 (d, 1H), 8.05-8.01 (m, 1H), 7.82 (d, 2H), 7.60 (d, 1H), 7.45-7.39 (m, 3H), 7.37 (d, 2H), 7.34-7.28 (m, 3H), 7.23 (t, 2H), 7.20-7.14 (m, 2H), 6.80 (d, 2H), 6.72 (d, 1H), 4.42 (d, 1H), 3.97 (br, 1H), 3.84-3.74 (m, 1H), 3.27-3.21 (m, 2H), 3.19-3.07 (m, 3H), 3.06-2.97 (m, 1H), 2.69-2.61 (m, 1H), 2.55-2.48 (m, 1H), 2.16-2.00 (m, 5H), 1.98-1.89 (m, 2H), 1.88-1.80 (m, 1H), 1.78-1.69 (m, 1H), 1.63-1.57 (m, 2H), 1.54-1.49 (m, 1H), 1.26-1.20 (m, 1H), 1.15-1.04 (m, 1H), 1.01-0.94 (m, 1H).

Example 2-9

4-(4-((*R*)-(4'-chloro-[1,1'-biphenyl]-2-yl)(hydroxy)methyl)piperidin-1-yl)-*N*-((4-(((*R*)-1-((*S*)-1-(2-hydroxyethyl)pyrrolidin-2-yl)-3-(phenylthio)propan-2-yl)amino)-3-((trifluoro methyl)sulfonyl)phenyl)sulfonyl)benzamide **9**

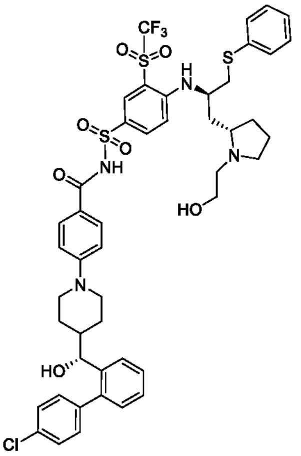

9

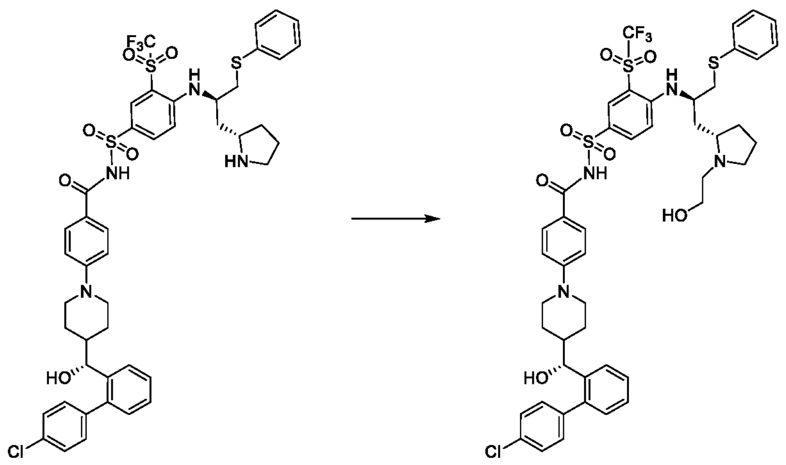

8 9

**8** (15 mg, 0.016 mmol) was dissolved in acetonitrile (2 mL), and triethylamine (16 mg, 0.16 mmol) and bromoethanol (20 mg, 0.16 mmol) were sequentially added. The reaction system was heated to 70 °C and reacted for 12 h under nitrogen atmosphere. The reaction solution was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **9** (10 mg, yield: 63.4%).

MS m/z (ESI): 971.2 [M+1].

$^1$H NMR (500 MHz, $CD_3OD$) δ 8.25 (d, 1H), 8.06 (dd, 1H), 7.80 (d, 2H), 7.61 (dd, 1H), 7.49-7.35 (m, 5H), 7.35-7.28 (m, 3H), 7.24 (dd, 2H), 7.20-7.14 (m, 2H), 6.79 (t, 3H), 4.42 (d, 1H), 4.02 (t, 1H), 3.84-3.70 (m, 3H), 3.61 (d, 2H), 3.28-3.14 (m, 2H), 3.06 (br, 1H), 2.96 (br, 1H), 2.71-2.59 (m, 1H), 2.56-2.45 (m, 1H), 2.45-2.35 (m, 1H), 2.32-2.22 (m, 1H), 2.10-1.97 (m, 4H), 1.96-1.86 (m, 1H), 1.85-1.69 (m, 2H), 1.60 (t, 2H), 1.26-1.18 (m, 1H), 1.06-1.08 (m, 1H), 1.01-0.92 (m, 1H), 0.92-1.86 (m, 1H).

Example 2-10

4-(4-((R)-hydroxy(4'-(trifluoromethyl)-[1,1'-biphenyl]-2-yl)methyl)piperidin-1-yl)-N-((4 -(((*R*)-4-((2-hydroxyethyl)(methyl)amino)-1-(phenylthio)butan-2-yl)amino)-3-((trifluor omethyl)sulfonyl)phenyl)sulfonyl)benzamide **10**

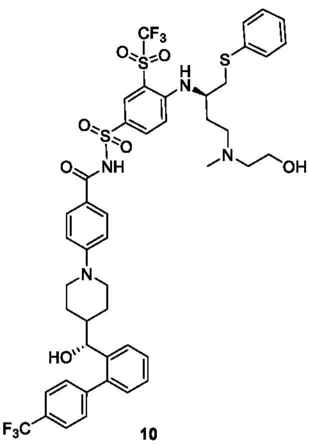

10

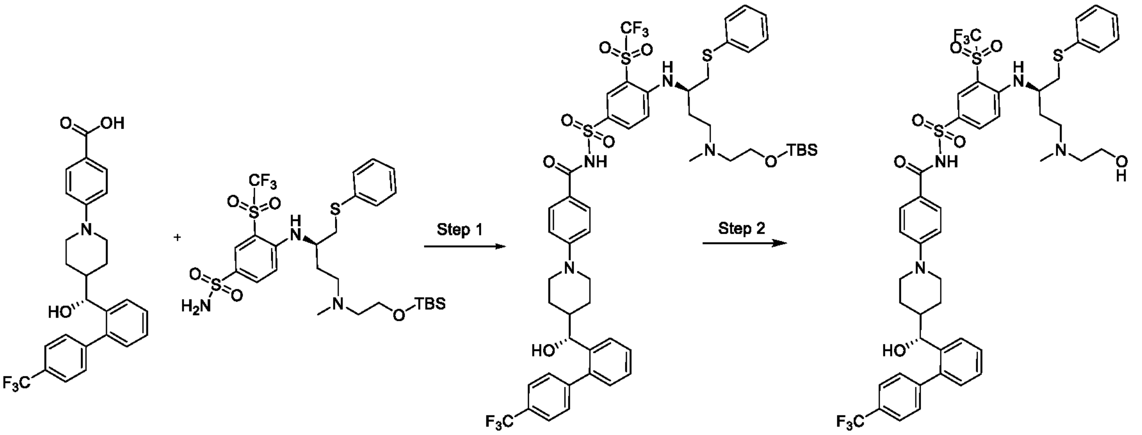


7j 6g 10a 10

Step 1

*N*-((4-(((*R*)-4-((2-((*tert*-butyldimethylsilyl)oxy)ethyl)(methyl)amino)-1-(phenylthio)buta n-2-yl)amino)-3-((trifluoromethyl)sulfonyl)phenyl)sulfonyl)-4-(4-((*R*)-hydroxy(4'-(trifl uoromethyl)-[1,1'-biphenyl]-2-yl)methyl)piperidin-1-yl)benzamide **10a**

**6g** (30 mg, 0.040 mmol) was dissolved in dichloromethane (3 mL), and **7j** (25 mg, 0.055 mmol) was added, followed by the sequential addition of 1-(3-dimethylaminopropyl)-3-ethylcarbodiimide hydrochloride (17.5 mg, 0.091 mmol) and 4-dimethylaminopyridine (11.2 mg, 0.091 mmol). After the addition, the mixture was stirred at room temperature for 4 h under nitrogen atmosphere, supplemented with **7j** (16.6 mg, 0.037 mmol), 1-(3-dimethylaminopropyl)-3-ethylcarbodiimide hydrochloride (10.5 mg, 0.055 mmol) and 4-dimethylaminopyridine (6.7 mg, 0.055 mmol), and stirred at room temperature overnight after the supplementation. The reaction solution was sequentially washed with water (5 mL) and saturated brine (5 mL), dried over anhydrous sodium sulfate, filtered, and concentrated under reduced pressure. The resulting residue was purified by thin layer chromatography with a developing solvent system A to give the title product **10a** (40 mg, yield: 79.9%).

MS m/z (ESI): 1093.4 [M+1].

Step 2

4-(4-((*R*)-hydroxy(4'-(trifluoromethyl)-[1,1'-biphenyl]-2-yl)methyl)piperidin-1-yl)-*N*-((4 -(((*R*)-4-((2-hydroxyethyl)(methyl)amino)-1-(phenylthio)butan-2-yl)amino)-3-((trifluor omethyl)sulfonyl)phenyl)sulfonyl)benzamide **10**

**10a** (40 mg, 0.036 mmol) was added to tetrahydrofuran (2 mL), and tetrabutylammonium fluoride (16 mg, 0.073 mmol) was added. The mixture was stirred at room temperature for 4 h. The reaction solution was concentrated under reduced pressure, and the residue was dissolved in dichloromethane (8 mL) and washed with a saturated ammonium chloride solution (10 mL × 2). The organic phase was concentrated under reduced pressure, and the residue was

dissolved in ethyl acetate (8 mL), washed sequentially with a saturated ammonium chloride solution (10 mL × 6), water (10 mL) and saturated brine (10 mL), dried over anhydrous sodium sulfate, filtered, and concentrated under reduced pressure. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **10** (17 mg, yield: 47.5%).

MS m/z (ESI): 979.2 [M+1].

$^1$H NMR (500 MHz, $CD_3OD$) δ 8.26 (d, 1H), 8.04 (dd, 1H), 7.79 (d, 2H), 7.72 (d, 2H), 7.64 (d, 1H), 7.53 (d, 2H), 7.46 (t, 1H), 7.40-7.31 (m, 3H), 7.29-7.12 (m, 4H), 6.86-6.71 (m, 3H), 4.39 (d, 1H), 3.98 (br, 1H), 3.84-3.71 (m, 3H), 3.62 (d, 1H), 3.26-3.16 (m, 3H), 3.14-2.96 (m, 2H), 2.80-2.60 (m, 4H), 2.56-2.49 (m, 1H), 2.19 (t, 1H), 2.11-1.93 (m, 3H), 1.76 (d, 1H), 1.27-1.18 (m, 1H), 1.17-1.10 (m, 1H), 1.03-0.93 (m, 1H), 0.94-0.83 (m, 1H).

Example 2-11

4-(4-((*R*)-(4'-chloro-[1,1'-biphenyl]-2-yl)(hydroxy)methyl)piperidin-1-yl)-*N*-((4-(((*R*)-1-((*S*)-1-(3-hydroxypropyl)pyrrolidin-2-yl)-3-(phenylthio)propan-2-yl)amino)-3-((trifluor omethyl)sulfonyl)phenyl)sulfonyl)benzamide **11**

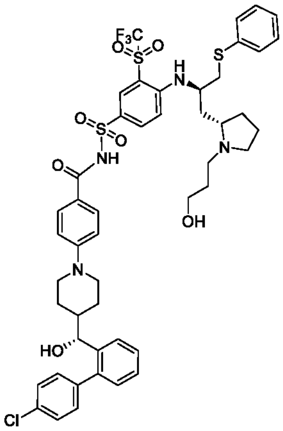

**11**

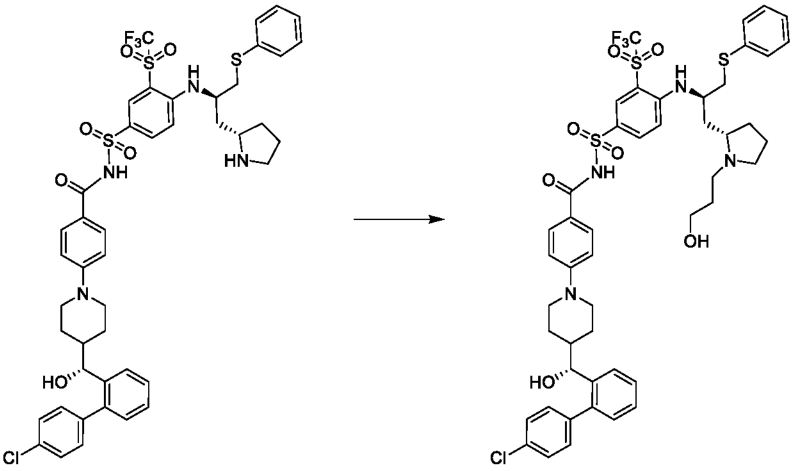

**8** **11**

**8** (20 mg, 0.021 mmol) was dissolved in acetonitrile (2 mL), and triethylamine (22 mg, 0.22 mmol) and bromoethanol (30 mg, 0.22 mmol) were sequentially added. The reaction system was heated to 70 °C and reacted for 12 h under nitrogen atmosphere. The reaction solution was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **11** (13 mg, yield: 61.1%).

MS m/z (ESI): 985.1 [M+1].

$^1$H NMR (500 MHz, $CD_3OD$) δ 8.28 (d, 1H), 8.09 (dd, 1H), 7.86-7.78 (m, 2H), 7.63 (dd, 1H), 7.48-7.38 (m, 5H),

7.37-7.31 (m, 3H), 7.27 (dd, 2H), 7.23-7.17 (m, 2H), 6.81 (t, 3H), 4.44 (d, 1H), 4.08-4.00 (m, 1H), 3.81 (d, 1H), 3.72-3.55 (m, 4H), 3.25 (dd, 2H), 3.05 (s, 1H), 2.94 (s, 1H), 2.73-2.63 (m, 1H), 2.59-2.50 (m, 1H), 2.48-2.39 (m, 1H), 2.29 (s, 1H), 2.21 (t, 1H), 2.10-2.01 (m, 4H), 1.97-1.70 (m, 5H), 1.62 (s, 1H), 1.31-1.20 (m, 3H), 1.15 (d, 1H), 1.04-0.95 (m, 1H), 0.93 (d, 1H).

Example 2-12

4-(4-((*R*)-hydroxy(4'-(trifluoromethyl)-[1,1'-biphenyl]-2-yl)methyl)piperidin-1-yl)-*N*-((4 -(((*R*)-1-(phenylthio)-3-((S)-pyrrolidin-2-yl)propan-2-yl)amino)-3-((trifluoromethyl)sulf onyl)phenyl)sulfonyl)benzamide **12**

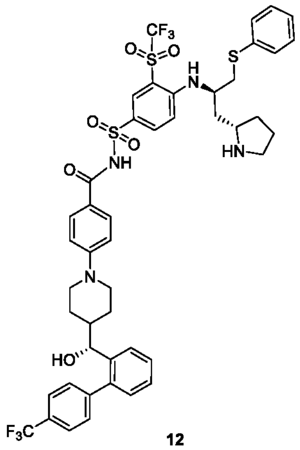

12

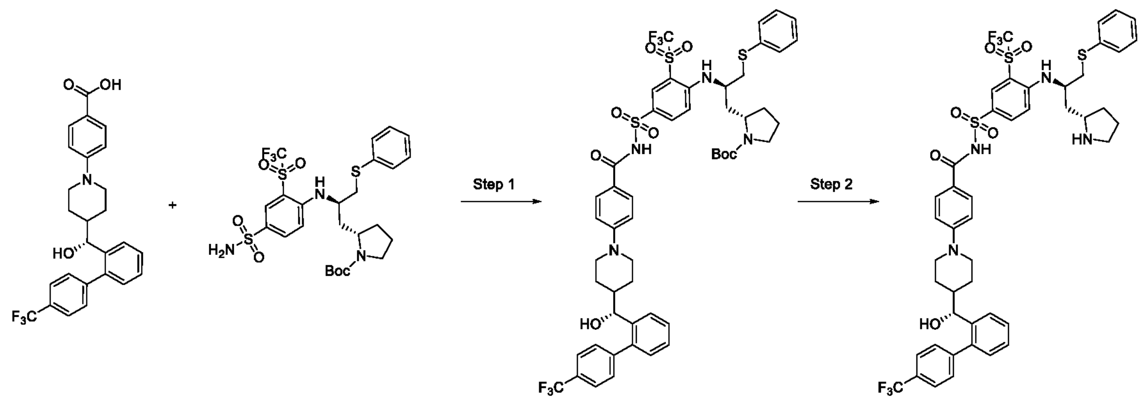


7j 8i 12a 12

Step 1

*Tert*-butyl (*S*)-2-((*R*)-2-((4-(*N*-(4-(4-((*R*)-hydroxy(4'-(trifluoromethyl)-[1,1'-biphenyl]-2-yl)methyl) piperidin-1-yl)benzoyl)sulfamoyl)-2-((trifluoromethyl)sulfonyl)phenyl)amino)-3-(pheny lthio)propyl)pyrrolidine-1-carboxylate **12a**

**8i** (80 mg, 0.13 mmol) was dissolved in dichloromethane (3 mL), and **7j** (70 mg, 0.15 mmol) was added, followed by the sequential addition of 1-(3-dimethylaminopropyl)-3-ethylcarbodiimide hydrochloride (49 mg, 0.26 mmol) and 4-dimethylaminopyridine (32 mg, 0.26 mmol). After the addition, the mixture was stirred at room temperature for 4 h under nitrogen atmosphere, supplemented with **7j** (47 mg, 0.10 mmol), 1-(3-dimethylaminopropyl)-3-ethylcarbodiimide hydrochloride (49 mg, 0.26 mmol) and 4-dimethylaminopyridine (32 mg, 0.26 mmol), and stirred at room temperature overnight after the supplementation. The reaction solution was sequentially washed with water (5 mL) and saturated brine (5 mL), dried over anhydrous sodium sulfate, filtered, and concentrated under reduced pressure. The resulting residue was purified by thin layer chromatography with a developing solvent system A to give the title product **12a** (80 mg, yield: 58.8%).
MS m/z (ESI): 1059.0 [M-1].

Step 2

4-(4-((*R*)-hydroxy(4'-(trifluoromethyl)-[1,1'-biphenyl]-2-yl)methyl)piperidin-1-yl)-*N*-((4 -(((*R*)-1-(phenylthio)-3-((*S*)-pyrrolidin-2-yl)propan-2-yl)amino)-3-((trifluoromethyl)sulf onyl)phenyl)sulfonyl)benzamide **12**

**12a** (50 mg, 0.047 mmol) was dissolved in dichloromethane (2 mL), and a solution of 4 *M* hydrogen chloride in 1,4-dioxane (2 mL) was added. The mixture was stirred at room temperature for 20 min. The reaction solution was concentrated under reduced pressure to remove the organic solvent. The residue was dissolved in dichloromethane and concentrated under reduced pressure to dryness, which were repeated three times. The resulting residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **12** (6 mg, yield: 66.2%).

MS m/z (ESI): 961.2 [M+1].

$^1$H NMR (500 MHz, $CD_3OD$) δ 8.23 (s, 1H), 8.03 (d, 1H), 7.80 (d, 2H), 7.72 (d, 2H), 7.64 (d, 1H), 7.53 (d, 2H), 7.47 (t, 1H), 7.35 (t, 3H), 7.27-7.14 (m, 4H), 6.79 (d, 2H), 6.73 (d, 1H), 4.39 (d, 1H), 3.98 (s, 1H), 3.81-3.73 (m, 1H), 3.63-3.58 (m, 1H), 3.56-3.50 (m, 1H), 3.21-3.13 (m, 2H), 2.64 (t, 1H), 2.50 (t, 1H), 2.33-2.25 (m, 1H), 2.25-2.20 (m, 1H), 2.11-1.91 (m, 6H), 1.80-1.65 (m, 2H), 1.64-1.54 (m, 2H), 1.25-1.17 (m, 2H), 1.16-1.10 (m, 1H), 1.00-0.93 (m, 1H).

Example 2-13

4-(4-((*R*)-hydroxy(4'-(trifluoromethyl)-[1,1'-biphenyl]-2-yl)methyl)piperidin-1-yl)-N-((4 -(((*R*)-1-((*S*)-1-(2-hydroxyethyl)pyrrolidin-2-yl)-3-(phenylthio)propan-2-yl)amino)-3-((t rifluoromethyl)sulfonyl)phenyl)sulfonyl)benzamide **13**

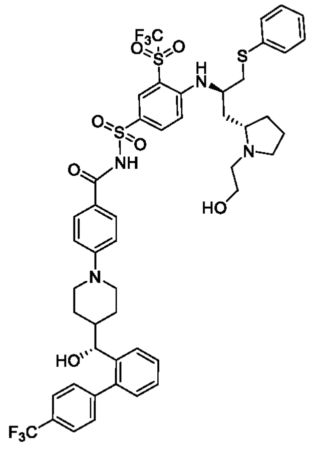

**13**

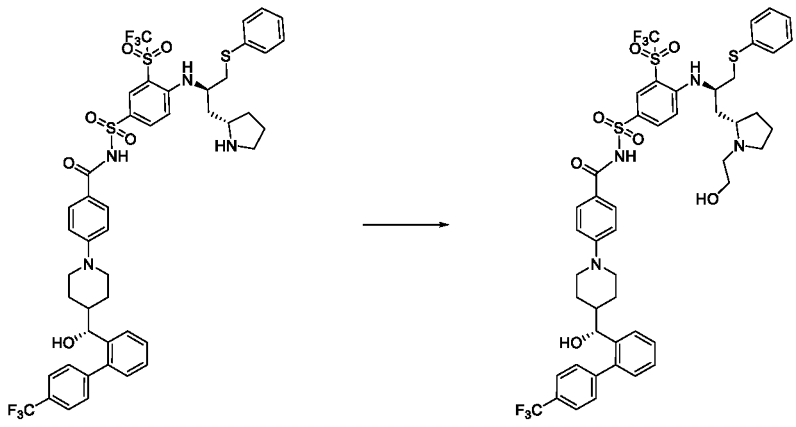

**12** **13**

**12** (30 mg, 0.031 mmol) was dissolved in acetonitrile (4 mL), and triethylamine (32 mg, 0.31 mmol) and bromoethanol (39 mg, 0.31 mmol) were sequentially added. The reaction system was heated to 70 °C and reacted for 12

h under nitrogen atmosphere. The reaction solution was concentrated under reduced pressure. The residue was purified by high-performance liquid chromatography (separation conditions: column: Sharpsil-T Prep C18; mobile phase: ammonium bicarbonate, water, acetonitrile), and the corresponding fraction was collected and concentrated under reduced pressure to give the title product **13** (25 mg, yield: 56.9%).

MS m/z (ESI): 1005.1[M+1].

$^1H$ NMR (500 MHz, $CD_3OD$) δ 8.27 (d, 1H), 8.08 (dd, 1H), 7.85-7.79 (m, 2H), 7.74 (d, 2H), 7.68-7.63 (m, 1H), 7.55 (d, 2H), 7.50-7.46 (m, 1H), 7.42-7.35 (m, 3H), 7.29-7.18 (m, 4H), 6.87-6.69 (m, 3H), 4.41 (d, 1H), 4.03 (br, 1H), 3.83-3.73 (m, 3H), 3.67-3.56 (m, 2H), 3.25-3.18 (m, 2H), 3.14-2.86 (m, 2H), 2.72-2.59 (m, 1H), 2.58-2.47 (m, 1H), 2.46-2.36 (m, 1H), 2.33-2.23 (m, 1H), 2.10-1.97 (m, 4H), 1.96-1.87 (m, 1H), 1.84-1.72 (m, 2H), 1.66-1.55 (m, 1H), 1.28-1.19 (m, 1H), 1.16 (d, 1H), 1.03-0.95 (m, 1H), 0.95-0.89 (m, 1H).

Example 2-14

4-(4-((*R*)-hydroxy(4'-(trifluoromethyl)-[1,1'-biphenyl]-2-yl)methyl)piperidin-1-yl)-N-((4 -(((2*R*)-4-((2-hydroxyethyl)(methyl)amino)-3-methyl-1-(phenylthio)butan-2-yl)amino)-3-((trifluoromethyl)sulfonyl)phenyl)sulfonyl)benzamide **14**

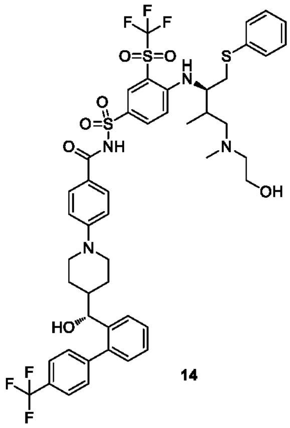

**14**

Example 2-15

4-(4-((*R*)-hydroxy(4'-(trifluoromethyl)-[1,1'-biphenyl]-2-yl)methyl)piperidin-1-yl)-*N*-((4 -(((*R*)-5-((2-hydroxyethyl)(methyl)amino)-1-(phenylthio)pentan-2-yl)amino)-3-((trifluor omethyl)sulfonyl)phenyl)sulfonyl)benzamide **15**

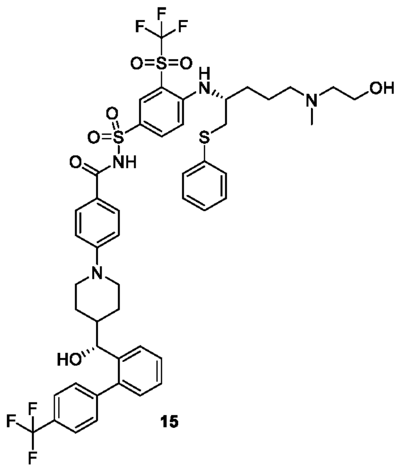

**15**

Example 2-16

4-(4-((*R*)-hydroxy(4'-(trifluoromethyl)-[1,1'-biphenyl]-2-yl)methyl)piperidin-1-yl)-*N*-((4 -(((*R*)-1-((*R*)-1-(2-hydroxyethyl)pyrrolidin-2-yl)-3-(phenylthio)propan-2-yl)amino)-3-((t rifluoromethyl)sulfonyl)phenyl)sulfonyl)benzamide **16**

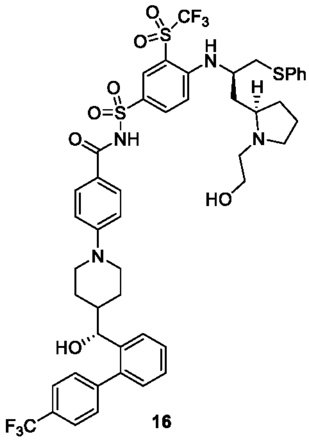

**16**

Example 2-17

4-(4-((*R*)-(4'-chloro-[1,1'-biphenyl]-2-yl)(hydroxy)methyl)piperidin-1-yl)-*N*-((4-(((*R*)-1-((*R*)-1-methylpyrrolidin-2-yl)-3-(phenylthio)propan-2-yl)amino)-3-((trifluoromethyl)sul fonyl)phenyl)sulfonyl)benzamide **17**

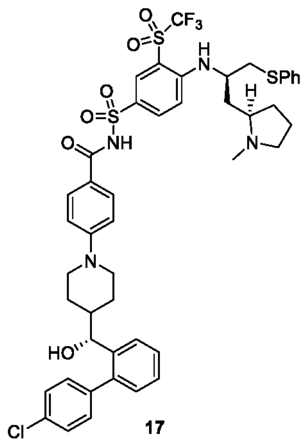

**17**

Example 2-18

4-(4-((*R*)-(4'-chloro-[1,1'-biphenyl]-2-yl)(hydroxy)methyl)piperidin-1-yl)-*N*-((4-(((*R*)-1-((*S*)-1-methylpyrrolidin-2-yl)-3-(phenylthio)propan-2-yl)amino)-3-((trifluoromethyl)sul fonyl)phenyl)sulfonyl)benzamide **18**

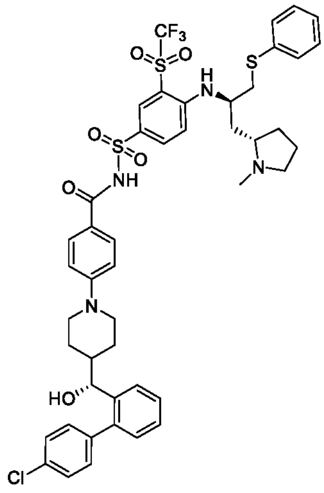

**18**

Example 2-19

4-(4-((*R*)-hydroxy(4'-(trifluoromethyl)-[1,1'-biphenyl]-2-yl)methyl)piperidin-1-yl)-*N*-((4 -(((*R*)-1-((*S*)-1-methylpyrrolidin-2-yl)-3-(phenylthio)propan-2-yl)amino)-3-((trifluorom ethyl)sulfonyl)phenyl)sulfonyl)benzamide **19**

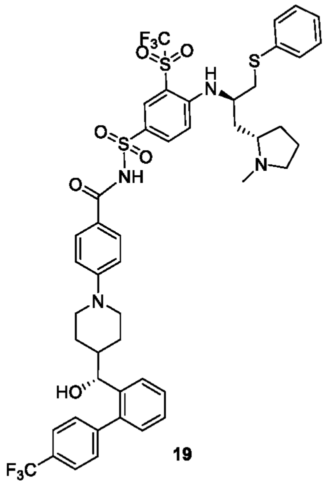

**19**

Example 2-20

4-(4-((*R*)-(4'-chloro-[1,1'-biphenyl]-2-yl)(hydroxy)methyl)piperidin-1-yl)-*N*-((4-(((*R*)-1-((*S*)-1-(2-hydroxyethyl)-2-methylpyrrolidin-2-yl)-3-(phenylthio)propan-2-yl)amino)-3-( (trifluoromethyl)sulfonyl)phenyl)sulfonyl)benzamide **20**

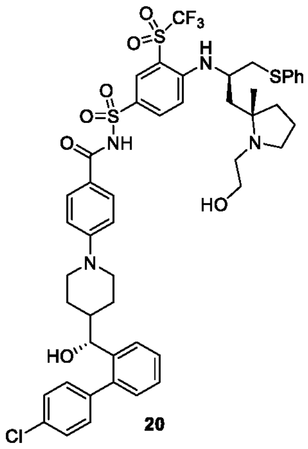

20

Example 2-21

4-(4-((*R*)-hydroxy(4'-(trifluoromethyl)-[1,1'-biphenyl]-2-yl)methyl)piperidin-1-yl)-*N*-((4 -(((*R*)-1-((*S*)-1-(2-hydroxyethyl)-2-methylpyrrolidin-2-yl)-3-(phenylthio)propan-2-yl)a mino)-3-((trifluoromethyl)sulfonyl)phenyl)sulfonyl)benzamide **21**

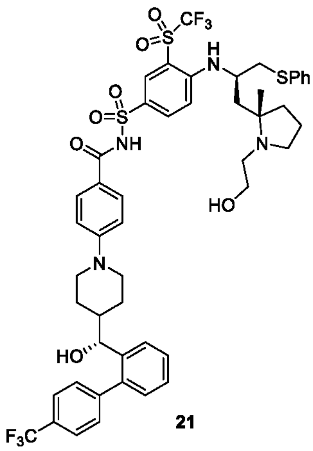

21

Example 2-22

4-(4-((*R*)-(4'-chloro-[1,1'-biphenyl]-2-yl)(hydroxy)methyl)piperidin-1-yl)-*N*-((4-(((2*R*)-1-(1-(2-hydroxyethyl)azetidin-2-yl)-3-(phenylthio)propan-2-yl)amino)-3-((trifluorometh yl)sulfonyl)phenyl)sulfonyl)benzamide **22**

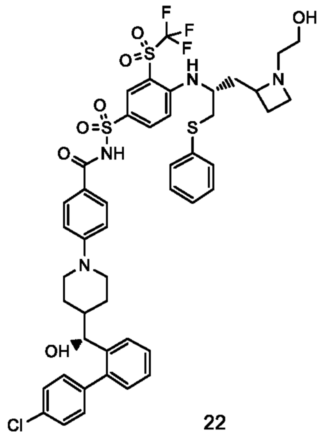

22

Example 2-23

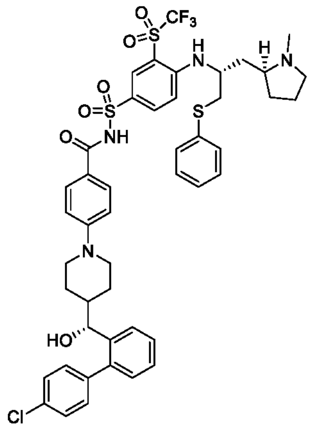

Example 2-24 AZD4320

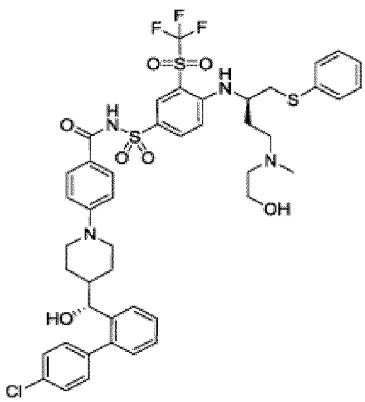

The compound was prepared by referring to the method described in Example 3 (positive control compound AZD4320) on page 160-161 of patent WO2012017251.

Example 3-1 LD-1

4-((*S*)-2-((*S*)-2-(6-(2,5-dioxo-2,5-dihydro-1*H*-pyrrol-1-yl)hexanamido)-3-methylbutana mido)-5-ureidopentanamido)benzyl

(*R*)-2-((*R*)-2-((4-(*N*-(4-(4-((*R*)-(4'-chloro-[1,1'-biphenyl]-2-yl)(hydroxy)methyl)piperidi n-1-yl)benzoyl)sulfamoyl)-2-((trifluoromethyl)sulfonyl)phenyl)amino)-3-(phenylthio)pr opyl)pyrrolidine-1-carboxylate **LD-1**

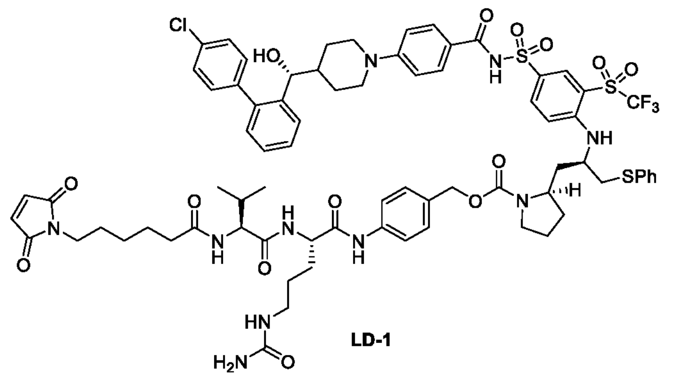

LD-1

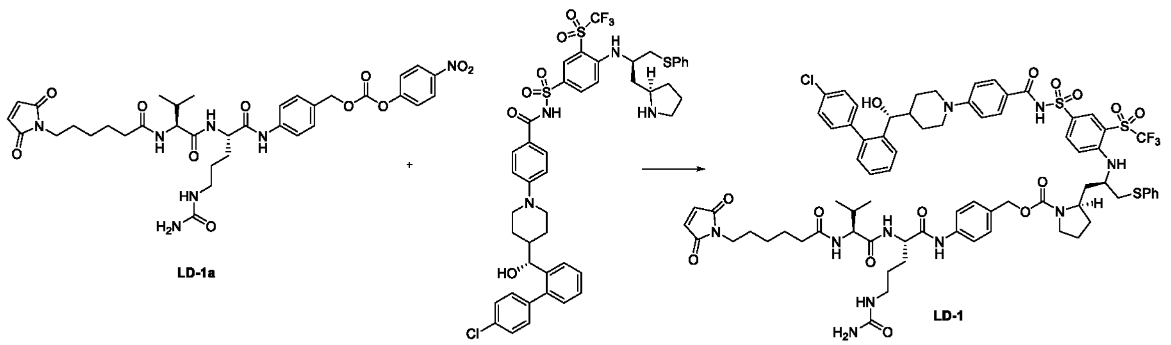

LD-1a

1

LD-1

**1** (3 mg, 0.003 mmol) was added to 0.5 mL of *N*,*N*-dimethylformamide, and 4-((*S*)-2-((*S*)-2-(6-(2,5-dioxo-2,5-dihydro-1*H*-pyrrol-1-yl)hexanamido)-3-methylbutana mido)-5-ureidopentanamido)benzyl (4-nitrophenyl) carbonate **LD-1a** (3.1 mg, 0.004 mmol, Haoyuan) was added, followed by 0.1 mL of pyridine. The mixture was purged with argon three times, and 1-hydroxybenzotriazole (2 mg, 0.014 mmol) and *N*,*N*-diisopropylethylamine (2 mg, 0.015 mmol) were added. The resulting mixture was stirred at room temperature for 16 h. The reaction solution was purified by high-performance liquid chromatography (separation conditions: column: XBridge Prep C18; mobile phase: ammonium acetate, water, acetonitrile), and the corresponding fractions were collected and concentrated under reduced pressure to give the title product **LD-1** (2 mg, yield: 40.5%).

MS m/z (ESI): 1525.0 [M+1].

$^1$H NMR (500 MHz, $CD_3OD$) $\delta$ 8.17 (s, 1H), 7.92-7.80 (m, 3H), 7.64-7.51 (m, 3H), 7.46-7.09 (m, 12H), 6.84-6.74 (m, 3H), 6.48-6.40 (m, 2H), 5.35 (t, 4H), 4.41 (d, 1H), 4.15 (d, 1H), 4.00-3.71 (m, 6H), 3.66-3.54 (m, 2H), 3.44 (t, 2H), 3.22-3.05 (m, 6H), 2.77 (t, 1H), 2.72-2.62 (m, 2H), 2.53 (t, 2H), 2.23 (t, 2H), 2.18 (t, 4H), 2.11-2.06 (m, 2H), 1.96-1.82 (m, 4H), 1.80-1.71 (m, 2H), 1.65-1.51 (m, 8H), 1.17-1.09 (m, 2H), 1.00-0.89 (m, 9H).

Biological Evaluation

Test Example 1. Assay on the Binding Activity of Compounds of the Present Disclosure for BCL-xL

*In vitro* BCL-xL-binding activity was tested by the following method.

Reagents used in this experiment: His-tagged BCL-xL protein (R&D, Cat# 894-BX-050); biotin-labeled BIM protein (R&D, Cat# 3526/1); binding buffer (cisbio, Cat# 62DLBDDF); assay buffer (cisbio, Cat# 62DB1FDG); MAb anti-6His-Eu cryptate (cisbio, Cat# 61HI2KLA); and affinity streptomycin linked XL665 (cisbio, Cat# 611SAXLA).

The *in vitro* binding assay described below can determine the binding activity of test compounds for BCL-xL.

The test compounds were dissolved in dimethyl sulfoxide and diluted to gradient concentrations as required for the experiment, and the compounds at various concentrations formulated in dimethyl sulfoxide were subjected to a 100-fold dilution with a binding buffer. BCL-xL was diluted to the corresponding concentration with a binding buffer, and added to a white 384-well plate at 4 μL/well. Then the diluted test compound was added to the white 384-well plate at 2 μL/well. The plate was incubated at 25 °C for 1 h after the mixture was uniformly mixed. Biotin-labeled BIM protein was diluted to the corresponding concentration with a binding buffer, and added to a white 384-well plate at 4 μL/well. The plate was incubated at 25 °C for 2 h after the mixture was uniformly mixed. The MAb anti-6His-Eu cryptate and the affinity streptomycin linked XL665 were each diluted to the corresponding concentration with an assay buffer, and added to a white 384-well plate at 5 μL/well. The plate was incubated at 25 °C for 2-4 h after the mixture was uniformly mixed. The signal values were read with the HTRF program in a microplate reader (BMG Labtech, model: PHERAstar FS). The test results are shown in Table 1.

Table 1. Binding activity of the compounds of the present disclosure for BCL-xL

| Example No. | Ki (nM) |
|---|---|
| 2-1 | 0.09 |
| 2-2 | 0.24 |
| 2-3 | 0.17 |
| 2-4 | 0.32 |
| 2-5 | 0.45 |
| 2-8 | 0.3 |
| 2-9 | 0.07 |
| 2-10 | 0.07 |
| 2-11 | 0.1 |
| 2-12 | 0.15 |
| 2-13 | 0.3 |

Conclusion: the compounds of the present disclosure have a relatively high binding activity to BCL-xL.

Test Example 2. Assay on the Binding Activity of Compounds of the Present Disclosure for BCL-2

*In vitro* BCL-2-binding activity was tested by the following method.

Reagents used in this experiment: His-tagged BCL-2 protein (R&D systems, Cat# 827-BC-050); biotin-labeled BIM protein (R&D, Cat# 3526/1); binding buffer (cisbio, Cat# 62DLBDDF); assay buffer (cisbio, Cat# 62DB1FDG); MAb anti-6His-Eu cryptate (cisbio, Cat# 61HI2KLA); and affinity streptomycin linked XL665 (cisbio, Cat# 611SAXLA).

The *in vitro* binding assay described below can determine the binding activity of test compounds for BCL-2.

The test compounds were dissolved in dimethyl sulfoxide and diluted to gradient concentrations as required for the experiment, and the compounds at various concentrations formulated in dimethyl sulfoxide were subjected to a 100-fold dilution with a binding buffer. BCL-2 was diluted to the corresponding concentration with a binding buffer solution, and added to a white 384-well plate at 4 μL/well. Then the diluted test compound was added into the white 384-well plate at 2 μL/well. The plate was incubated at 25 °C for 1 h after the mixture was uniformly mixed. Biotin-labeled BIM protein was diluted to the corresponding concentration with a binding buffer, and added to a white 384-well plate at 4 μL/well. The plate was incubated at 25 °C for 2 h after the mixture was uniformly mixed. The MAb anti-6His-Eu cryptate and the affinity streptomycin linked XL665 were each diluted to the corresponding concentration with an assay buffer, and added to a white 384-well plate at 5 μL/well. The plate was incubated at 25 °C for 2-4 h after the mixture was uniformly mixed. The signal values were read with the HTRF program in a microplate reader (BMG Labtech, model: PHERAstar FS).

The test results are shown in Table 2.

Table 2. Binding activity of the compounds of the present disclosure for BCL-2

| Example No. | Ki (nM) |
|---|---|
| 2-1 | 0.07 |
| 2-2 | 0.07 |
| 2-3 | 0.1 |
| 2-4 | 0.13 |
| 2-5 | 0.13 |
| 2-7 | 0.34 |
| 2-8 | 0.11 |
| 2-9 | 0.02 |
| 2-10 | 0.03 |
| 2-11 | 0.04 |
| 2-12 | 0.04 |
| 2-13 | 0.04 |

Conclusion: the compounds of the present disclosure have a relatively high binding activity to BCL-2.

Test Example 3. Determination of Inhibitory Activity of Compounds of the Present Disclosure for Proliferation of RS4;11 Cells and NCI-H146 Cells

*In vitro* inhibitory activity for the proliferation of RS4;11 cells and NCI-H146 cells was tested by the following method.

Reagents used in this experiment: RPMI-1640 medium (HyCLone, Cat# SH30243018); fetal bovine serum (Gibco, Cat# 10099-141); pancreatin (Gibco, Cat# 25200-072); Cell Titer-Glo (Promega, Cat# G7571).

Materials used: RS4;11 cells (Nanjing Kebai Biotechnology Co., Ltd, Cat# CBP60641); and NCI-H146 cells (ATCC, Cat# HTB-173).

The *in vitro* cell proliferation assay described below can determine the inhibitory activity of the test compounds for the proliferation of RS4;11 cells and NCI-H146 cells. RS4;11 cells (or NCI-H146 cells) were cultured in an RPMI-1640 culture medium containing 10% FBS, and were passaged for 2-3 times a week in a passage ratio of 1:3 or 1:5. During passage, the cells were transferred to a centrifuge tube, and centrifuged at 1200 rpm for 3 min. The supernatant was discarded, and fresh culture medium was added to resuspend the cells. 90 μL of cell suspension was added to a 96-well cell culture plate, wherein RS4;11 cells and NCI-H146 cells were added at a density of $2.22\times10^5$ cells/mL. Only 100 μL of complete medium (RPMI-1640 medium containing 10% FBS) was added to the periphery of the 96-well plate.The plate was incubated in an incubator for 24 h (37 °C, 5% $CO_2$).

The test sample was diluted to a proper concentration gradient with dimethyl sulfoxide as required for the experiment. 5 μL of the solution of the test compound prepared in a gradient concentration was added to 95 μL of fresh culture medium and mixed uniformly, and then 10 μL of the culture medium containing the above compound was added to a cell culture plate.The cell culture plate was incubated in an incubator for 3 days (37 °C, 5% $CO_2$). 50 μL of CellTiter-Glo reagent was added into each well of the 96-well cell culture plate. The plate was let stand for 5-10 min away from the light at room temperature. The chemiluminescence signals were read by a microplate reader (BMG Labtech, model: PHERAstar FS) and the data were processed by GraphPad software. The results are shown in Table 3.

Table 3. Inhibitory activity $IC_{50}$ (nM) of the compounds of the present disclosure for the proliferation of RS4;11 cells and NCI-H146 cells

| Example No. | RS4;11 cells | NCI-H146 cells |
|---|---|---|
| 2-1 | 23 | 36 |
| 2-2 | 7 | 33 |
| 2-3 | 58 | 74 |
| 2-4 | 34 | 94 |

(continued)

| Example No. | RS4;11 cells | NCI-H146 cells |
|---|---|---|
| 2-5 | 9 | 55 |
| 2-8 | 18 | 46 |
| 2-9 | 1.8 | 7 |
| 2-10 | 1 | 4 |
| 2-11 | 5 | 16 |
| 2-13 | 7 | 35 |

Conclusion: the compounds of the present disclosure have a relatively high inhibitory activity for the proliferation of RS4;11 cells and NCI-H146 cells.

Test Example 4. Toxicity Test on SD rats by Repeated Intravenous Administrations for 7

Days

1. Objective

This experiment was performed to evaluate the toxicity of compound 9 and AZD4320 to SD rats by repeated intravenous administrations for 7 days.

2. Test compounds

Storage condition: stored in a dryer at room temperature away from the light.
Compound 9, in 100% purity, molecular weight: 976.1.
Compound AZD4320, in 100% purity, molecular weight: 945.5.

3. Experimental design and procedures

3.1 Experimental animals and housing conditions

30 SD rats, weighing about 180-200 g and aged 6-7 weeks, half male and half female, purchased from Zhejiang Vital River Laboratory Animal Technology Co., Ltd., SPF grade. Animal certification number: 200917Aazz0619000298 and 20200917Aazz0619000104. Production license number: SCXK (Zhejiang) 2019-0001. Housing conditions: SPF grade, animals were housed in plastic and transparent rat cages, 2-3 per cage. The room temperature was 20-26 °C and the relative humidity was 40%-70%. The illumination was controlled in a 12/12 light/dark cycle. The granulated feed for mice was freely taken in an unlimited amount.

3.2 Grouping of animals

The rats were randomly divided into the following 3 groups: a vehicle control group, a compound 9-1 mg/kg group and a compound AZD4320-1 mg/kg group, with 6 rats in each group, half male and half female.

3.3 Experimental procedures

Dosage of the test sample for the 7-day toxicity test in rats: 1 mg/kg (qd), for 7 consecutive days. Administration route: intravenously, dosing volume: 10 mL/kg, dosing frequency and dosing period: daily in the morning for 7 days.
Vehicle: 1.5% DMA + 98.5% (10% HS-15), wherein DMA is N,N-dimethylacetamide (Shanghai Titan Scientific Co., Ltd., Cat# 01013630); and HS-15 is polyethylene glycol stearate 15 (Solutol HS15) (Shanghai Xietai Chemical Co., Ltd., Cat# 50253404).

3.4 Experimental Instruments

Hitachi 7100 automatic biochemical analyzer
Siemens advia 2120i automatic blood cell analyzer
Sysmex ca1500 automatic coagulation analyzer
Toxicokinetic data collection: AB 4000 liquid chromatograph/mass spectrometer

3.5 Data expression and statistical processing

The detection results were input and statistically processed by Excel of the computer. Clinical symptoms, food intake and general visual observation results of dissection were not statistically calculated, and indexes such as body weight, organ weight and clinical examination were counted by using T-test.

4. Results

SD rats were intravenously administered with 1 mg/kg compound 9 or 1 mg/kg AZD4320 at 5 mL/kg for 7 repeated days.

No significant abnormalities were observed in the clinical observation of the animals in the compound 9 administration group during treatment. Compared with the vehicle control group, there was no significant difference in body weight and weight growth rate of animals ($p > 0.05$) among the dose groups, and no significant change in food intake was observed.

The platelets of the rats in the compound 9 administration group were slightly reduced but within the background normal range, while the platelets of the AZD 4320 rats were more significantly reduced ($p < 0.01$). The results are shown in Table 4.

Table 4. Comparison of platelet values

| Group | PLT ($10^9$/L) |
|---|---|
| Vehicle control group | 1,503.83 |
| Compound 9-1 mg/kg | 1,258.67* |
| AZD 4320-1mg/kg | 807.50** |
| Note: * denotes $p < 0.05$; and ** denotes $p < 0.01$ | |

5. Conclusion

Under the conditions of this experiment, compared with the vehicle control group and the positive control AZD 4320 group, compound 9 reduces platelets less than the positive control group, and has the advantage of higher safety.

Test Example 5. Pharmacokinetic Evaluation of Compounds of the Present Disclosure in Mice

1. Abstract

The drug concentration in the plasma of the test animals (mice) at different time points after intravenous (iv) administration of compound 10 and AZD4320 was determined by an LC/MS/MS method. The pharmacokinetic behavior of the compounds of the present disclosure in mice was studied and their pharmacokinetic profile was valuated.

2. Methodology

2.1. Test compounds

Compound 10

2.2. Test animals

18 C57 mice, female, randomly divided into 2 groups, purchased from Zhejiang Vital River Laboratory Animal Technology Co., Ltd, animal production license number: SCXK (Shanghai) 2017-0005.

2.3. Drug preparation

A certain amount of compound 10 was weighed, dissolved by adding 5% by volume of DMSO and 5% Tween 80 (Shanghai Titan Scientific Co., Ltd.), and then prepared into a 0.1 mg/mL clear solution by adding 90% normal saline. Compound AZD4320 was formulated as with compound 10.

2.4. Administration

Intravenous administration: compounds 10 and AZD4320 were separately administered intravenously to mice at a dose of 2.0 mg/kg and a volume of 20.0 mL/kg.

3. Procedures

The mice were intravenously administered with compound 10, and 0.1 mL of blood was collected before the administration and 5 min, 0.25 h, 0.5 h, 1.0 h, 2.0 h, 4.0 h, 8.0 h, 11.0 h and 24.0 h after the administration, put into an EDTA-K2 anticoagulation tube, centrifuged at 10,000 rpm for 1 min (4 °C). Plasma was separated within 1 hour, and stored at -20 °C for testing. The process from blood collection to centrifugation was performed under an ice bath.

The mice were intravenously administered with compound AZD4320, and 0.1 mL of blood was collected before the administration and 5 min, 0.25 h, 0.5 h, 1.0 h, 2.0 h, 4.0 h, 8.0 h, 11.0 h and 24.0 h after the administration, put into an EDTA-K2 anticoagulation tube, centrifuged at 10,000 rpm for 1 min (4 °C). Plasma was separated within 1 hour, and stored at -20 °C for testing. The process from blood collection to centrifugation was performed under an ice bath.

The content of the test compounds at different concentrations in plasma of mice after the administration was determined: 10 μL of plasma of mice at each time point after the administration was mixed with 200 μL of methanol (containing an internal standard solution camptothecin (100 ng/mL)); the mixture was vortexed for 1 min, and centrifuged for 10 min (18,000 r/min), and 3 μL of supernatant was taken for LC/MS/MS analysis.

4. Pharmacokinetic parameters

Table 5. Pharmacokinetic parameters of compounds of the present disclosure in mice

| Route of administration | Example No. | Dose of administration | Plasma concentration | Area under curve | Half-life | Clearance | Apparent distribution volume | Mean retention time |
|---|---|---|---|---|---|---|---|---|
| | | (mg/kg) | CSmin (ng /mL) | $AUC_{0-t}$ (ng /mL*h) | Tl/2 (h) | CLz/F (ml/min/kg) | Vz/F (ml/kg) | MRT $0_{-\infty}$ (h) |
| Intravenous administration | Compound 10 | 1 | 608 | 296 | 13.0 | 50.3 | 56688 | 7.3 |
| Intravenous administration | AZD4320 | 1 | 436 | 206 | 3.1 | 72.7 | 19472 | 2.4 |

Conclusion: compound 10 of the present disclosure is cleared slowly in mice, has a longer elimination phase half-life than the positive control AZD 4320, and has pharmacokinetic advantages.

Test Example 6. Therapeutic Effect on Human Acute Lymphoblastic Leukemia RS4;11 Mouse Subcutaneous Xenograft Tumor

This experiment was performed to evaluate and compare the therapeutic effect of the compounds of the present disclosure on human acute lymphoblastic leukemia RS4;11 mouse subcutaneous xenograft tumor.

1. Experimental animals and housing conditions

NOD-Scid mice, 5 weeks old, purchased from Beijing Huafukang Biotechnology Co., Ltd. Production license No.: SCXK (Beijing) 2019-0008; animal certification No.: 110322211100992176. Housing environment: SPF grade. 6 mice in a vehicle group, and 8 mice in a compound group.

2. Test compounds

Compound 9, compound 10 and vehicle group.

5 mg of compound was added to 250 μL of DMSO and vortexed to provide a 20 mg/mL solution, which was diluted to the desired concentration with 30% hydroxypropyl-β-cyclodextrin (HP-β-CD, InnoStar Biotechnology Nantong Co., Ltd, Nantong).

3. Experimental design and procedures

Human acute lymphoblastic leukemia RS4;11 was purchased from American Type Culture Collection. RS4;11 cells were cultured in a 10 cm petri dish with an RPMI 1640 culture medium (Gibco) containing 10% fetal bovine serum (Gibco) and penicillin and streptomycin in an incubator at 37 °C containing 5% $CO_2$. The cells were passaged twice a week, and then collected, counted and inoculated when in the exponential growth phase.

Each mouse was inoculated subcutaneously with $1 \times 10^7$ RS4;11 cells. When tumors grew to 100~200 $mm^3$, the mice were grouped according to tumor volume, intravenously (IV) injected with the drug once a week (QW) for a total of 4 times, at an injection volume of 0.1 mL/10 g body weight and a dose of 10 mg/kg.

4. Results

The experimental index was to investigate the influence of the drug on the tumor growth, and the specific index was T/C% or tumor growth inhibition (TGI%).

Tumor diameters were measured twice a week with a vernier caliper and tumor volume (V) was calculated according to the following formula:

$$V = 1/2 \times a \times b^2$$

where a and b represent length and width, respectively.

$T/C(\%) = (T - T_0)/(C - C_0) \times 100$, where T and C represent the tumor volume of animals at the end of the experiment in the treatment group and control group, respectively; $T_0$ and $C_0$ represent the tumor volume of animals at the beginning of the experiment in the treatment group and control group, respectively.

$$\text{Tumor growth inhibition \% (TGI\%)} = 100 - T/C\ (\%).$$

$$\text{When tumor started to regress, tumor growth inhibition \% (TGI\%)} = 100 - (T - T_0)/T_0 \times 100.$$

If the tumor had reduced volume compared with the initial volume, i.e., $T < T_0$ or $C < C_0$, it was defined as partial regression (PR) of tumor; if the tumor completely disappeared, it was defined as complete regression (CR) of tumor.

Table 6. Therapeutic effect of test compounds on human acute lymphoblastic leukemia RS4;11 mouse subcutaneous xenograft tumor

| Group | Mean tumor volume ($mm^3$) | | Mean tumor volume ($mm^3$) | | Tumor inhibition rate TGI (%) |
|---|---|---|---|---|---|
| | D0 | SEM | D20 | SEM | |
| Vehicle group | 156.0 | ±6.0 | 1804.3 | ±83.8 | - |
| Compound 9 10 mg/kg | 151.0 | ±4.8 | 612.7 | ±85.2 | 72 |
| Compound 10 10 mg/kg | 151.9 | ±2.8 | 443.3 | ±52.7 | 82 |

Conclusion: compound 9 (10 mg/kg, IV, QW × 3) and compound 10 (10 mg/kg, IV, QW × 3) have significant inhibition effect on the growth of human acute lymphoblastic leukemia RS4;11 mouse subcutaneous xenograft tumor (FIG. 1), with the tumor inhibition rates being 72% and 82%, respectively; the tumor-bearing mice are all able to well tolerate the above drugs, and no symptoms such as significant weight loss are observed (FIG. 2).

## Claims

**1.** A compound of general formula (D) or a pharmaceutically acceptable salt thereof:

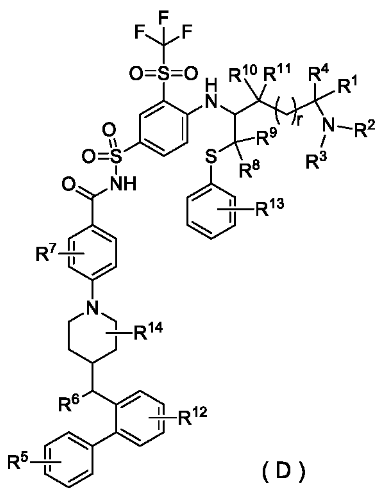

(D)

wherein:

$R^1$ is selected from the group consisting of deuterium, hydrogen, alkyl and deuterated alkyl;
$R^2$ is selected from the group consisting of alkyl and hydroxyalkyl;
or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;
$R^3$ is selected from the group consisting of hydrogen, hydroxyalkyl, alkyl, deuterated alkyl, cycloalkyl and cycloalkylalkyl;
$R^4$ is selected from the group consisting of hydrogen, halogen, deuterated alkyl and alkyl;
$R^5$ is selected from the group consisting of halogen and haloalkyl;
$R^6$ is selected from the group consisting of alkyl, amino, hydroxy and alkoxy;
$R^7$ is selected from the group consisting of hydrogen, halogen, alkyl, deuterated alkyl, hydroxy and alkoxy;
$R^8$ and $R^9$ are each independently selected from the group consisting of hydrogen, alkyl, deuterated alkyl and cycloalkyl; or $R^8$ and $R^9$, together with the atom to which they are attached, form cycloalkyl;
$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;

$R^{12}$ is selected from the group consisting of hydrogen, deuterium, deuterated alkyl, halogen, hydroxy, alkyl, hydroxyalkyl, alkoxy and cycloalkyl;
$R^{13}$ is selected from the group consisting of hydrogen, deuterium, deuterated alkyl, halogen, hydroxy, alkyl, hydroxyalkyl, alkoxy and cycloalkyl;
$R^{14}$ is selected from the group consisting of hydrogen, deuterium, deuterated alkyl, halogen, hydroxy, alkyl, hydroxyalkyl, alkoxy and cycloalkyl; and
r is selected from the group consisting of 0, 1, 2 and 3;
provided that when $R^1$ and $R^2$ do not, together with the atom to which they are attached, form heterocyclyl, $R^5$ is haloalkyl.

**2.** The compound of general formula (D) or the pharmaceutically acceptable salt thereof according to claim 1, wherein $R^1$ is hydrogen, and $R^2$is alkyl.

**3.** The compound of general formula (D) or the pharmaceutically acceptable salt thereof according to claim 1, wherein $R^1$ and $R^2$, together with the atom to which they are attached, form heterocyclyl; preferably, the heterocyclyl optionally further comprises 1 to 3 heteroatoms selected from the group consisting of nitrogen and oxygen; and more preferably, $R^1$ and $R^2$, together with the atom to which they are attached, form pyrrolidinyl.

**4.** The compound of general formula (D) or the pharmaceutically acceptable salt thereof according to claim 1, being a compound of general formula (D-I) or a pharmaceutically acceptable salt thereof:

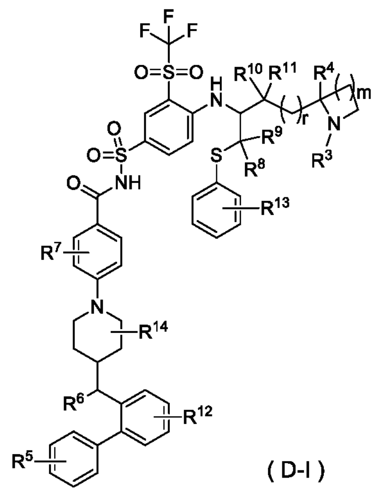

( D-I )

wherein m is selected from the group consisting of 0, 1, 2 and 3; and
r and $R^3$-$R^{14}$ are as defined in claim 1.

**5.** The compound of general formula (D) or the pharmaceutically acceptable salt thereof according to claim 1, being a compound of general formula (D-II) or a pharmaceutically acceptable salt thereof:

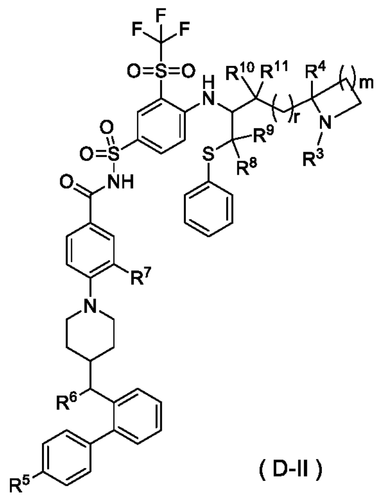

( D-II )

wherein m is selected from the group consisting of 0, 1, 2 and 3; and r and $R^3$-$R^{11}$ are as defined in claim 1.

**6.** The compound of general formula (D) or the pharmaceutically acceptable salt thereof according to claim 1, being a compound of general formula (D-III) or a pharmaceutically acceptable salt thereof:

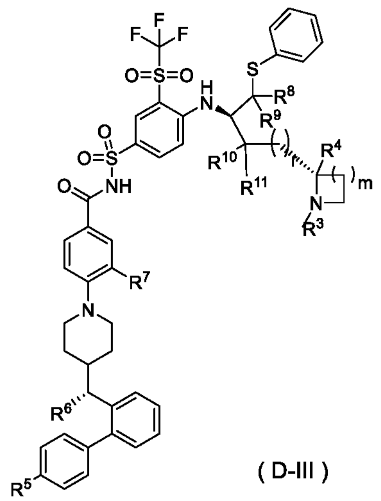

( D-III )

wherein m is selected from the group consisting of 0, 1, 2 and 3; and r and $R^3$-$R^{11}$ are as defined in claim 1.

**7.** The compound of general formula (D) or the pharmaceutically acceptable salt thereof according to claim 1, being a compound of general formula (D-IV) or a pharmaceutically acceptable salt thereof:

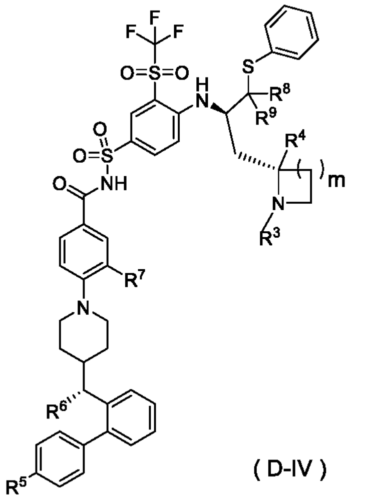

( D-IV )

wherein,

m is selected from the group consisting of 0, 1, 2 and 3; and
$R^3$-$R^9$ are as defined in claim 1.

**8.** The compound of general formula (D) or the pharmaceutically acceptable salt thereof according to any one of claims 4 to 7, wherein m is 2.

**9.** The compound of general formula (D) or the pharmaceutically acceptable salt thereof according to any one of claims 1 to 8, wherein $R^5$ is chlorine.

**10.** The compound of general formula (D) or the pharmaceutically acceptable salt thereof according to any one of claims 1 to 8, wherein $R^5$ is trifluoromethyl.

**11.** The compound of general formula (D) or the pharmaceutically acceptable salt thereof according to any one of claims 1 to 10, wherein $R^3$ is selected from the group consisting of hydrogen, hydroxyalkyl and alkyl; preferably hydroxyalkyl.

**12.** The compound of general formula (D) or the pharmaceutically acceptable salt thereof according to claim 1, being a compound of general formula (D-V) or a pharmaceutically acceptable salt thereof:

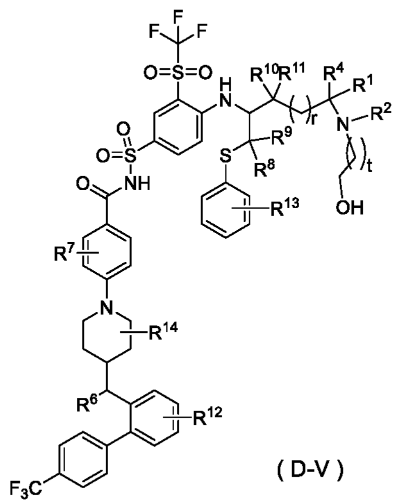

( D-V )

wherein t is selected from the group consisting of 0, 1, 2 and 3, preferably 1; and
r, $R^1$, $R^2$, $R^4$, and $R^6$-$R^{14}$ are as defined in claim 1.

**13.** The compound of general formula (D) or the pharmaceutically acceptable salt thereof according to claim 1, being

a compound of general formula (D-VI) or a pharmaceutically acceptable salt thereof:

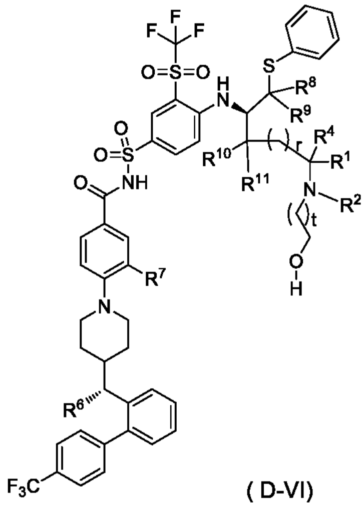

( D-VI)

wherein t is selected from the group consisting of 0, 1, 2 and 3, preferably 1; and r, $R^1$, $R^2$, $R^4$, and $R^6$-$R^{11}$ are as defined in claim 1.

**14.** The compound of general formula (D) or the pharmaceutically acceptable salt thereof according to claim 1, being a compound of general formula (D-VII) or a pharmaceutically acceptable salt thereof:

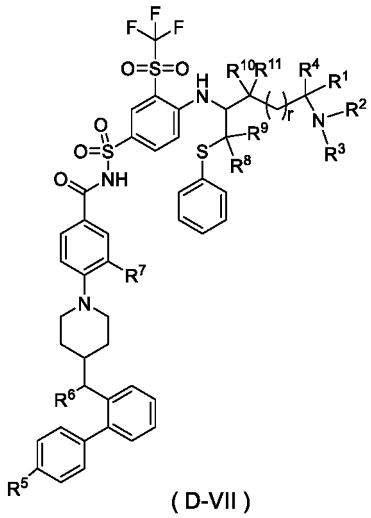

( D-VII )

wherein r and $R^1$-$R^{11}$ are as defined in claim 1.

**15.** The compound of general formula (D) or the pharmaceutically acceptable salt thereof according to any one of claims 1 to 14, wherein $R^4$ is selected from the group consisting of hydrogen and alkyl; preferably hydrogen.

**16.** The compound of general formula (D) or the pharmaceutically acceptable salt thereof according to any one of claims 1 to 15, wherein $R^6$ is selected from the group consisting of hydrogen and alkoxy; preferably hydroxy.

**17.** The compound of general formula (D) or the pharmaceutically acceptable salt thereof according to any one of claims 1 to 16, wherein $R^7$, $R^8$ and $R^9$ are hydrogen.

**18.** The compound of general formula (D) or the pharmaceutically acceptable salt thereof according to any one of claims 1 to 6 and 8 to 17, wherein $R^{10}$ and $R^{11}$ are each independently selected from the group consisting of hydrogen and alkyl; preferably hydrogen.

**19.** The compound of general formula (D) or the pharmaceutically acceptable salt thereof according to any one of claims 1 to 6 and 8 to 18, wherein r is 0 or 1; preferably 0.

**20.** The compound of general formula (D) or the pharmaceutically acceptable salt thereof according to claim 1, wherein the compound is selected from the group consisting of:

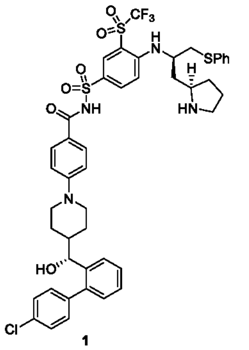
1 ,
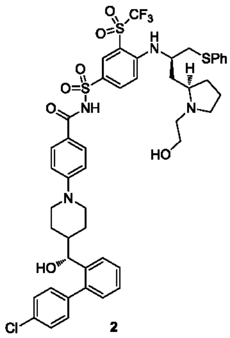
2 ,
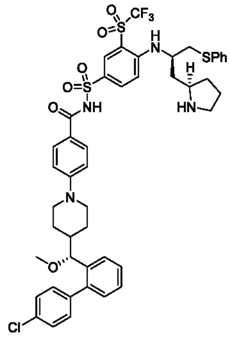
3 ,
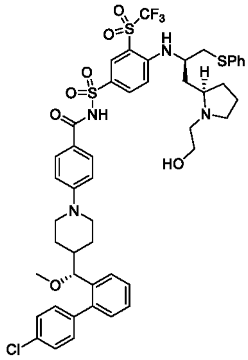
4 ,
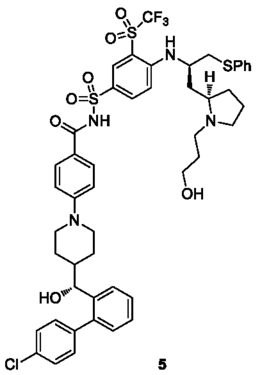
5 ,
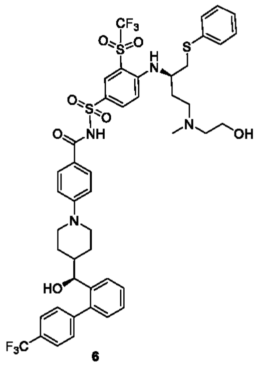
6 ,
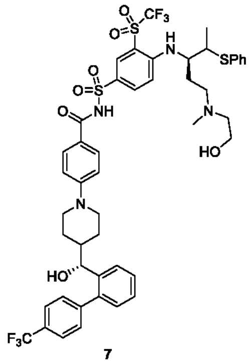
7 ,
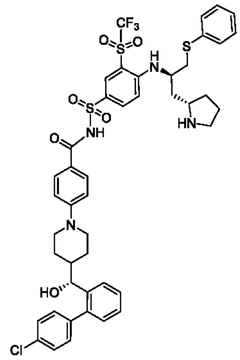
8 ,
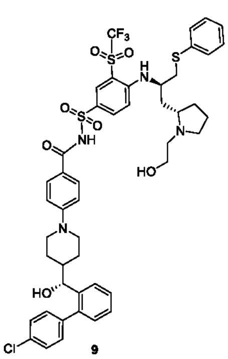
9 ,
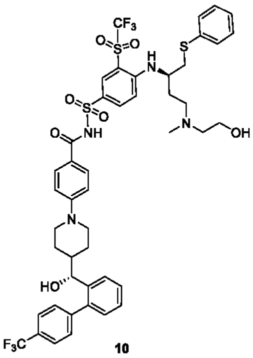
10 ,
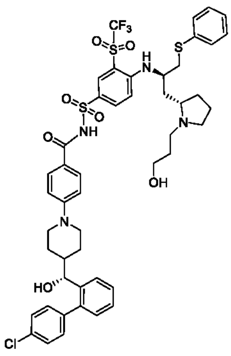
11 ,
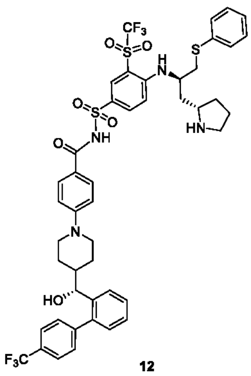
12 ,

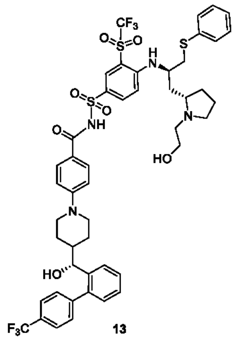
13 ,
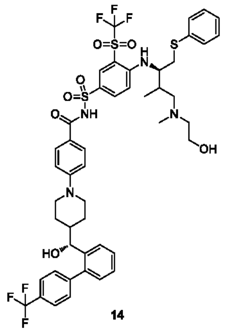
14 ,
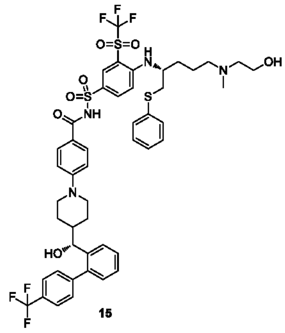
15 ,
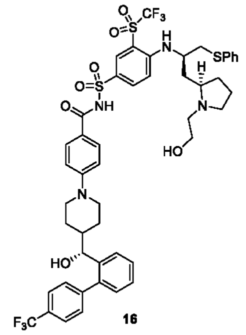
16 ,

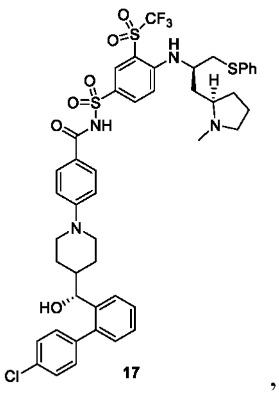
17 ,
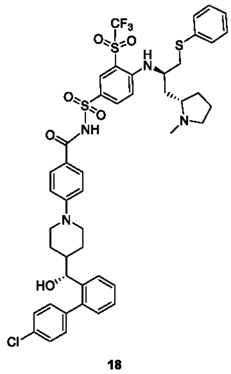
18 ,
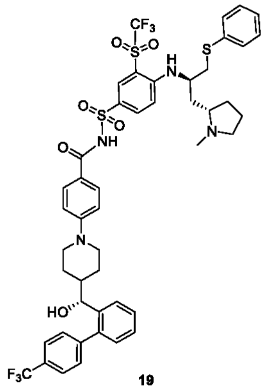
19 ,
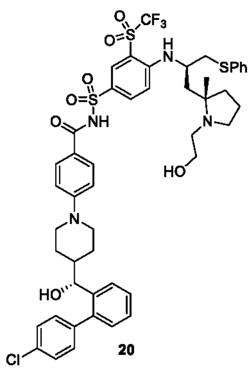
20 ,

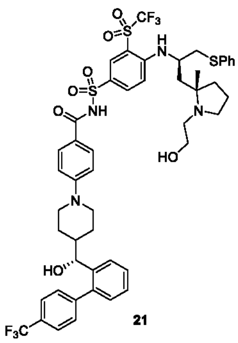
21 ,
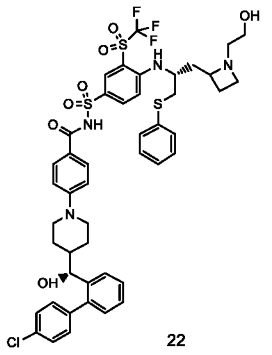
22 and
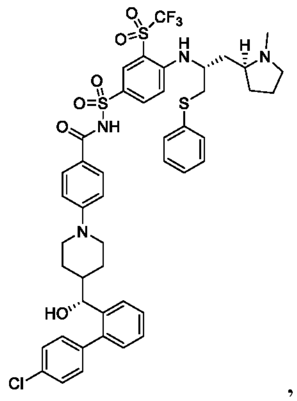
,

or the pharmaceutically acceptable salt thereof.

**21.** A compound of general formula (DA1) or (DB1) or a pharmaceutically acceptable salt thereof,

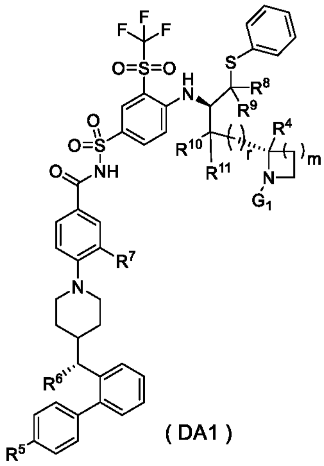

( DA1 )

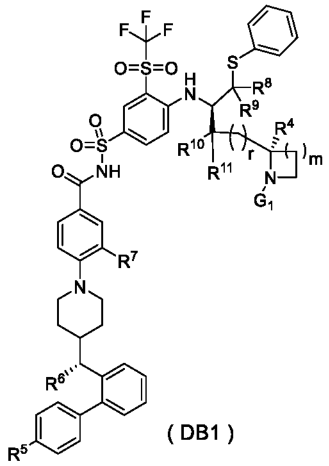

( DB1 )

wherein:

$G_1$ is an amino protecting group, preferably Boc;
m is selected from the group consisting of 0, 1, 2 and 3; and
r and $R^4$-$R^{11}$ are as defined in claim 1.

**22.** The compound of general formula (DA1) or (DB1) according to claim 21, selected from any one of the following compounds:

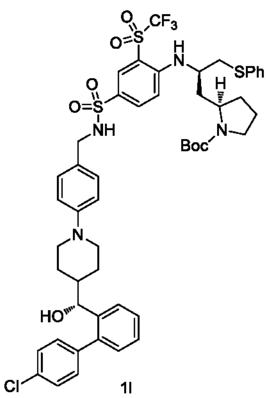

1l ,

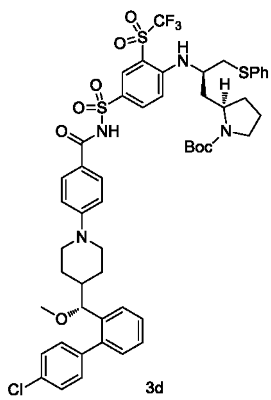

3d ,

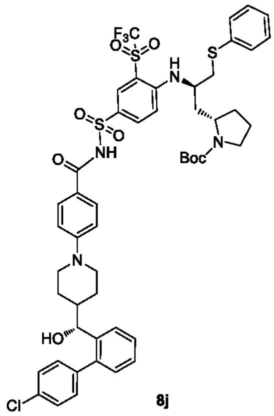

8j

and

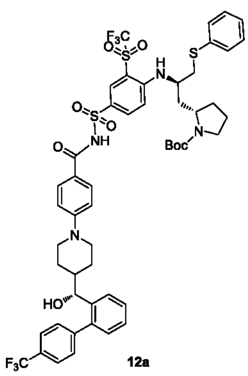

12a .

**23.** A method for preparing a compound of general formula (D-III) or a pharmaceutically acceptable salt thereof, comprising:

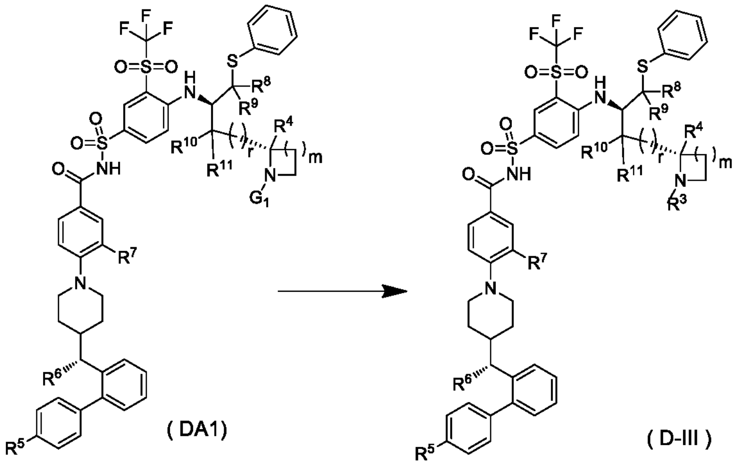

( DA1)

( D-III )

subjecting a compound of general formula (DA1) or a pharmaceutically acceptable salt thereof to a deprotection reaction under an acidic condition to obtain the compound of general formula (D-III) or the pharmaceutically acceptable salt thereof; preferably, a reagent used in the acidic condition being HCl;
wherein:

$G_1$ is an amino protecting group, preferably Boc;
m is selected from the group consisting of 0, 1, 2 and 3;
$R^3$ is hydrogen; and
r and $R^4$-$R^{11}$ are as defined in claim 1.

**24.** A compound of general formula (DA2) or (DB2) or a pharmaceutically acceptable salt thereof,

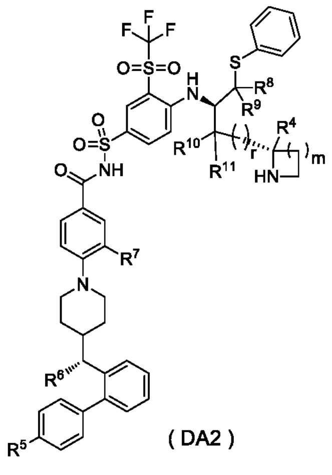

( DA2 )

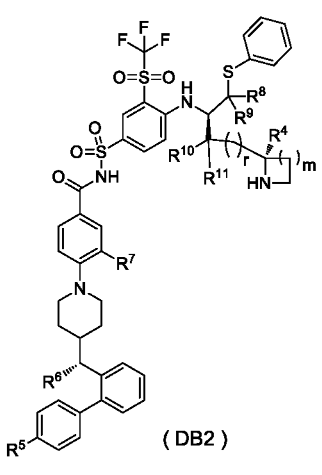

( DB2 )

wherein:

m is selected from the group consisting of 0, 1, 2 and 3; and
r and $R^4$-$R^{11}$ are as defined in claim 1.

**25.** The compound of general formula (DA2) or (DB2) according to claim 24, selected from any one of the following compounds:

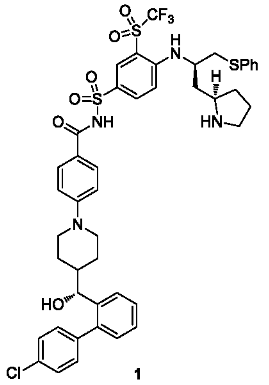

1 ,

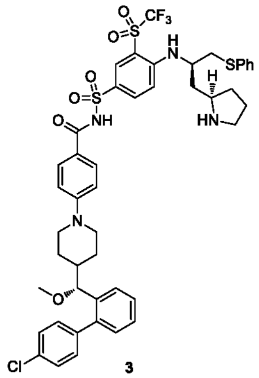

3 ,

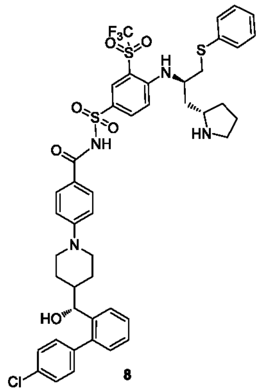

8

and

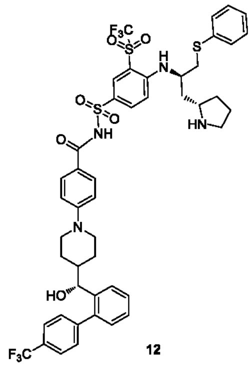

12 .

**26.** A method for preparing a compound of general formula (D-III) or a pharmaceutically acceptable salt thereof, comprising:

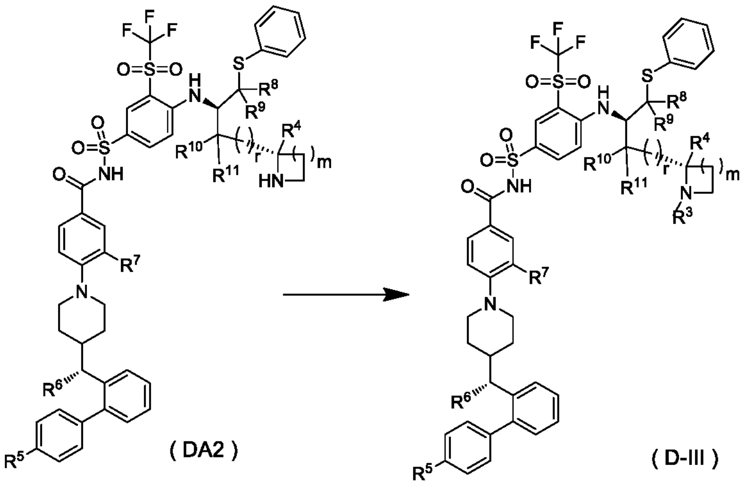

( DA2 ) ( D-III )

subjecting a compound of general formula (DA2) or a pharmaceutically acceptable salt thereof to a substitution reaction under an alkaline condition to obtain the compound of general formula (D-III) or the pharmaceutically acceptable salt thereof; preferably, a reagent used in the alkaline condition being triethylamine;
wherein:

m is selected from the group consisting of 0, 1, 2 and 3; and
$R^3$ is hydroxyalkyl; and
r and $R^4$-$R^{11}$ are as defined in claim 1.

**27.** A compound of general formula (DA3), (DB3) or (DC3) or a pharmaceutically acceptable salt thereof,

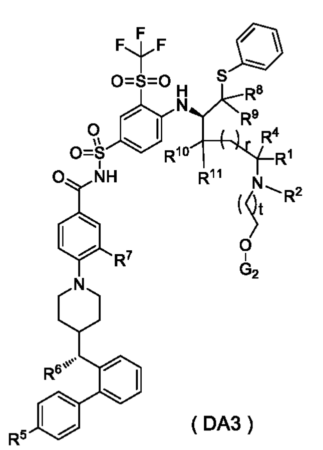
( DA3 )

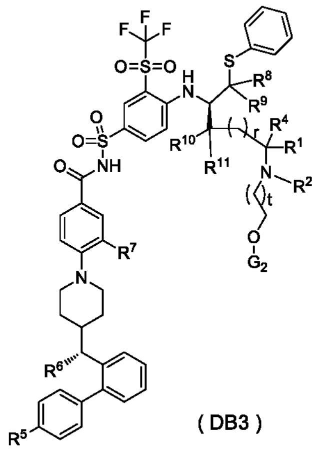
( DB3 )

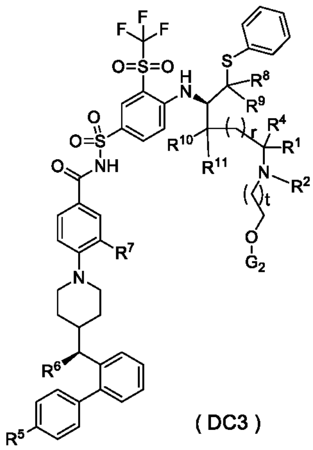
( DC3 )

wherein:

$G_2$ is a hydroxy protecting group, preferably TBS;
t is selected from the group consisting of 0, 1, 2 and 3; and
r, $R^1$, $R^2$, and $R^4$-$R^{11}$ are as defined in claim 1.

**28.** The compound of general formula (DA3), (DB3) or (DC3) or the pharmaceutically acceptable salt thereof according to claim 27, selected from any one of the following compounds:

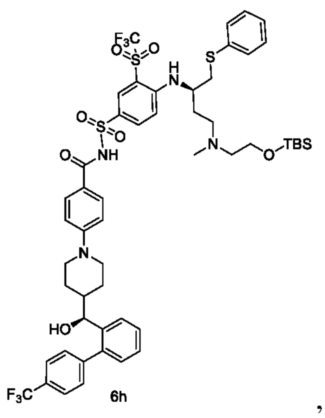
6h ,

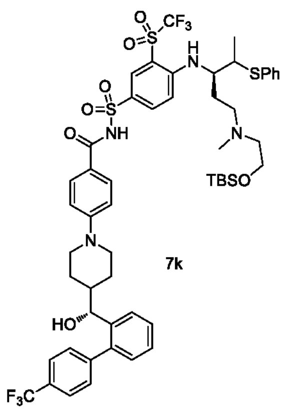
7k

and

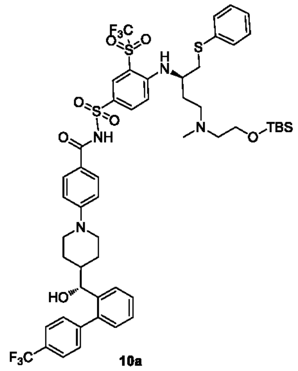
10a .

**29.** A method for preparing a compound of general formula (D-VI) or a pharmaceutically acceptable salt thereof, comprising:

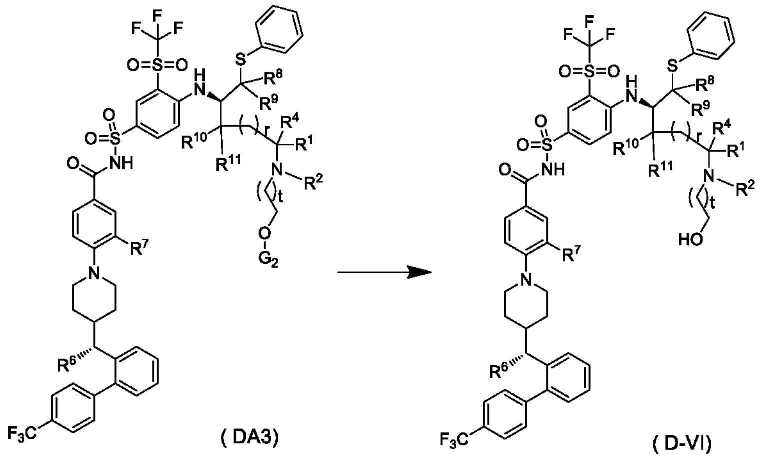

( DA3)          ( D-VI)

subjecting a compound of general formula (DA3) or a pharmaceutically acceptable salt thereof to a deprotection reaction under an alkaline condition to obtain the compound of general formula (D-VI) or the pharmaceutically acceptable salt thereof;

wherein:

$G_2$ is a hydroxy protecting group, preferably TBS;
t is selected from the group consisting of 0, 1, 2 and 3; and
r, $R^1$, $R^2$, and $R^4$-$R^{11}$ are as defined in claim 1.

**30.** A ligand-drug conjugate comprising a structure of formula (-D) or a pharmaceutically acceptable salt thereof:

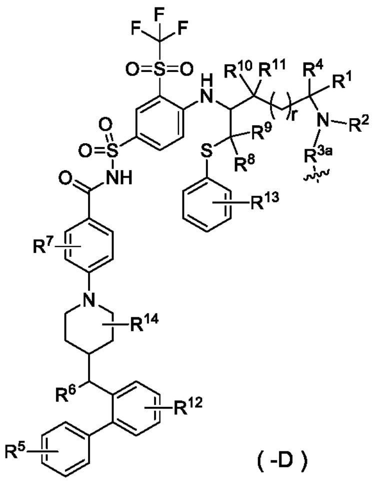

( -D )

wherein:

$R^1$ is selected from the group consisting of deuterium, hydrogen, alkyl and deuterated alkyl;
$R^2$ is selected from the group consisting of alkyl and hydroxyalkyl;
or $R^1$ and $R^2$, together with the atom to which they are attached, form heterocyclyl optionally further substituted with a substituent selected from the group consisting of halogen, hydroxy, alkyl, hydroxyalkyl, alkoxy and cycloalkyl;
$R^{3a}$ is selected from the group consisting of a bond and $-(CH_2)_t-CH_2-O-$;
$R^4$ is selected from the group consisting of hydrogen, halogen, deuterated alkyl and alkyl;
$R^5$ is selected from the group consisting of halogen and haloalkyl;
$R^6$ is selected from the group consisting of alkyl, amino, hydroxy and alkoxy;
$R^7$ is selected from the group consisting of hydrogen, halogen, alkyl, deuterated alkyl, hydroxy and alkoxy;
$R^8$ and $R^9$ are each independently selected from the group consisting of hydrogen, alkyl, deuterated alkyl and cycloalkyl; or $R^8$ and $R^9$, together with the atom to which they are attached, form cycloalkyl;

$R^{10}$ and $R^{11}$ are each independently selected from the group consisting of hydrogen, alkyl, deuterated alkyl and cycloalkyl; or $R^{10}$ and $R^{11}$, together with the atom to which they are attached, form cycloalkyl;
$R^{12}$ is selected from the group consisting of hydrogen, deuterium, deuterated alkyl, halogen, hydroxy, alkyl, hydroxyalkyl, alkoxy and cycloalkyl;
$R^{13}$ is selected from the group consisting of hydrogen, deuterium, deuterated alkyl, halogen, hydroxy, alkyl, hydroxyalkyl, alkoxy and cycloalkyl;
$R^{14}$ is selected from the group consisting of hydrogen, deuterium, deuterated alkyl, halogen, hydroxy, alkyl, hydroxyalkyl, alkoxy and cycloalkyl; and
r is selected from the group consisting of 0, 1, 2 and 3;
t is selected from the group consisting of 0, 1, 2 and 3; and
the wavy line indicates covalent linking to a linker unit or to a ligand.

**31.** The ligand-drug conjugate comprising the structure of formula (-D) or the pharmaceutically acceptable salt thereof according to claim 30, wherein the formula (-D) is selected from the group consisting of structures of formulae (-DIII), (-DIV) and (-DV):

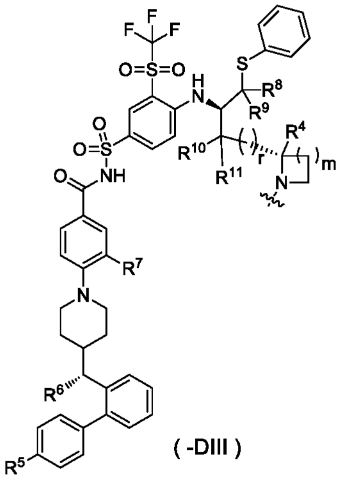
( -DIII )

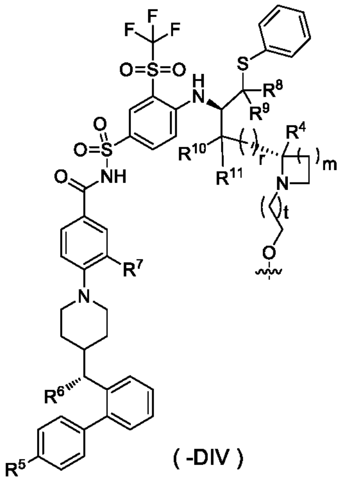
( -DIV )

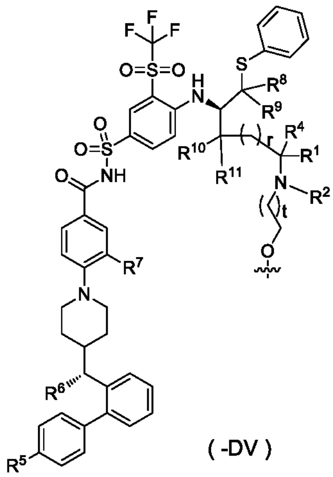
( -DV )

wherein:

t is selected from the group consisting of 0, 1, 2 and 3;
m is selected from the group consisting of 0, 1, 2 and 3; and
the wavy line, r, $R^1$, $R^2$, and $R^4$-$R^{11}$ are as defined in claim 30.

**32.** A pharmaceutical composition comprising a therapeutically effective amount of the compound of general formula (D) or the pharmaceutically acceptable salt thereof according to any one of claims 1 to 20, or the ligand-drug conjugate comprising the structure of formula (-D) or the pharmaceutically acceptable salt thereof according to claim 30 or 31, and a pharmaceutically acceptable carrier, diluent or excipient.

**33.** Use of the compound of general formula (D) or the pharmaceutically acceptable salt thereof according to any one of claims 1 to 20, or the ligand-drug conjugate comprising the structure of formula (-D) or the pharmaceutically acceptable salt thereof according to claim 30 or 31, or the pharmaceutical composition according to claim 32 in the preparation of a Bcl-2 and/or Bcl-XL inhibitor.

**34.** Use of the compound of general formula (D) or the pharmaceutically acceptable salt thereof according to any one of claims 1 to 20, or the ligand-drug conjugate comprising the structure of formula (-D) or the pharmaceutically acceptable salt thereof according to claim 30 or 31, or the pharmaceutical composition according to claim 32 in the preparation of a medicament for treating or preventing a tumor.

**35.** Use of the compound of general formula (D) or the pharmaceutically acceptable salt thereof according to any one of claims 1 to 20, or the ligand-drug conjugate comprising the structure of formula (-D) or the pharmaceutically acceptable salt thereof according to claim 30 or 31, or the pharmaceutical composition according to claim 32 in the preparation of a medicament for treating and/or preventing a cancer, wherein the cancer is preferably melanoma,

liver cancer, kidney cancer, lung cancer, small cell lung cancer, non-small cell lung cancer, nasopharyngeal cancer, colorectal cancer, colon cancer, rectal cancer, pancreatic cancer, cervical cancer, ovarian cancer, breast cancer, bladder cancer, prostate cancer, leukemia, acute myeloid leukemia, chronic myeloid leukemia, chronic lymphocytic leukemia, acute lymphocytic leukemia, acute myelogenous leukemia, mantle cell lymphoma, diffuse large B-cell lymphoma, follicular central lymphoma, non-Hodgkin’s lymphoma, T cell lymphoma, B cell lymphoma, head and neck squamous cell carcinoma, cervical cancer, thyroid cancer, lymphoma, sarcoma, neuroblastoma, brain tumor, myeloma, astrocytoma and glioma.

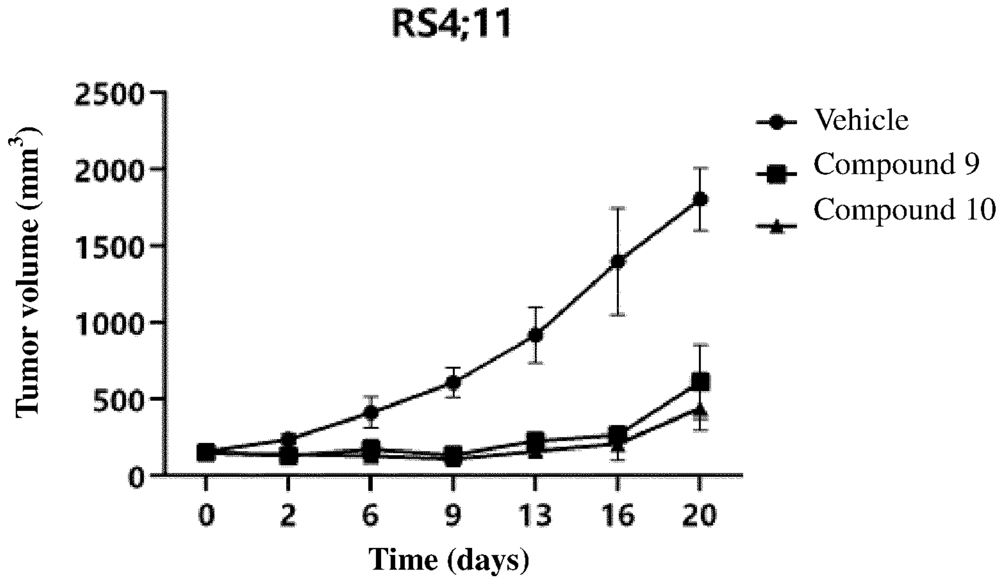
RS4;11
Tumor volume (mm3)
2500
2000
1500
1000
500
0
0
2
6
9
13
16
20
Time (days)
Vehicle
Compound 9
Compound 10

FIG. 1

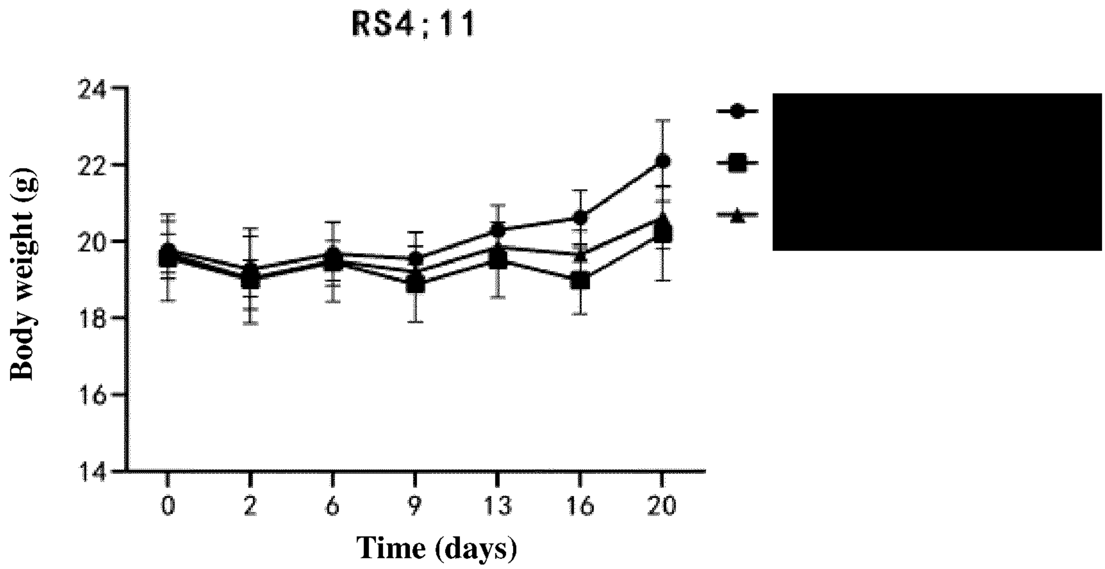
RS4;11
Body weight (g)
24
22
20
18
16
14
0
2
6
9
13
16
20
Time (days)
[redacted]

FIG. 2

**INTERNATIONAL SEARCH REPORT**

International application No.
**PCT/CN2021/105436**

**A. CLASSIFICATION OF SUBJECT MATTER**

C07D 211/34(2006.01)i; C07D 401/12(2006.01)i; A61K 31/451(2006.01)i; A61K 31/454(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

WPI, EPODOC, CNKI, CNPAT, CNTXT, CAPLUS（STN）, REGISTRY（STN）, MARPAT（STN）, 江苏恒瑞医药, 许建烟, 高建红, 章瑛, 贺峰, 陶维康, 哌啶, 磺酰, 苯甲酰胺, 细胞凋亡, 肿瘤, 癌, sulfonyl, benzamide, piperidyl, cancer?, tumor?, apoptosis, Bcl+

**C. DOCUMENTS CONSIDERED TO BE RELEVANT**

| Category* | Citation of document, with indication, where appropriate, of the relevant passages | Relevant to claim No. |
|---|---|---|
| X | CN 110312531 A (ASTRAZENECA AB) 08 October 2019 (2019-10-08) claims 46, 62, description pages 2-3, 29-30 | 30-35 |
| A | CN 103153954 A (ASTRAZENECA AB) 12 June 2013 (2013-06-12) description, pages 1, 5, 129-131 | 1-35 |
| A | CN 1906183 A (ABBOTT LAB) 31 January 2007 (2007-01-31) description, pages 1-7 and 65 | 1-35 |
| A | VARNES, J.G. et al. "Towards the next generation of dual Bcl-2/Bcl-xL inhibitors" *Bioorganic & Medicinal Chemistry Letters,* Vol. 24, 20 May 2014 (2014-05-20), pp. 3026-3033 | 1-35 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2021** | **29 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/105436**

| Patent document cited in search report | Publication date (day/month/year) | Patent family member(s) | Publication date (day/month/year) |
|---|---|---|---|
| CN 110312531 A | 08 October 2019 | TW 201841659 A | 01 December 2018 |
| | | WO 2018154004 A1 | 30 August 2018 |
| | | US 10314920 B2 | 11 June 2019 |
| | | JP 2020508985 A | 26 March 2020 |
| | | MX 2019009442 A | 09 October 2019 |
| | | KR 20190112330 A | 04 October 2019 |
| | | AU 2018223149 B2 | 01 April 2021 |
| | | SG 11201907334 Q A | 27 September 2019 |
| | | CL 2019002240 A1 | 29 November 2019 |
| | | US 2020069810 A1 | 05 March 2020 |
| | | CR 20190361 A | 24 September 2019 |
| | | PE 20191660 A1 | 11 November 2019 |
| | | NI 201900088 A | 18 March 2020 |
| | | PH 12019501896 A1 | 21 October 2019 |
| | | CO 2019009722 A2 | 18 September 2019 |
| | | US 2018264127 A1 | 20 September 2018 |
| | | DO P2019000204 A | 30 August 2019 |
| | | AU 2018223149 A1 | 03 October 2019 |
| | | BR 112019016409 A2 | 07 April 2020 |
| | | WO 2018154004 A9 | 27 December 2018 |
| | | US 10888624 B2 | 12 January 2021 |
| | | EC SP19056271 A | 30 August 2019 |
| | | JO P20190191 A1 | 16 June 2017 |
| | | CA 3053069 A1 | 30 August 2018 |
| | | US 2021060172 A1 | 04 March 2021 |
| | | EP 3585441 A1 | 01 January 2020 |
| | | IL 268648 D0 | 31 October 2019 |
| CN 103153954 A | 12 June 2013 | ME 02885 B | 20 April 2018 |
| | | ES 2653936 T3 | 09 February 2018 |
| | | NI 201300018 A | 16 October 2013 |
| | | CA 2806190 A1 | 09 February 2012 |
| | | MX 343043 B | 21 October 2016 |
| | | CU 20130020 A7 | 31 July 2013 |
| | | AU 2011287398 A1 | 07 March 2013 |
| | | WO 2012017251 A1 | 09 February 2012 |
| | | PL 2601177 T3 | 28 February 2018 |
| | | SG 10201506194 Y A | 29 September 2015 |
| | | JP 5886855 B2 | 16 March 2016 |
| | | US 2012035134 A1 | 09 February 2012 |
| | | UA 110943 C2 | 10 March 2016 |
| | | KR 20130137599 A | 17 December 2013 |
| | | US 9018381 B2 | 28 April 2015 |
| | | PT 2601177 T | 18 December 2017 |
| | | US 2016176906 A1 | 23 June 2016 |
| | | AR 082573 A1 | 19 December 2012 |
| | | AU 2011287398 C1 | 24 August 2017 |
| | | UY 33547 A | 30 March 2012 |
| | | CL 2015002372 A1 | 05 February 2016 |
| | | GT 201300034 A | 03 August 2015 |
| | | HU E035254 T2 | 02 May 2018 |

Form PCT/ISA/210 (patent family annex) (January 2015)

**INTERNATIONAL SEARCH REPORT**
**Information on patent family members**

International application No.
**PCT/CN2021/105436**

| Patent document cited in search report | Publication date (day/month/year) | Patent family member(s) | Publication date (day/month/year) |
|---|---|---|---|
| | | CR 20130051 A | 15 May 2013 |
| | | CO 6670585 A2 | 15 May 2013 |
| | | EA 027788 B1 | 29 September 2017 |
| | | TW I535712 B | 01 June 2016 |
| | | EP 2601177 A1 | 12 June 2013 |
| | | BR 112013002914 A2 | 08 January 2019 |
| | | US 2013310344 A1 | 21 November 2013 |
| | | JP 2016138109 A | 04 August 2016 |
| | | LT 2601177 T | 27 December 2017 |
| | | CL 2013000322 A1 | 19 April 2013 |
| | | KR 101840893 B1 | 21 March 2018 |
| | | CY 1120031 T1 | 12 December 2018 |
| | | MY 165624 A | 18 April 2018 |
| | | SA 4022 B1 | 19 May 2015 |
| | | US 9248140 B2 | 02 February 2016 |
| | | EA 201300211 A1 | 30 August 2013 |
| | | SG 187178 A1 | 28 March 2013 |
| | | NO 2601177 T3 | 17 March 2018 |
| | | AU 2015210452 A1 | 03 September 2015 |
| | | HR P20171899 T1 | 26 January 2018 |
| | | CA 2806190 C | 17 July 2018 |
| | | AU 2011287398 B2 | 27 August 2015 |
| | | JP 2013532718 A | 19 August 2013 |
| | | TW 201209049 A | 01 March 2012 |
| | | IL 224218 A | 31 July 2016 |
| | | SI 2601177 T1 | 31 January 2018 |
| CN 1906183 A | 31 January 2007 | TW I337601 B | 21 February 2011 |
| | | EP 1685119 A1 | 02 August 2006 |
| | | EP 2308812 B1 | 26 February 2014 |
| | | DK 2308812 T3 | 02 June 2014 |
| | | KR 20060114704 A | 07 November 2006 |
| | | US 2005159427 A1 | 21 July 2005 |
| | | WO 2005049593 A3 | 07 July 2005 |
| | | ZA 200603823 B | 31 October 2007 |
| | | PT 2308812 E | 26 May 2014 |
| | | SI 2308812 T1 | 30 May 2014 |
| | | PT 1685119 E | 17 June 2016 |
| | | TW 200526559 A | 16 August 2005 |
| | | EP 3048098 B1 | 15 August 2018 |
| | | PL 1685119 T3 | 30 September 2016 |
| | | WO 2005049593 A2 | 02 June 2005 |
| | | HU E028622 T2 | 28 December 2016 |
| | | WO 2005049594 A1 | 02 June 2005 |
| | | ES 2461615 T3 | 20 May 2014 |
| | | US 8084607 B2 | 27 December 2011 |
| | | CN 1906183 B | 20 June 2012 |
| | | PL 2308812 T3 | 29 August 2014 |
| | | AU 2004290666 A1 | 02 June 2005 |
| | | SI EP1685119 T1 | 29 July 2016 |
| | | EP 1685119 B1 | 09 March 2016 |

Form PCT/ISA/210 (patent family annex) (January 2015)

**INTERNATIONAL SEARCH REPORT**
**Information on patent family members**

International application No.
**PCT/CN2021/105436**

| Patent document cited in search report | Publication date (day/month/year) | Patent family member(s) | | | Publication date (day/month/year) |
|---|---|---|---|---|---|
| | | BR | PI0416602 | A | 30 January 2007 |
| | | CA | 2546101 | A1 | 02 June 2005 |
| | | HK | 1156295 | A1 | 08 June 2012 |
| | | US | 2010022773 | A1 | 28 January 2010 |
| | | ES | 2697403 | T3 | 23 January 2019 |
| | | EP | 2308812 | A2 | 13 April 2011 |
| | | HK | 1098738 | A1 | 27 July 2007 |
| | | KR | 101157408 | B1 | 06 July 2012 |
| | | JP | 2007511530 | A | 10 May 2007 |
| | | EP | 2308812 | A3 | 23 May 2012 |
| | | ES | 2569327 | T3 | 10 May 2016 |
| | | CA | 2546101 | C | 11 June 2013 |
| | | SI | EP2308812 | T1 | 30 May 2014 |
| | | EP | 3048098 | A1 | 27 July 2016 |
| | | JP | 4870570 | B2 | 08 February 2012 |
| | | NZ | 547160 | A | 27 November 2009 |
| | | IL | 175609 | A | 24 March 2013 |
| | | SI | 1685119 | T1 | 29 July 2016 |
| | | AU | 2004290666 | B2 | 26 February 2009 |
| | | DK | 1685119 | T3 | 13 June 2016 |
| | | IL | 175609 | D0 | 05 September 2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050238649 A1
- US 5208020 A
- CN 103153954 B
- US 2004171833 A1
- WO 2012017251 A


### Non-patent literature cited in the description


- **CHARI et al.** *Cancer Research,* 1992, vol. 52, 127-131
- *J. biol. chem,* 1968, vol. 243, 3558
- Antibodies: A Laboratory Manual. Cold Spring Harbor Press